# EUROPEAN PATENT APPLICATION

(11) **EP 2 712 869 A1**
(43) Date of publication of application: **02.04.2014**
(21) Application number: 12186576.0
(22) Date of filing: 28.09.2012
(51) Int. Cl.: C07H 19/073, C07H 19/067, C07H 19/173, C07H 21/04, C12Q 1/68

(54) **Reactive, lipophilic nucleoside building blocks for the synthesis of hydrophobic nucleic acids**

(71) Applicant: Ionovation GmbH, 49084 Osnabrück (DE)
(72) Inventor: Rosemeyer, Helmut, 49076 Osnabrück (DE); Malecki, Edith, 26160 Bad Zwischenahn (DE); Korneev, Sergei, 49074 Osnabrück (DE); Werz, Emma, 48465 Schüttdorf (DE); Gall, Karsten, 27616 Lunestedt (DE)
(74) Representative: von Kreisler Selting Werner

(57) **Abstract**

The present invention relates to a method for the isolation and/or identification of known or unknown sequences of nucleic acids (target sequences) optionally marked with reporter groups by base specific hybridation with, essentially, complementary sequences (in the following referred to as sample oligonucleotides, sample sequences or sample nucleic acids), which belong to a library of sequences. Further, the invention relates to nucleolipids used in the method of the invention and a process for the preparation of said nucleolipids.

## Description

The invention relates to a method for the isolation and/or identification of known or unknown sequences of nucleic acids (target sequences) optionally marked with reporter groups by base specific hybridation with, essentially, complementary sequences (in the following referred to as sample oligonucleotides, sample sequences or sample nucleic acids), which belong to a library of sequences. Further, the invention relates to nucleolipids used in the method of the invention and a process for the preparation of said nucleolipids.

With the discovery of gene silencing - also of human genes - by short nucleic acids (s*i*RNA) a new therapeutic principle has evolved. This culminated in the synthesis of the so-called antagomires, short modified oligomers which are built-up from 2'-O-methyl-ß-D-ribonucleosides and which carry at both termini several phosphorothioate internucleotide linkages as well as a cholesterol tag at the 5'-end. Particularly, the latter modification makes the oligomer permeable for the cell membrane. However, it has been reported that already one cholesterol moiety fraughts the oligomer synthesis and its purification and handling with difficulties.

Moreover, cholesterol binds very tightly into bilayer membranes, therewith handicapping an effective transfection of an appending nucleic acid.

For these reasons there is a demand for alternative methods for a less strong and stepped lipophilization of oligonucleotides.

An object of the invention is the provision of lipophilized oligonucleotides and lipophilized building blocks for the oligonucleotide synthesis which overcome the drawbacks of the prior art.

A first embodiment of the present invention is a compound as represented by formula (I) in claim 1. Further, preferred embodiments of the compound of the invention are reflected in the dependent claims.

The substituents referred to in claim 1 of present invention are described in the following in more detail.
R² is H; or
R² is selected from a Mono-phosphate, Di-phosphate, Tri-phosphate or phosphoramidite moiety; or
R² is -Y-X or -Y-L-Y¹- X;
Y and Y¹ are independently from each other a single bond or a functional connecting moiety,
X is a fluorescence marker (FA) and/or a polynucleotide moiety having up to 50 nucleotide residues, preferably 10 to 25 nucleotides, especially a polynucleotide having an antisense or antigen effect;
L is a linker by means of which Y and X are covalently linked together;
R³ and R⁴ represent independently from each other a C₁-C₂₈-alkyl moiety, preferably a C₂-C₂₀-alkyl moiety, more preferably C₈-C₁₈-alkyl moiety, which may optionally be substituted or interrupted by one or more heteroatom(s) and/or functional group(s);or
R³ and R⁴ form a ring having at least 5 members, preferably a ring having 5 to 8 carbon atoms and wherein the ring may be substituted or interrupted by one or more hetero atom(s) and/or functional group(s); or
R³ and R⁴ represent independently from each other a C₁-C₂₈-alkyl moiety, preferably a C₂-C₂₀-alkyl moiety, more preferably C₈-C₁₈-alkyl moiety, substituted with one or more moieties selected from the group -Y-X or -Y-L-Y¹-X; or
R³ and R⁴ represent independently from each other -Y-X or -Y-L-Y¹- X;
R⁵ and R⁶ represent independently from each other a C₁-C₂₈-alkyl moiety, preferably a C₂-C₂₀-alkyl moiety, more preferably C₈-C₁₈-alkyl moiety, which may optionally be substituted or interrupted by one or more heteroatom(s) and/or functional group(s); or
R⁵ and R⁶ represent independently from each other a C₁-C₂₈-alkyl moiety, preferably a C₂-C₂₀-alkyl moiety, more preferably C₈-C₁₈-alkyl moiety, substituted with one or more moieties selected from the group -Y-X or -Y-L-Y¹-X; or
R⁵ and R⁶ form a ring having at least 5 members, preferably a ring having 5 to 18 carbon atoms and wherein the ring may be substituted or interrupted by one or more hetero atom(s) and/or functional group(s);
and/or one or more moieties selected from the group -Y-X or -Y-L-Y¹- X;
R⁵ and R⁶ represent independently from each other -Y-X or -Y-L-Y¹- X;
R⁷ is a hydrogen atom or -O-R⁸;
R⁸ is H or C₁-C₂₈ chain, preferably a C₂-C₂₀ chain, more preferably C₈-C₁₈ chain, which may be branched or linear and which may be saturated or unsaturated and which may optionally be interrupted and/or substituted by one or more hetero atom(s) (Het1) and/or functional group(s)(G1); or
R⁸ is -Y-X or -Y-L-Y¹- X;

R¹⁰, R¹¹, R¹², R¹³, R¹⁴, R¹⁶, R¹⁷, R¹⁹, R²³,R²⁴, R²⁶, R²⁷, R²⁸, R³⁰, R³¹, R³², R³³R³⁴, R³⁵, R³⁸, R³⁹ and R⁴⁰ are independently selected from H or
a C₁-C₅₀ chain, preferably a C₂-C₃₀ chain, more preferably C₈-C₁₈ chain, which may be branched or linear and which may be saturated or unsaturated and which may optionally be interrupted and/or substituted by one or more hetero atom(s)
(Het1) and/or functional group(s)(G1); or
a C₃-C₂₈ moiety, preferably C₅-C₂₀ moiety, more preferably C₈-C₁₈ moiety, which comprises at least one cyclic structure and which may be saturated or unsaturated and which may optionally be interrupted and/or substituted by one or more hetero atom(s) (Het1) and functional group(s)(G1);

R¹⁵, R¹⁸, R²¹, R²², R²⁵, R³⁶ and R³⁷ are independently selected from a C₁-C₅₀ chain, preferably a C₂-C₃₀ chain, more preferably C₈-C₁₈ chain, which may be branched or linear and which may be saturated or unsaturated and which may optionally be interrupted and/or substituted by one or more hetero atom(s) (Het1) and/or functional group(s)(G1); or
a C₃-C₂₈ moiety, preferably C₅-C₂₀ moiety, more preferably C₈-C₁₈ moiety, which comprises at least one cyclic structure and which may be saturated or unsaturated and which may optionally be interrupted and/or substituted by one or more hetero atom(s) (Het1) and functional group(s)(G1);

R²⁰ and R⁴¹ are selected from H, Cl, Br, I, CH₃, C₂-₅₀ chain which may be branched or linear and which may be saturated or unsaturated and which may optionally be interrupted and/or substituted by one or more hetero atom(s) (Het1) and/or functional group(s)(G1); or
a C₃-C₂₈ moiety, preferably C₅-C₂₀ moiety, more preferably C₈-C₁₈ moiety, which comprises at least one cyclic structure and which may be saturated or unsaturated and which may optionally be interrupted and/or substituted by one or more hetero atom(s) (Het1) and functional group(s)(G1); or -O-C₁-₂₈-alkyl, - S-C₁₋₂₈-alkyl, -NR⁴²R⁴³ with R⁴² and R⁴³ independently being H or a C₁₋₂₈-alkyl;
R³⁴ = H or CH₃;
R⁴⁴ is selected from H, F, Cl, Br and I;
Z is O or S; and
A is CH or N.

In a preferred embodiment substituents R¹², R¹⁶, R¹⁷, R¹⁹, R³⁰ and R³⁵ are a linear or branched chain comprising 1 to 50 carbon, preferably 2 to 30 carbon, which may be interrupted and/or substituted by one or more hetero atom(s) (Het1) and/or functional group(s) (G1). Preferably, R¹², R¹⁶, R¹⁷, R¹⁹, R³⁰ and R³⁵ are selected from a linear or branched chain comprising 2 to 40, more preferably 3 to 30, especially 4 to 28 or 6 to 20 or 8 to 16 carbon atoms. In one aspect of the invention R¹², R¹⁶, R¹⁷, R¹⁹, R³⁰ and R³⁵ are a linear or branched C₁-C₂₈-alkyl, preferably C₂-C₂₀-alkyl, more preferably C₄-C₂₀-alkyl or C₆-C₁₈-alkyl, especially C₈-C₁₆-alkyl which may be substituted or unsubstituted. In a further aspect of the invention the carbon chain is interrupted by one or more hetero atom(s) (Het1) wherein the Het1 is preferably selected from O, S and N, more preferably selected from O or N. In one aspect the substituents R¹², R¹⁶, R¹⁷, R¹⁹, R³⁰ and R³⁵ are interrupted by up to 3 hetero atom(s) (Het1), preferably 1 or 2 hetero atoms such as O. In a further aspect of the invention the carbon chain of substituent R¹², R¹⁶, R¹⁷, R¹⁹, R³⁰ and R³⁵ are interrupted by nitrogen which preferably further branches the chain. An exemplary embodiment of this type of substituent is reflected in the following formula: 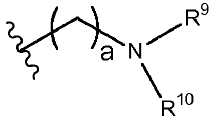
wherein R⁹ and R^{9'} are independently selected from a C₁ to C₃₀ chain which can be saturated or unsaturated, preferably a C₁ to C₃₀ alkyl, further preferably C₄ to C₂₄ alkyl, more preferably C₈ to C₂₂ alkyl and especially C₁₂ to C₁₈ alkyl; or a C₂ to C₃₀ chain having one or more carbon-carbon double and/or carbon-carbon triple bond(s); and
"a" is an integer ranging from 1 to 20, preferably 2 to 18, more preferably 3 to 12 or 4 to 8. However, the linking moiety which links the nitrogen atom with substituents R⁹ and R^{9'} to the base moiety can also be a unsaturated carbon chain having one 2 to 20 carbon atoms and one or more carbon- carbon double and or carbon-carbon triple bonds. The exemplary substituent of the following formula: 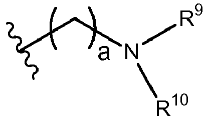 can be synthesized by various synthetic routes. Scheme 7 shows exemplary synthetic route for precursors which can be attached to the base moiety to form the compound of the present invention.

Further, R¹ is preferably a C₂ to C₄₀ chain which is unsaturated, more preferably a C₈ to C₂₈ chain which is unsaturated, In one embodiment of the invention R¹², R¹⁶, R¹⁷, R¹⁹, R³⁰ and R³⁵ comprise one or more carbon-carbon double bond(s) and/or one or more carbon-carbon triple bond(s). In a particular preferred embodiment R¹², R¹⁶, R¹⁷, R¹⁹, R³⁰ and R³⁵ comprise two or more, especially 2 to 6, such as 2 to 4 carbon-carbon double bonds.

In a specially preferred embodiment the substituents are derived from nature. Suitable naturally derived substituents have a structure derived from terpenes. When terpenes are chemically modified such as by oxidation or rearrangement of the carbon skeleton, the resulting compounds are generally referred to as terpenoids. In a preferred embodiment R¹², R¹⁶, R¹⁷, R¹⁹, R³⁰ and R³⁵ is a cyclic or alicyclic terpenoid, preferably a terpenoid having 8 to 36 carbon atoms.

The terpenes are preferably selected from monoterpenes, sesquiterpenes, diterpenes, sesterterpenes, triterpenes and sesquaterpenes.

Suitable monoterpenes or monoterpenoids which can be acyclic or cyclic are selected from the group consisting of geraniol, limonene, pinen, bornylen, nerol.

Suitable sesquiterpenes sesquiterpenoids which can be acyclic or cyclic may inter alia be selected from farnesol.

Suitable sesterterpenes or sesterterpenoids are inter alial selected from geranylfarnesol.

Suitable diterpenes or diterpenoids can be selected from the group consisting of abietic acid, aphidicolin, cafestol, cembrene, ferruginol, forskolin, guanacastepene A, kahweol, labdane, lagochilin, sclarene, stemarene, steviol, taxadiene (precursor of taxol), tiamulin, geranylgeraniol and phytol.

According to a especially preferred embodiment of the invention R¹², R¹⁶, R¹⁷, R¹⁹, R³⁰ and R³⁵ is selected from the group consisting of geranyl, farnesyl, neryl and phythyl.

According to a further alternative aspect R¹², R¹⁶, R¹⁷, R¹⁹, R³⁰ and R³⁵ are H or C₃-C₂₈ chain which may be branched or linear and which may be saturated or unsaturated and which may optionally be interrupted and/or substituted by one or more hetero atom(s) (Het1) and/or functional group(s)(G1); or
R¹², R¹⁶, R¹⁷, R¹⁹, R³⁰ and R³⁵ are a C₁-C₂₈ moiety which comprises at least one cyclic structure and which may be saturated or unsaturated and which may optionally be interrupted and/or substituted by one or more hetero atom(s) (Het1) and functional group(s)(G1);

According to a preferred embodiment R¹², R¹⁶, R¹⁷, R¹⁹, R³⁰ and R³⁵ are selected from H, 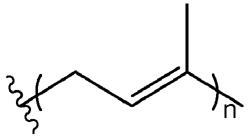 and 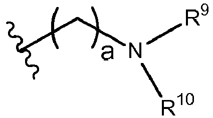 and substituted or unsubstituted cyclic terpene moieties,
wherein
R⁹ and R^{9'} are independently selected from C₁ to C₃₀ alkyl,
n is an integer ranging 1 to 4, preferably n is 1 or 2; and
a is an integer ranging from 1 to 20, preferably 2 to 18

Preferably, Y and Y¹ is are functional connecting groups which are independently selected from a group consisting of carboxylic acid ester, carboxylic acid amides, urethane, ether, amino group, thioester, thioamides, thioether and phosphate ester.

According to preferred embodiment the hetero atom(s) Het1 is selected from O, S and NH.

Further, preferably the functional group(s) (G1) are selected from ester, amide, carboxylic acid, thioester, thioamides and thioether.

In a further aspect of the invention linker L is a moiety comprising 1 to 30 carbon atoms which can be saturated or unsaturated, cyclic or alicyclic, branched or unbranched and which may be substituted or interrupted by heteroatoms.

Preferably, linker L is selected from C₂ to C₂₀alkandiyls, preferably selected from ethylene or propylene.

In a further aspect of the invention linker L is selected from a single bond or a saturated or unsaturated moiety having 1 to 30, preferably 2 to 20 carbon atoms, more preferably a carbon chain which may be substituted and/or interrupted by one or more functional groups selected from carboxylic acid ester, phosphate ester, carboxylic acid amides, urethane, ether and amine groups. L may also comprise cyclic moieties.

According to a preferred embodiment linker L is selected from a single bond; alkandiyl, preferably C₁-C₂₀-alkandiyl; alkendiyl, preferably a C₂-C₂₀-alkendiyl; alkyndiyl, preferably a C₂-C₂₀-alkyndiyl; aryl moiety, aralkyl moiety and herterocyclic moiety.

Preferably, the alkandiyl represents a straight-chain or branched-chain alkandiyl group bound by two different carbon atoms to the molecule, it preferably represents a straight-chain or branched-chain C₁₋₁₂ alkandiyl, particularly preferably represents a straight-chain or branched-chain C₁₋₆ alkandiyl; for example, methandiyl (--CH₂--), 1,2-ethanediyl (--CH₂-CH₂--), 1,1-ethanediyl ((-CH(CH₃)--), 1,1-, 1,2-, 1,3-propanediyl and 1,1-, 1,2-, 1,3-, 1,4-butanediyl, with particular preference given to methandiyl, 1,1-ethanediyl, 1,2-ethanediyl, 1,3-propanediyl, 1,4-butanediyl.

Further, preferably the alkendiyl represents a straight-chain or branched-chain alkendiyl group bound by two different carbon atoms to the molecule, it preferably represents a straight-chain or branched-chain C₂₋₆ alkendiyl; for example, --CH=CH--, --CH=C(CH₃)--, --CH=CH-CH₂--, --C(CH₃)=CH-CH₂--,-CH=C(CH₃)--CH₂--, --CH=CH-C(CH₃)H--, --CH=CH-CH=CH--, --C(CH₃)=CH-CH=CH--, --CH=C(CH₃)-CH=CH--, with particular preference given to --CH=CH-CH₂--, --CH=CH-CH=CH--.

The aryl moiety preferably represents an aromatic hydrocarbon group, preferably a C₆₋₁₀ aromatic hydrocarbon group; for example phenyl, naphthyl, especially phenyl which may optionally be substituted.

Aralkyl moiety denotes an "Aryl" bound to an "Alkyl" and represents, for example benzyl, α-methylbenzyl, 2-phenylethyl, α,α-dimethylbenzyl, especially benzyl.

Heterocyclic moiety represents a saturated, partly saturated or aromatic ring system containing at least one hetero atom. Preferably, heterocycles consist of 3 to 11 ring atoms of which 1-3 ring atoms are hetero atoms. Heterocycles may be present as a single ring system or as bicyclic or tricyclic ring systems; preferably as single ring system or as benz-annelated ring system. Bicyclic or tricyclic ring systems may be formed by annelation of two or more rings, by a bridging atom, e.g. oxygen, sulfur, nitrogen or by a bridging group, e.g. alkandiyl or alkenediyl. A Heterocycle may be substituted by one or more substituents selected from the group consisting of oxo (=O), halogen, nitro, cyano, alkyl, alkoxy, alkoxyalkyl, alkoxycarbonyl, alkoxycarbonylalkyl, halogenalkyl, aryl, aryloxy, arylalkyl. Examples of heterocyclic moieties are: pyrrole, pyrroline, pyrrolidine, pyrazole, pyrazoline, pyrazolidine, imidazole, imidazoline, imidazolidine, triazole, triazoline, triazolidine, tetrazole, furane, dihydrofurane, tetrahydrofurane, furazane (oxadiazole), dioxolane, thiophene, dihydrothiophene, tetrahydrothiophene, oxazole, oxazoline, oxazolidine, isoxazole, isoxazoline, isoxazolidine, thiazole, thiazoline, thiazlolidine, isothiazole, istothiazoline, isothiazolidine, thiadiazole, thiadiazoline, thiadiazolidine, pyridine, piperidine, pyridazine, pyrazine, piperazine, triazine, pyrane, tetrahydropyrane, thiopyrane, tetrahydrothiopyrane, oxazine, thiazine, dioxine, morpholine, purine, pterine, and the corresponding benz-annelated heterocycles, e.g. indole, isoindole, cumarine, cumaronecinoline, isochinoline, cinnoline and the like.

Hetero atoms" are atoms other than carbon and hydrogen, preferably nitrogen (N), oxygen (O) or sulfur (S).

In a preferred embodiment of the present invention linker L is selected from the group consisting of a single bond and a C₁-C₁₀ alkandiyl, preferably a C₂-C₆-alkandiyl, especially ethan-1,2-diyl (ethylene) or propan-1,2-diyl or propan-1,3-diyl.

According to an alternative embodiment X is a fluorescence marker which is selected from the group consisting of fluorescein isothiocyanate (FITC), phycoerythrin, rhodamide and 2-aminopyridine, carbocyamine dyes and bodipy dyes.

According to a further alternative embodiment X is a polynucleotide moiety having up to 50 nucleotide residues, preferably 10 to 25 nucleotides, especially a polynucleotide having an antisense or antigen effect wherein the polynucleotide residue has preferably been coupled via a phosphoamidite precursor.

According to a preferred aspect of the invention the compound of the present invention and represented in formula (I) of claim 1 comprises one or more, preferably two or more, substituents having a C₆ to C₃₀ moiety, more preferably a C₁₀ to C₂₄ moiety which is saturated or unsaturated.

According to a further preferred aspect of the invention the compound of the present invention and represented in formula (I) of claim 1 comprises one or more, preferably two or more, C₆ to C₃₀ chains, more preferably a C₁₀ to C₂₄ chain which is saturated or preferably unsaturated. The compound of the invention preferably comprises one or more, preferably two or more, carbon chains having 6 to 30 carbon atoms wherein the chains comprise one or more, especially preferred two or more, unsaturated carbon-carbon bonds, in particular carbon-carbon double bonds. The chains may be branched or unbranched. Preferably the carbon chains one or no substituents, especially substituents comprising hetero atoms, such as oxygen, sulfur or nitrogen, and more preferably the carbon chains are interrupted by one or no functional group.

It has been found that the compounds of the invention demonstrate a higher permeability for cell membranes. Due to the pharmacological acitivity of the compounds of the invention a further embodiment of the invention refers to a pharmaceutical composition comprising the compound of the invention.

Surprisingly it has been found that the hydroxyl functional lipophilic precursor (such as the amino alcohols reflected in Scheme 7 and 8 can be selectively reacted with the unsubstituted nitrogen atom of the base moiety by a Mitsunobu reaction. This reaction is carried out by first protecting any hydroxyl groups which may be present in the nucleotide.

A further embodiment of the invention is a process for preparing a compound represented by formula (I) 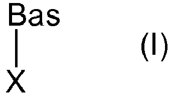 wherein the introduction of a carbon containing substituent as defined in the compound of the present invention to the H-containing nitrogen ring atom, if present, comprises the following steps:
a) providing a compound of formula (I) wherein nitrogen ring atoms bonded to H are present and introducing protecting groups for hydroxyl groups, if present
b) converting an alcohol group containing carbon containing substituent in a Mitsunobu type reaction with the compound of step a) and
c) optionally, removing the protecting groups.

The Mitsunobu reaction is generally carried out by reacting the alcohol and the nucleotide derivative which comprises the unsubstituted ring nitrogen atom in the presence of triphenylphosphine and diisopropylazo dicarboxylate (DIAD).

Preferably, the carbon containing substituent having a hydroxyl group is selected from the group consisting of nerol, phythol, abietol, eicosapentaenol and docosahexaenol.

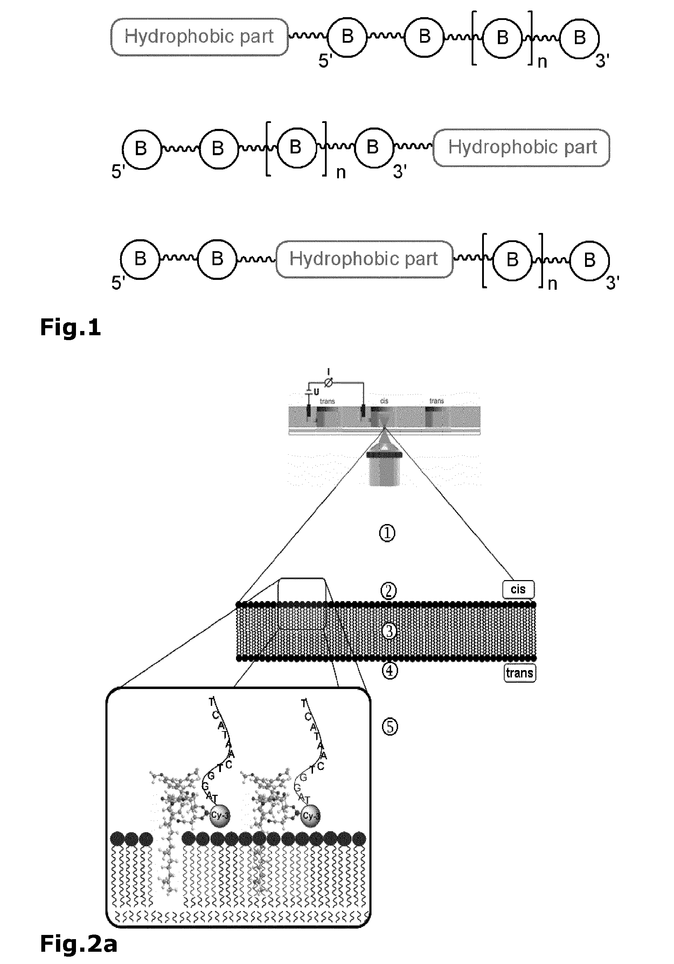

In an aspect of the invention a series of (i) base-alkylated 2'-deoxyinosine- and - thymidine as well as of (ii) sugar- and/or base-alkylated uridine- and 5-methyluridine cyanoethyl phosphoramidites are provided which can be used for the preparation of 5'-lipophilized oligonucleotides. Principally, phosphoramidites of the first group can be incorporated at each position of a growing nucleic acid chain using conventional solid-phase synthesis, so that the oligonucleotide can be hydrophobized at each predetermined locus (Figure 1). Phosphoramidites of the second group can be appended as 5'-terminators to a nucleic acid chain.

The synthesis of the first group (i) of nucleolipid phosphoramidites positioning of the lipophilic side chain can be performed at a nucleobase atom which is involved in *Watson-Crick* base pairing so that the resulting DNA building blocks are pure hydrophobization tools with a basic nucleoside structure. As side chains acyclic mono- and sesquiterpenes, particularly geranyl-, farnesyl- and other residues are preferably chosen because such residues are used in post-translational prenylation of various proteins in order to embed them within biological membranes. Further, it has been found that for the hydrophobization of the various base moieties either direct alkylation with alkyl halogenides or *Mitsunobu* reactions can be performed.

For the synthesis of the second group (ii) of nucleolipid phosphoramidites the lipid moieties can be introduced into the glyconic part of the nucleoside and/or into the base moiety for example via ketalization.

One of the major drawbacks of many chemotherapeutics is their insufficient penetration through cell membranes as well as the crossing of the blood-brain barrier due to their high hydrophilicity. This is particularly true for antisense and antigene oligonucleotides. One method to overcome these problems is the introduction of lipophilic residues to the drug in order to render them hydrophobic and to improve their pharmacokinetics. In the case of low-molecular-weight drugs this kind of chemical modification is heading for the fulfilment of *'Lipinski's Rule of Five'.* The rule describes molecular properties important for a drug's pharmacokinetics in the human body, including their absorption, distribution, metabolism, and excretion and is important for drug development where a pharmacologically active lead structure is optimized stepwise for increased activity and selectivity. One part of the rule concerns the drug's partition coefficient (log*P* between *n*-octanol and water) within the range of -0.4 and +5.5. In another aspect, the present invention further refers to the synthesis of a series of single- and double-chained lipids carrying different functional groups. Via these functional groups such as halogene, carboxylic ester, carboxylic acid, hydroxyl, ammonium, and alkine groups, the lipid residue can be introduced into chemotherapeutics such as nucleoside antimetabolites and others as well as into canonical nucleosides. A further embodiment of the invention refers to a process for preparing the compounds represented by formula (I) via a Mitsunobu type reaction. The compounds of the invention may be used in various fields.

Besides the applications in medicinal chemistry also other applications in nucleic acid analytics (detection and isolation) can be performed. The analysis of genes or gene segments is currently based on DNA chips. These chips or DNA microarrays are made up of a solid carrier (usually a glass object plate), on which single-stranded DNA molecules with a known sequence are attached in a regular and dense pattern. These DNA chips are i) either produced by direct synthesis on the solid carrier using masks and a photo-lithographic procedure, ii) or prefabricated, and terminally functionalized samples of nucleic acids are chemically attached to activated surfaces by covalent bonds. The DNA analysis may involve multiple steps: i) preparation of the sample (extraction, PCR, etc.), ii) hybridization on the chip, iii) stringend washing, iv) detection, and v) bioinformatic analysis. Both ways of producing DNA chips are afflicted with numerous issues. The first method requires the synthesis of oligonucleotides directly on the carrier, and includes several deprotection reactions and washing steps. This renders a complex method, especially when the array includes a multitude of different nucleic acid samples. In case of the second method, the solid surface - usually a glass plate - has to be activated with functional groups in a complicated manner in order to apply the likewise pre-made functionalized nucleic acids with a known sequence. This spotting is also a challenging procedure and requires special equipment. What follows is a chemical reaction between the ready-made functionalized nucleic acids and the activated functional groups on the surface of the array in order to achieve a covalent bond between the array and the nucleic acid. The present invention refers to a novel DNA chip technology which renounces any chemistry on solid supports by using instead the self-organization and duplex formation of lipid oligonucleotide conjugates at a lipid bilayer water interface.

In an exemplary embodiment of the invention the compound of the invention is a base-alkylated 2'-deoxynucleoside such as 2'-deoxyinosine and 2'-deoxythymidine. Preferably the alkylation/lipophilization is carrid out with terpenoid moieties.

For the preparation of N-geranylated and -farnesylated nucleoterpenes of 2'-deoxyinosine **(1)** and thymidine **(7),** respectively, base-catalyzed alkylation, for example in dimethylformamide with the corresponding terpenyl bromides can be chosen. In order to avoid side reactions such as the O-alkylation of the sugar hydroxyls as well as of the base protecting groups can be used prior to the alkylation. A suitable protecting group for the sugar moiety is the 1,1,3,3-tetraisopropyldisiloxane group. Scheme 1 and Scheme 2 show in one aspect sugar protected derivatives 2 and 8 which are protected by the so-called Markiewicz silyl clamp. This so-called *Markiewicz* silyl clamp [10] can be easily introduced and cleaved off with tetra-N-butylammonium fluoride under mild conditions. Subsequent deprotonation of compound **2** and **8** can be performed under various reaction conditions with respect to solvent (DMF, MeCN) and base (NaH, K₂CO₃).

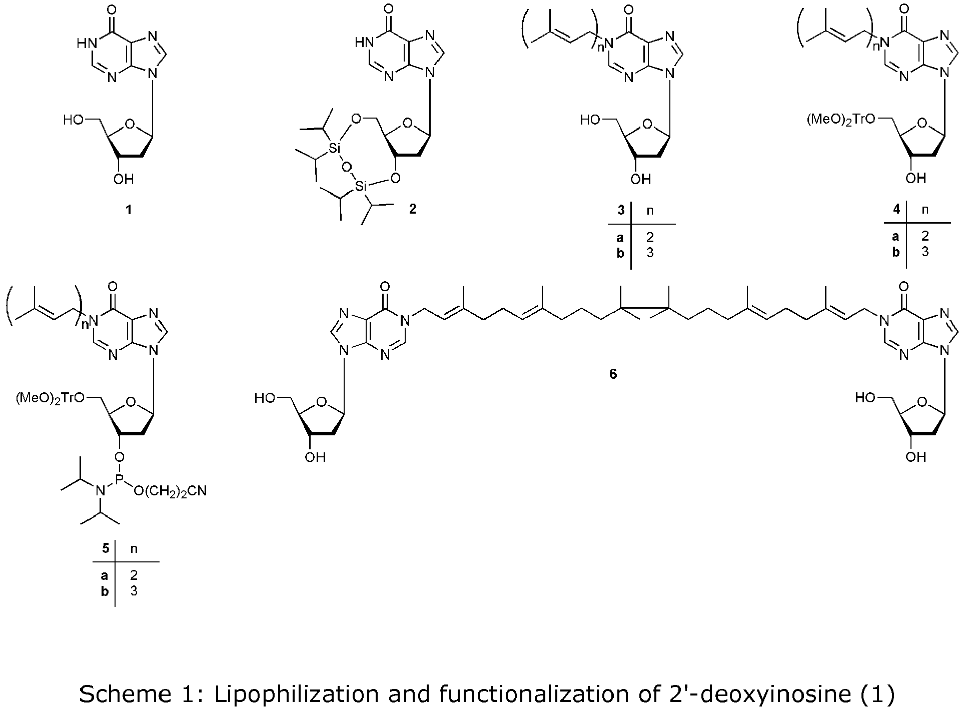

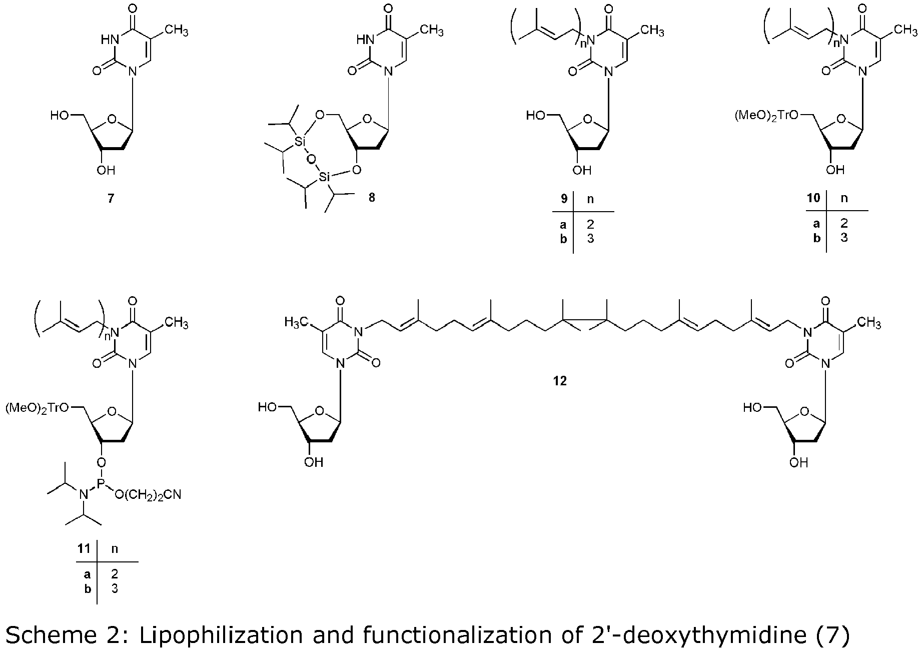

The unprotected 2'-deoxynucleosides **(1** and **7)** can be alkylated with geranyl bromide and farnesyl bromide under alkaline conditions, e.g. with K₂CO₃ in dimethylformamide. For example, in the case of 2'-deoxyinosine **(1)** a reaction time of 24 h at room temp. was sufficient for a moderate yield (50 - 75 %) of the products **3a,b** while for thymidine **(7)** 48 h and 40°C can be used to isolate the corresponding products **9a,b** in comparable yields. The following table (Table 1) shows the lipophilicity of the thymidine derivatives in form of their calculated log*P* values and their retention times in *RP-18* HPLC.

**Table 1. Calculated logP Values and Retention Times [min] of Thymidine Derivatives in RP-18 HPLC (for details, see Exp. Part).**

| Compound | Iog*P* Value | Retention Time t_{R} [min] |
|---|---|---|
| **7** | -1.11 ± 0.49 | 1.89 |
| **9a** | 4.57 ± 0.61 | 3.34 |
| **9b** | 6.60 ± 0.64 | 7.85 |

Compounds **3a,b** and **9a,b** represent synthetic nucleoterpenes of the invention. Dimeric molecules such as **6** and **12** can also be observed as side products.

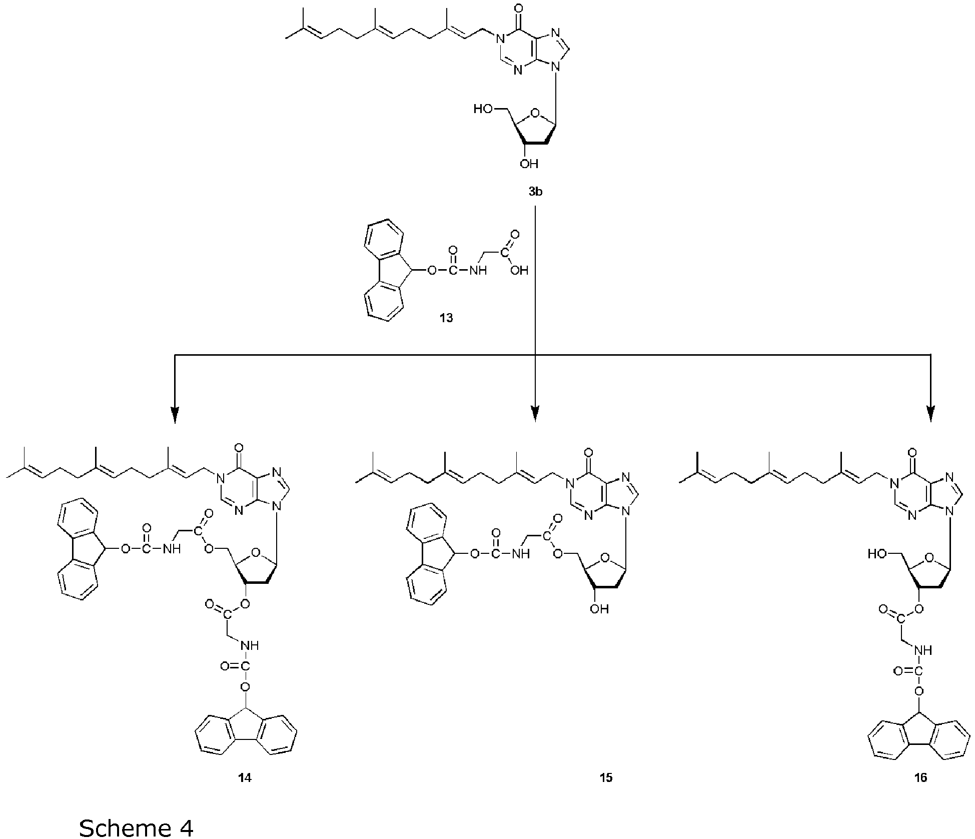

In a further exemplary embodiment nucleoterpenes, such as nucleoterpene 3b, can be labelled with a fluorescence marker (FA) auch as a fluorenyl moiety or Taxas Red. An exemplary reaction scheme for labelling compound 3b is shown in Scheme 4. The nucleoterpene **3b** can be labelled with different dyes, such as (i) with a fluorenyl moiety (Fmoc) via a glycine spacer and (ii) with Texas Red. For this labelling method compound **3b** was reacted at its 5'-hydroxyl with N-[(9H-fluoren-9-yl-methoxy)-carbonyl]-glycine **(13)** using a *Steglich* esterification (DCC, DMAP). TLC analysis showed the formation of three products which could be separated by chromatography. All compounds were characterized by ¹H-, ¹³C-NMR as well as by UV-VIS spectroscopy. The fastest migrating compound was assigned as the 3',5'-di-fluorenylated derivative **14,** the others as the 5'- **(15)** and the 3'- **(16)** labelled compounds.

In a further alternative and exemplary synthesis compound **3b** can be coupled with sulforhodamin-101-sulfonyl chloride *(Texas Red).* After extraction and silica gel chromatography the product **17** (Scheme 5) was obtained as a deep black, amorphous material.

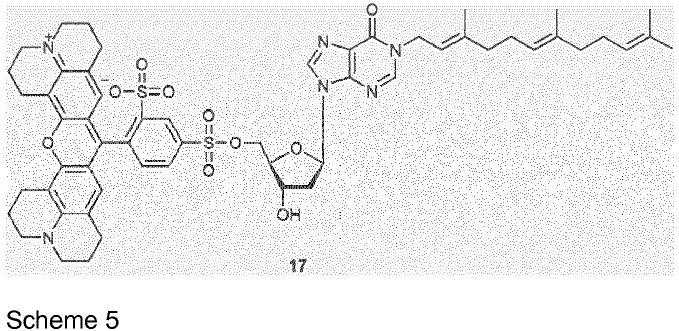

According to a further aspect of the invention lipophilized oligonecleotides can be prepared via the phosphoramidites of the lipophilized nucleotides. As an example the phosphoramidites **5b** and **11a,b** were used to prepare a series of lipophilized oligonucleotides and their insertion into artificial lipid bilayers was studied. The following oligonucleotides were synthesized and characterized by MALDI TOF mass spectrometry.

**Table 2. Sequences and MALDI TOF Data of Oligonucleotides.**

| *Oligonucleotide (sequence, formula no, abbreviation)* | *[M+H]⁺ (calc.)* | *[M+H]⁺ (found)* |
|---|---|---|
| 5'-d(**3b**-Cy3-TAG GTC AAT ACT)-3', **18,** KK1 | 4.671.6 | 4.671.1 |
| 5'-d(**9b**-Cy3-TAG GTC AAT, ACT)-3', **19,** EW1 | 4.660.6 | 4.659.5 |
| 5'-d(**9a**-Cy3-TAG GTC AAT, ACT)-3', **20,** EW2 | 4.592.5 | 4.590.5 |
| 5'-d(**9b**-TAG GTC AAT, ACT)-3', **21,** EW3 | 4.153.0 | 4.152.3 |
| 5'-d(**9b**-ATC CAG TTA TGA)-3', **22,** EW4 | 4.153.0 | 4.152.0 |
| 5'-d(Cy5-AGT ATT GAC CTA)-3', **23,** EW5 | 4.178.1 | 4.178.4 |

The oligonucleotides **18 - 20** contain - besides a nucleoterpene **(3b** or **9a,b) -** an indocarbocyanine dye at the 5'-(n-1) position which was introduced via its phosphoramidite. The oligomers **21** and **22** carry the thymidine terpene **9b** at the 5'-end while the oligomer **23** carries a *Cy5* fluorophore label and is complementary to the oligonucleotide **21** in an antiparallel strand orientation but not to **22.**

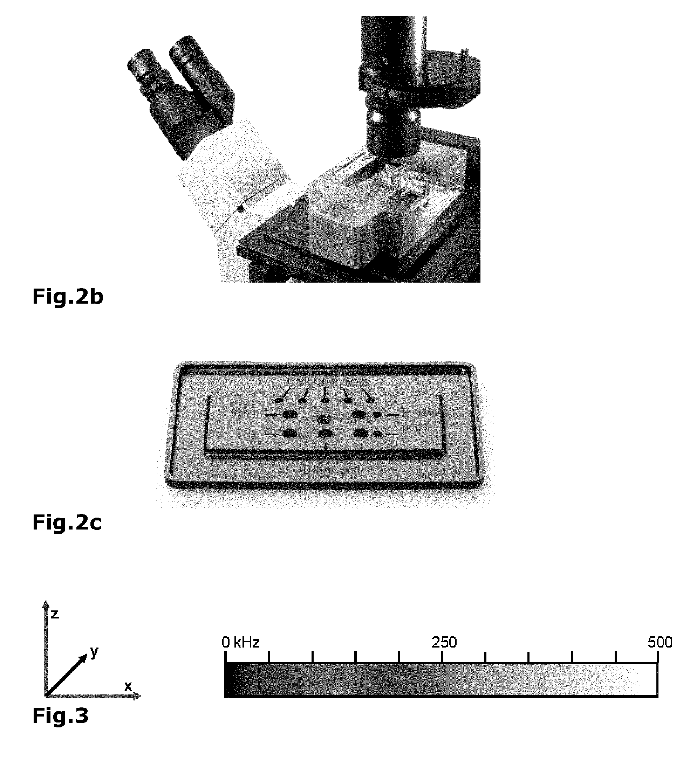

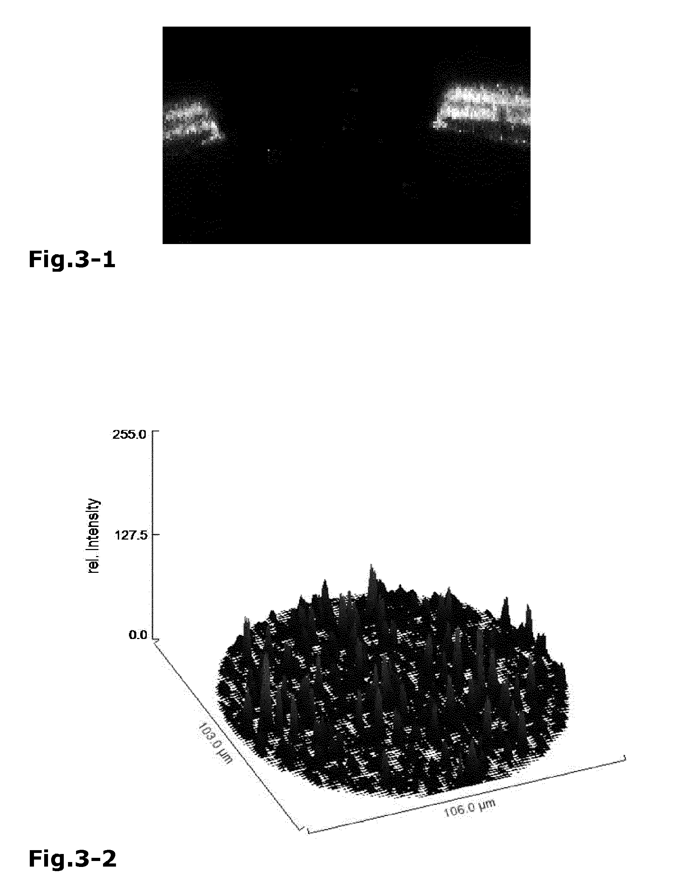

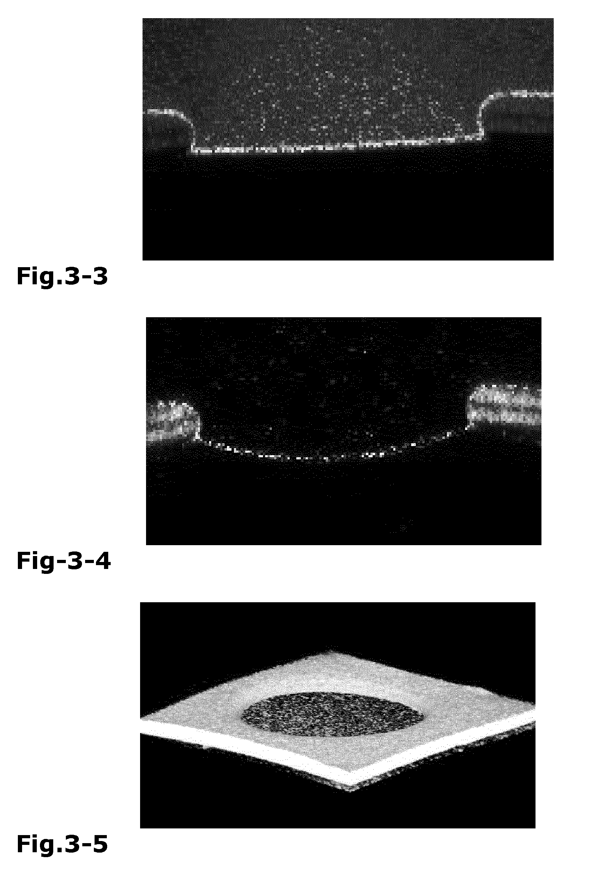

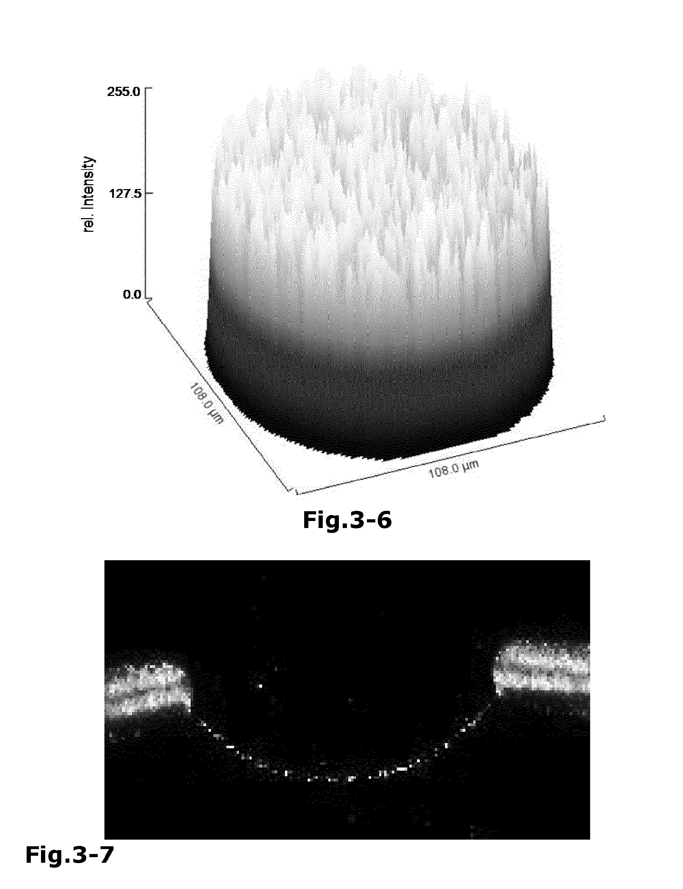

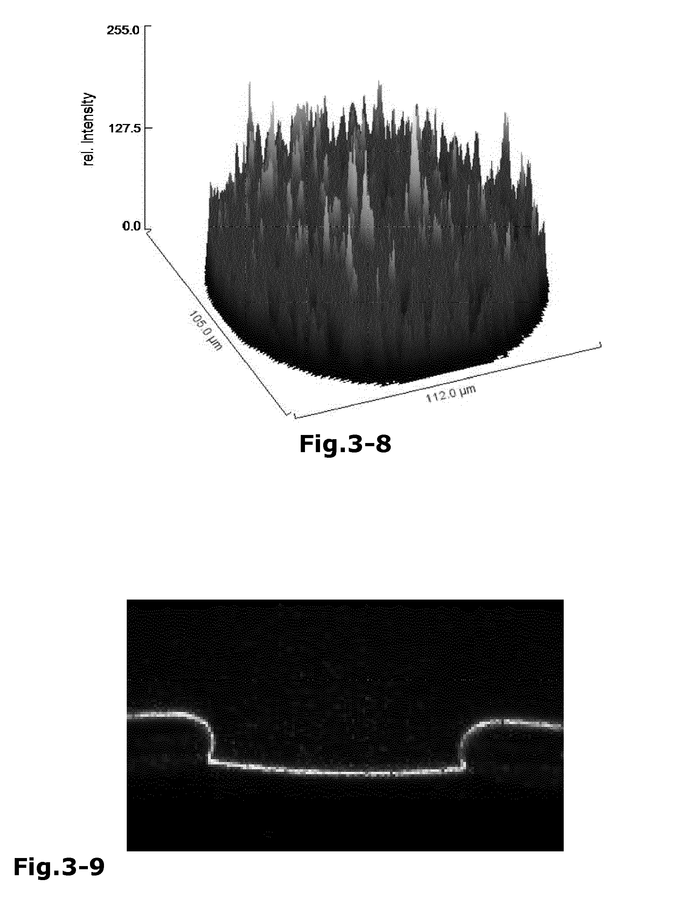

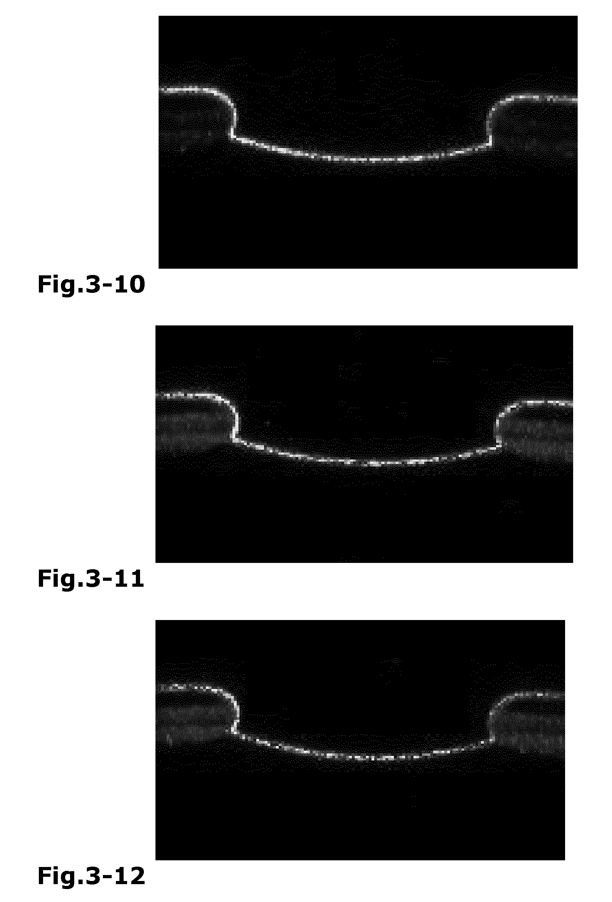

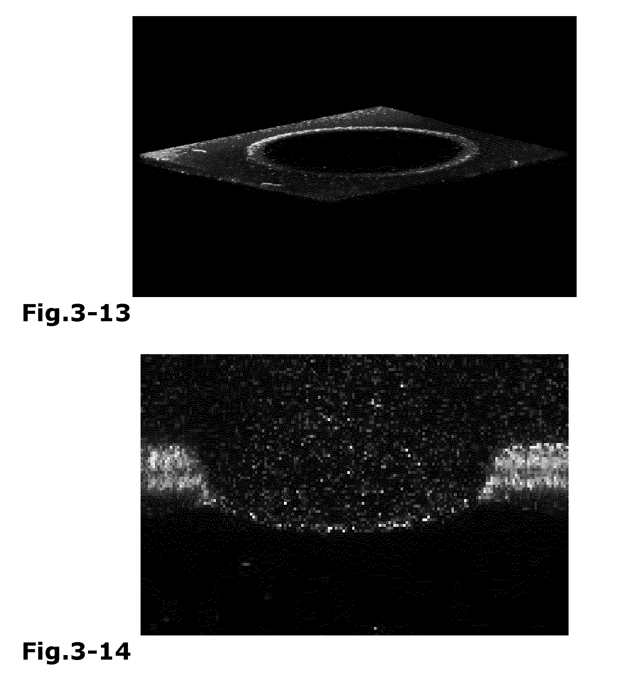

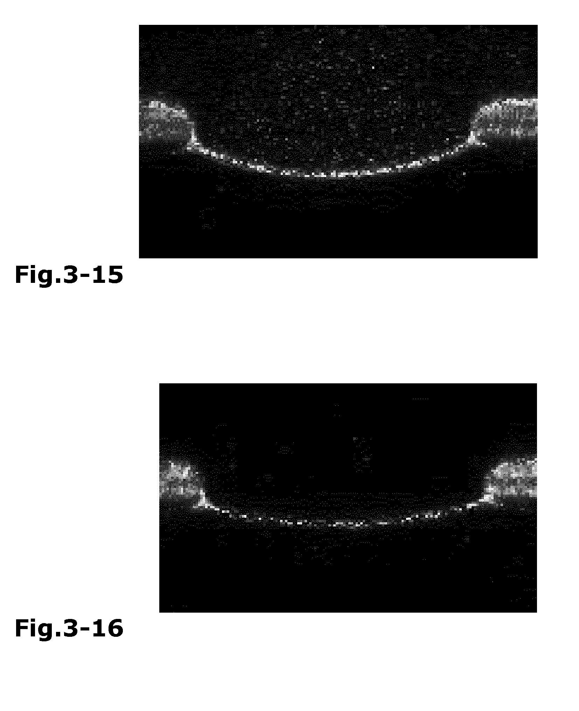

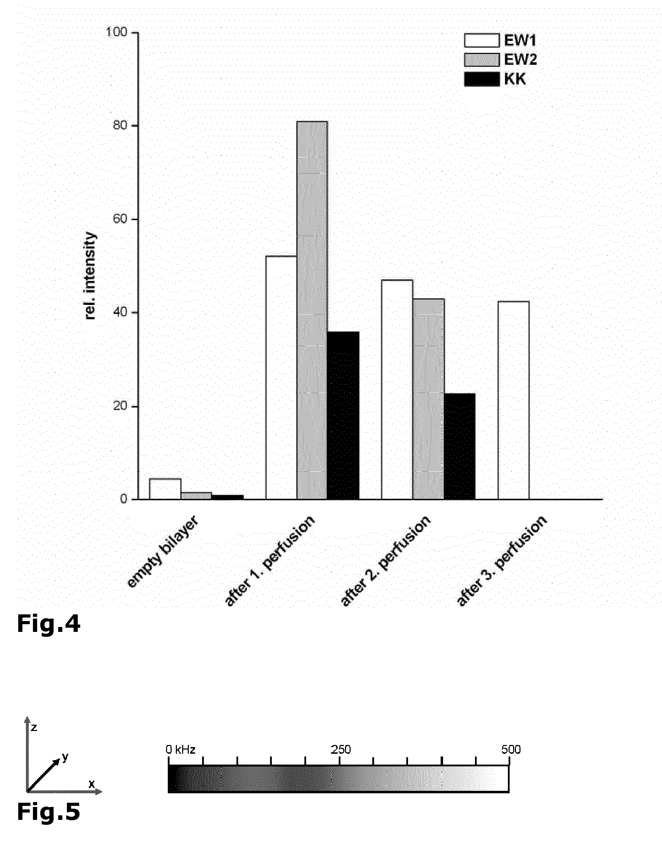

First, the insertion of the oligonucleotides **18 - 20** (KK1, EW1 and EW2) was tested at artificial bilayer membranes composed of 1-palmitoyl-2-oleyl-sn-glycero-3-phosphoethanolamine (POPE) and 1-palmitoyl-2-oleyl-sn-glycero-3-phosphocholine (POPC) (8:2, w/w) in n-decane (10 mg/ml) in a set-up shown in Fig. 2 (see E. Werz, et al. Chemistry & Biodiversity, Vol. 9 2012, 272-281 and A Honigmann, PhD Thesis, University of Osnabrück, Germany, 2010 for detailed construction plans). From *Figures 3* and *4* it can be clearly seen that all *Cy3-*labelled lipo-oligonucleotides are inserted into the lipid bilayer, but to a different extent and with different stability towards perfusion. It is obvious that the oligomer carrying the N(3)-geranyl-thymidine nucleoterpene is inserted to the highest extent, but is washed out by one perfusion already to about 50 %. The oligomers carrying farnesylated nucleosides at their 5'-end are significantly more stable towards perfusion.
Fig. 2-A shows a schematic drawing of the Laser Scanning Microscope, the optical transparent microfluidic bilayer slide, and the lipid bilayer with incorporated double-tailed nucleolipids. The bilayer slide encloses two microfluidic channels (cis and trans) which are separated by a thin medical grade PTFE foil. This foil hosts a central 100 µm aperture which is located 120 µm above the coverslip and thus within the working distance of high NA objectives. It is the only connection between the trans and cis channel. When a lipid solution is painted across the aperture a bilayer is formed spontaneously. Electrodes in the cis and trans channels allow an online monitoring of the bilayer integrity as well as electrophysiological recordings.
Fig. 2-B shows a stage unit of the *"Ionovation Explorer"* from Ionovation GmbH, Osnabrück, Germany,mounted on a standard inverted fluorescence microscope. The computer controlled perfusion unit is a side board and is not shown.
Fig. 2-C shows a *"Ionovation Bilayer Slide";* a disposable, optical transparent microfluidic sample carrier with perfusion capabilities. The "Bilayer Port" gives direct access to the lipid bilayer, while both sides of the bilayer can be perfused via the cis and trans channel. Calibration wells allow optical control experiments when needed.
Figure 3: Protocol of the Insertion of the Oligonucleotides **18 - 20** into an artificial Bilayer.
   Figure 3-1: z-scan of an empty bilayer; both channels (cis and trans) were perfused (30 s, 1.1 ml/min, each)
   Figure 3-2: Pixel-resolved 3D scan of an empty bilayer
   Figure 3-3: z-scan after addition of **18** (4 µl, 50 nM) to the cis channel and 25 min of incubation
   Figure 3-4: z-scan after 1. perfusion of the cis compartment (60 s, 1.1 ml/min)
   Figure 3-5: tilted 3D view onto the bilayer, filled with **18,** after 1. perfusion
   Figure 3-6: pixel-resolved 3D scan of the bilayer, filled with **18,** after 1. Perfusion
   Figure 3-7: z-scan after 2. perfusion of the cis compartment (60 s, 1.1 ml/min
   Figure 3-8: pixel-resolved 3D scan of the bilayer, filled with **18,** after 2. Perfusion
   Figure 3-9: z-scan of the bilayer after addition of **19** (4 µl, 50 nM) to the cis compartment and 25 min of incubation
   Figure 3-10: z-scan of the bilayer after 1. perfusion of the cis compartment (60 s, 1.1 ml/min)
   Figure 3-11: z-scan of the bilayer after 2. perfusion of the cis compartment (60 s, 1.1 ml/min)
   Figure 3-12: z-scan of the bilayer after 3. perfusion of the cis compartment (60 s, 1.1 ml/min)
   Figure 3-13: tilted 3D view onto the empty bilayer
   Figure 3-14: z-scan of the bilayer after addition of **20** (4 µl, 50 nM) and 25 min of incubation
   Figure 3-15: z-scan of the bilayer, filled with **20,** after 1. perfusion of the cis compartment (60 s, 1.1 ml/min)
   Figure 3-16: z-scan of the bilayer after 2. perfusion of the cis compartment (60 s, 1.1 ml/min)
Figure 4:. Relative Bilayer Brightness as a Function of the Perfusion Number.

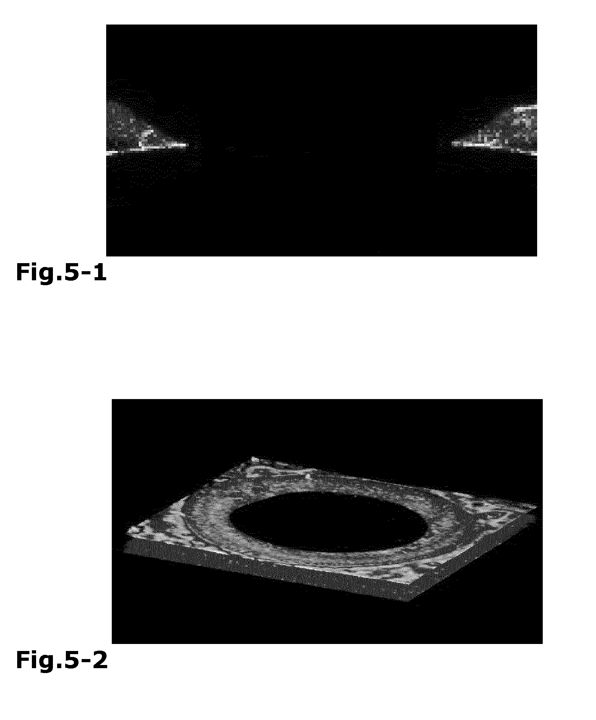

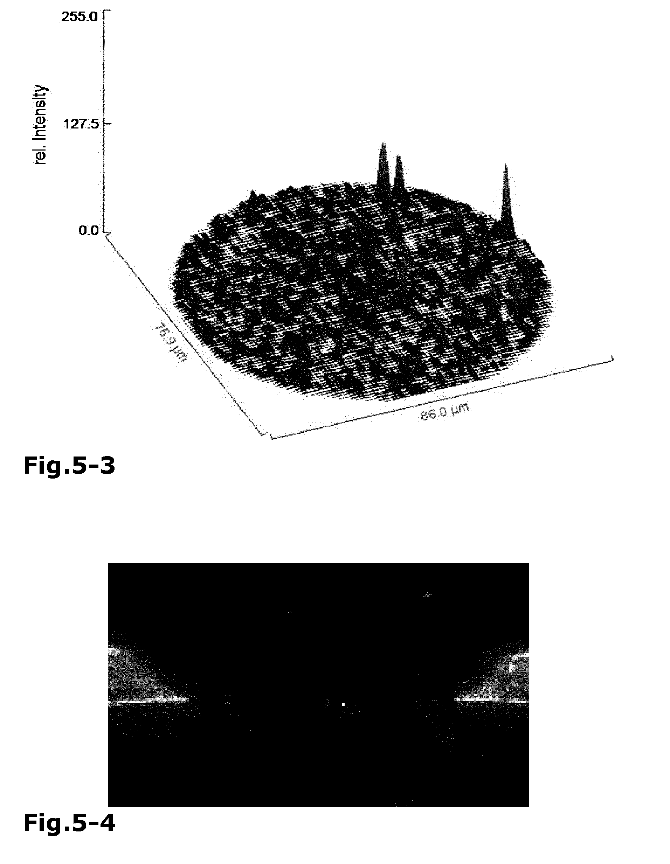

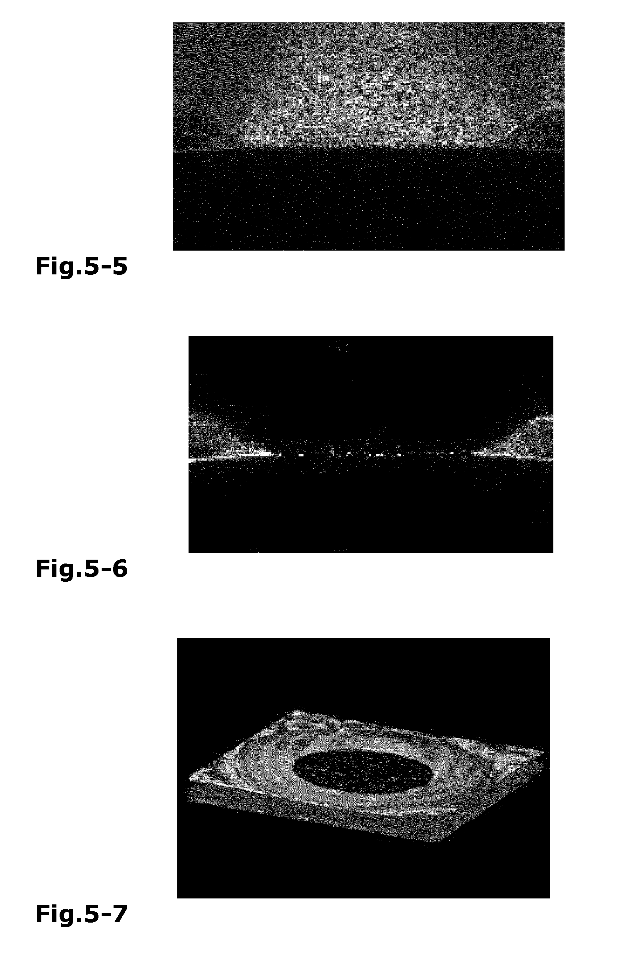

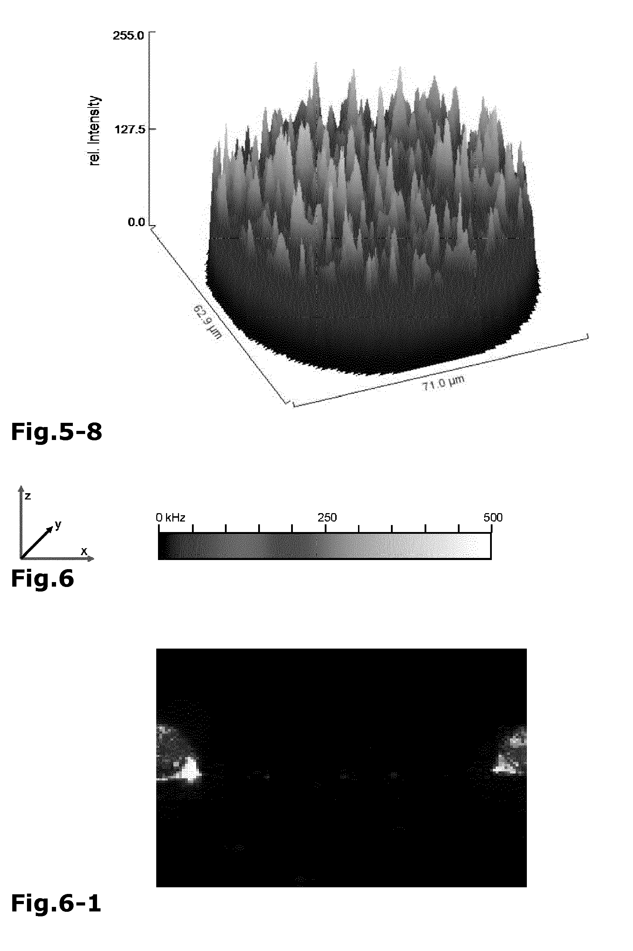

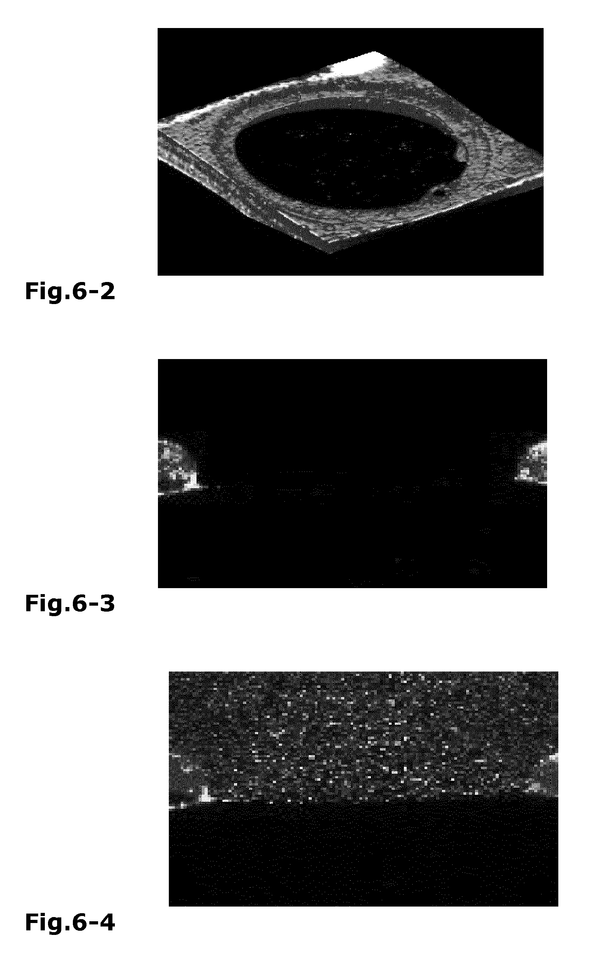

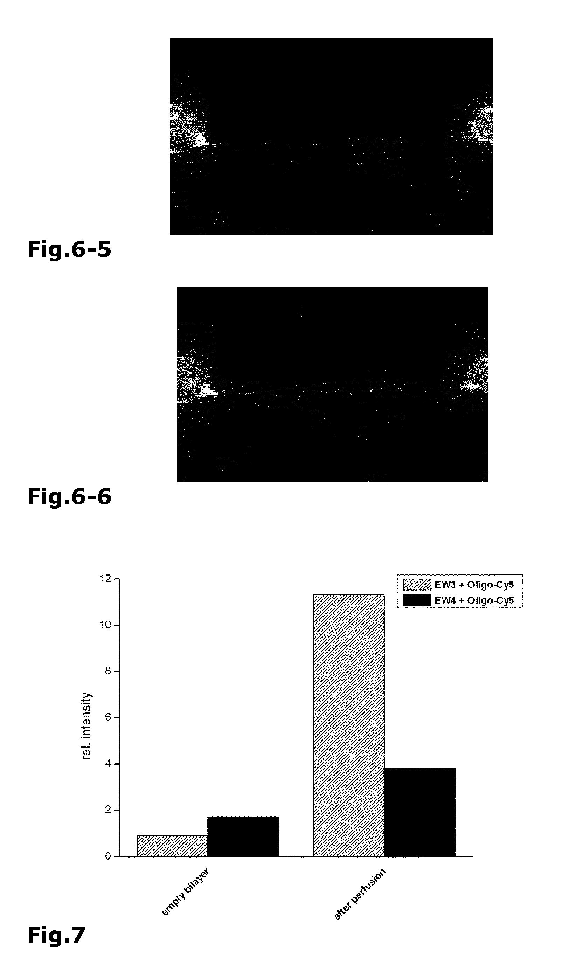

Further, the duplex formation between bilayer-immobilized lipo-oligonucleotides **(21** and **22,** EW3 and EW4) with a Cy5-labelled oligomer **(23,** EW5) which is complementary only to **21** (EW3) but not to **22** (EW4) has been analyzed. Fluorescence microscopy (Figures 5 and 6) clearly proves duplex formation for **21 ● 23** but not for **22 ● 23.**
Figure 5: Chronological protocol of duplex formation of the oligonucleotide EW3 with the complementary, CY3-labelled oligomer EW5 at an artificial lipid bilayer - aq. buffer boundary, followed by a perfusion
   Figure 5-1: z-scan of an empty bilayer
   Figure 5-2: (2) tlted 3D view on an empty bilayer
   Figure 5-3: (3) pixel-resolved 3D scan of an empty bilayer
   Figure 5-4: z-scan after addition of the oligomer EW3 (6 µl, 500 nM) to the cis compartment and 60 min of incubation
   Figure 5-5: z-scan after addition of the Cy-5 - labelled oligomer (6 µl, 50 nM) to the cis compartment and 60 min of incubation
   Figure 5-6: z-scan after perfusion of the cis compartment (30 s, 1.1 ml/min)
   Figure 5-7: tilted 3D view on the bilayer as in 6.
   Figure 5-8: pixel-resolved 3D scan of the bilayer as in 6.
Figure 6: Chronological control experiment with the non-complementary oligonucleotides EW4 and EW5.
   Figure 6-1: z-scan of the empty bilayer
   Figure 6-2: tilted 3D view of the empty bilayer
   Figure 6-3: z-scan after addition of the oligomer EW4 (6 µl, 500 nM) to the cis compartment and 50 min of incubation and perfusion (30 s, 1.1 ml/min)
   Figure 6-4: z-scan after addition of the CY-5-labelled oligonucleotide (6 µl, 50 nM) to the cis compartment and 40 min of incubation
   Figure 6-5: z-scan after perfusion of the cis compartment (30 s, 1.1 ml/min)
   Figure 6-6: z-scan after further incubation for 20 min and two perfusions of the cis compartment (60 s, 1.1 ml/min, each).
   Figure 7: Relative Bilayer Brightness

Furthermore, the diffusion times (T_{D} in ms) of the duplex **21 ● 23** were measured, both without and in the presence of an artificial bilayer (Table 3). For the determination of the free diffusion times the corresponding oligomer duplex solution (50 nM) was diluted so that there was only a single fluorescent molecule in the confocal measuring volume (∼ 1 fl). Each measurement was performed ten-times for 30 s. In order to determine the diffusion times of the lipophilized oligonucleotide duplex **(21 ● 23)** in the presence of a bilayer two measuring positions, one above (in solution but in close proximity to the bilayer), and one within the bilayer, were chosen. Each measurement was performed (i) by recording reference data of a stable, blank bilayer, (ii) after formation of the oligonucleotide duplex and a subsequent 30-min incubation, followed by recording of the data, (iii) recording of further data series after perfusion of the *Bilayer Slide.* Table 3 summarizes the results and show that the diffusion of oligomer **23** as well as of the duplex **21 ● 23** is fast. However, the broad diffusion time distribution of the duplex **21 ● 23** indicates aggregate formation of heterogeneous size. In the close proximity of a stable bilayer the diffusion time increases approximately by a factor of 10. Probably the molecules aggregate, and the aggregates interact partly with the bilayer. The diffusion time of the bilayer-immobilized DNA duplex increases further by a factor of 10.

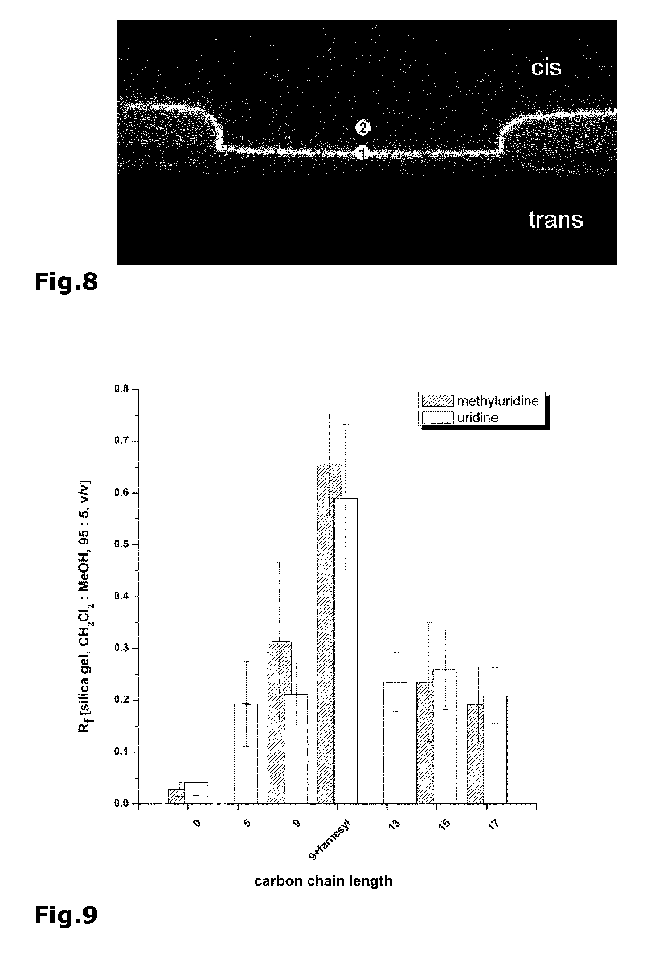

**Table 3. Diffusion times [T_{D} (ms)] of 21 ● 23 without and in the presence of a lipid bilayer. Location: 1, bilayer; 2, solution in close proximity to the bilayer (see Figure 8)**

| sample | position | T_{D} (ms) |
|---|---|---|
| **23** | diffusion (in solution | 0.24 ± 0.1 |
| **21 · 23** | without bilayer) | 0.12 ± 0.1 |
| **21 · 23** | 1 | 26.6 ± 2.0 |
| **21 · 23** | 2 | 2.39 ± 0.3 |

In a further aspect of the invention an exemplary synthetic route to compounds of the invention is demonstrated in Scheme 6. According to a preferred embodiment of the invention the compounds of the invention are lipophilized by ketalization of glyconic moiety. Scheme 6 shows as an example the *hydrophobization of uridine and methyluridine by long-chain ketal groups.* Uridine **(24)** and methyluridine **(29)** can be reacted with symmetrical long-chain ketones in acidic medium (DMF) which leads to the O-2',3'-ketals **25a-e** and **30a-c.** For this purpose two different synthetic routes may be applied (see Exp. Part). The compounds may directly be converted to their phosphoramidites **(26a-e, 31a-c)** or first N(3)-farnesylated and then phosphitylated **(28a-e, 33a-c).**

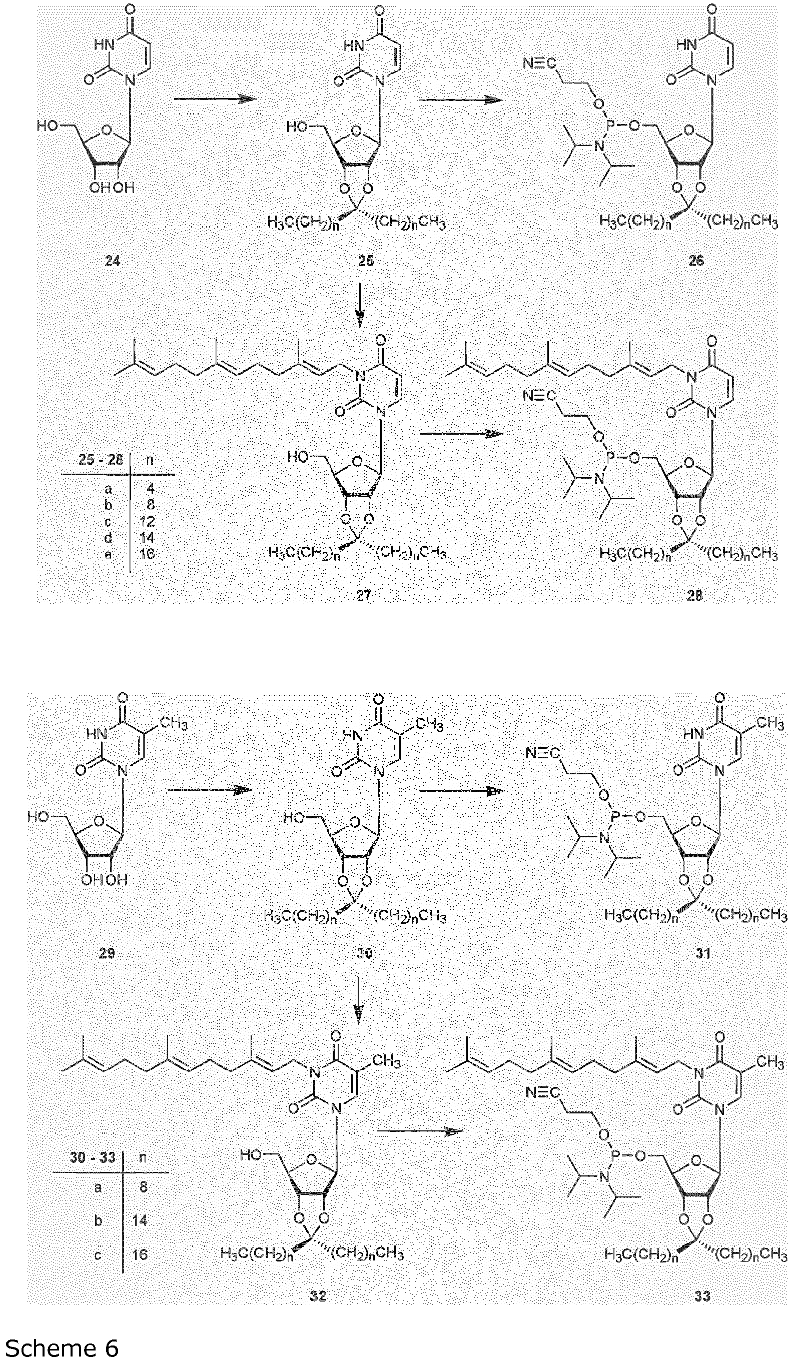

Figure 9 displays the *R*_{f} values of the various uridine- and methyluridine O-2',3'-ketals as a function of the carbon chain length.

Figure 9: *R*_{f} Values of various O-2',3'-ketals.

Scheme 7 shows several synthetic routes for precursors which can be attached to the nucleotides, especially to the base moity of the nucleotide. The funcationalized lipids shown in Cheme 7 can be used to hydrophobize the nucleosides. Prerferably, the compound of the invention comprise double chained lipids. As an example the synthesis of a series of functionalized lipidss carrying two octadecanyl chains is described and reflected in Scheme 7.

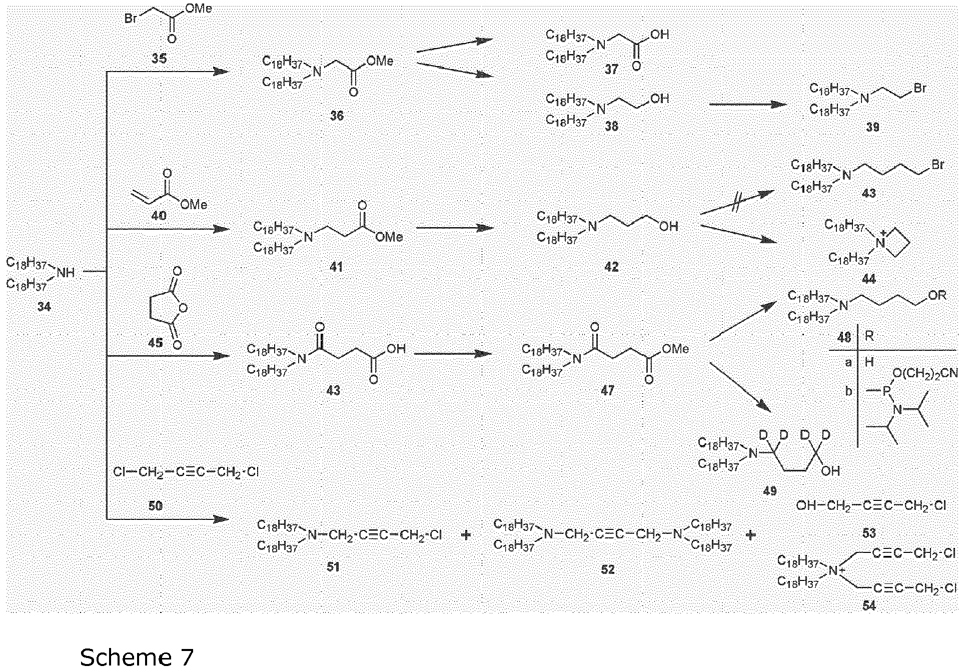

Reaction of dioctadecylamine **(34)** with methyl bromoacetate **(35)** in the presence of dibenzo-[18]-crown-6 leads to the pure ester **3** in almost quantitative yield. This was either saponificated to yield the acid **37** or reduced with LiAlH₄ to give the alcohole **38.** The latter can be submitted to an *Appel* reaction with tetrabromomethane and triphenyl phosphine which leads to the bromide **39** in low yield. In order to extend the spacer between the hydroxyl group and the nitrogen carrying the carbon chains the secondary amine **34** can be reacted with methyl acrylate **(40).** This lead in almost quantitative yield the ester **44** which can further be reduced with LiAlH₄ to give the lipophilic aminopropanol derivative **42.** Subsequent *Appel* bromination to produce the aminobromide **43,** however, was unsuccessful. NMR Spectroscopy revealed the formation of the quaternization product **44,** an N,N-di-alkylated azetidinium bromide. This implies that the low yield in case of bromide **39** is also due to the formation of a quaternization product, namely an N,N-di-alkylated aziridinium bromide.

The amine **34** can be reacted with succinic anhydride **(45)** to give the acid **46.** This was converted to the ester **47** by reaction with dimethyl sulphate in the presence of K₂CO₃. Compound **47** can be then reduced with LiAlH₄ yielding the further extended alcohole **48a** or with LiAlD₄ giving the deuterated lipophilized 4-aminobutanol derivative **49.** It should be noted that this way of labelling of the molecule allow one to introduce four isotope atoms of hydrogen in a single synthetic step which is important for the introduction of low radioactivity labels, such as tritium. Moreover, compound **48a** was phosphitylated to the 2-cyanoethylphosphoramidite **48b** ready for use for a terminal hydrophobization of nucleic acids.

In a further reaction the amine **34** can be alkylated with 1,4-dichlorobut-2-ine **(50)** in the presence of Na₂CO₃ in benzene. This leads in 61% yield of the alkine derivative **51,** besides the by-products **52 - 54,** each in low yield.

Further, single-chained lipids as precursors can be synthesized as reflected in Scheme 8. As an example, the preparation of single-chained lipids with terminal functional groups is shown in Scheme 8. Reaction of octadecylamine **(55)** with propargylbromide **(56)** leads in almost quantitative yield the tertiary amine **57.** Reaction of the starting amine **55** with succinic anhydride **(45)** afforded the acid **58** which can further be esterified to the ester **59.** Treatment of the latter with LiAlH₄ (under the same conditions as for the reduction of **47** into **48a)** yielded surprisingly the N-alkylated pyrrolidine **61** instead of the expected alcohol **60.** Reduction of the acid **58** with LiAlH₄ in THF at ambient temperature was attempted, however it has led to a reduction of carboxylic group only, but not of the amide moiety and lead to the amidoalcohole **62** in 82% yield. Increasing of the reaction temperature to 65°C leads to the desired aminoalkohole **60** but only in moderate yield of 23%. Replacement of THF by Et₂O leads to compound **60** in a high yield of 84%. Subsequent reaction of compound **60** with propargyl bromide leads to the alkine **63** in 61% yield.

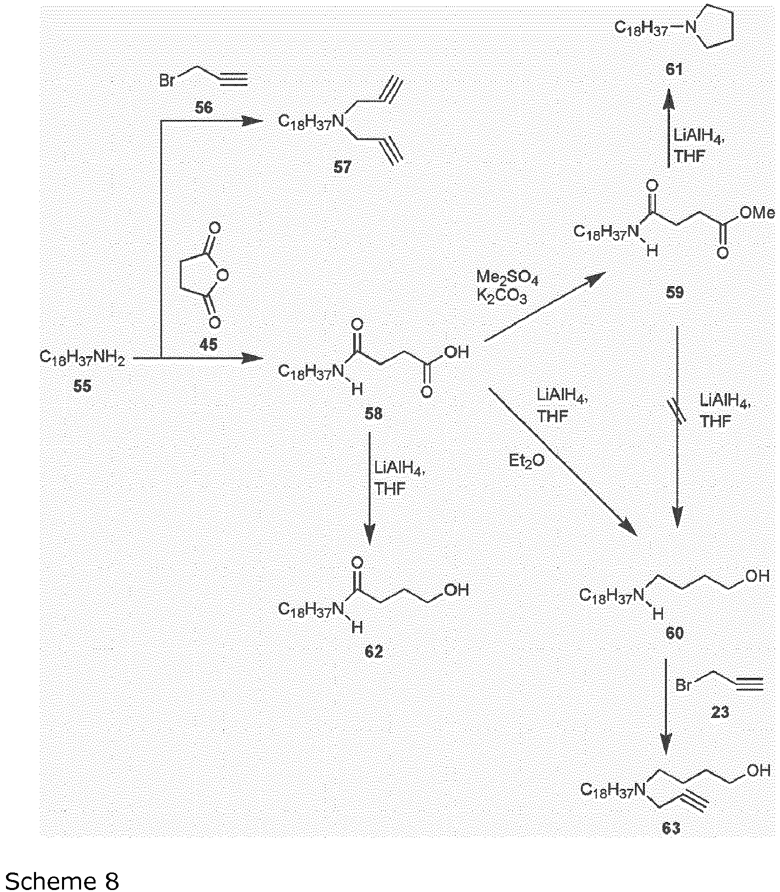

In a further aspect a further synthetic route to lipophilize the nucleoside the Mitsunobu reaction can be used to introduce the lipophilic moieties to the nucleosides. The regioselective introduction of lipophilic hydrocarbon chains into a nucleoside, particularly into a nucleoside with biomedical activity, is a difficult synthetic task. Such lipophilic groups can principally positioned either at the heterocyclic base or at the glyconic moiety and can be introduced by various methods, e.g. by base-catalysed alkylation with alkyl halides.

Some exemplary alkylation reactions of thymidine **(7)** with two of the functionalized lipids described above namely with compounds **42** and **51** are shown in Scheme 9. The reaction of unprotected thymidine with the alkine **51** was performed in DMF/K₂CO₃ (direct alkylation) and leads to the N(3)-alkylated compound **65.** This derivative can be further reacted with an azide in a ruthenium-catalysed variant of the azide-alkyne cycloaddition (RuAAC, *Huisgen-Sharpless-Meldal* [3+2] cycloaddition of azides with internal alkynes). Such reactions are underway. After dimethoxytritylation of **65** the derivative **66** was obtained, ready for further 3'-O-phosphitylation.

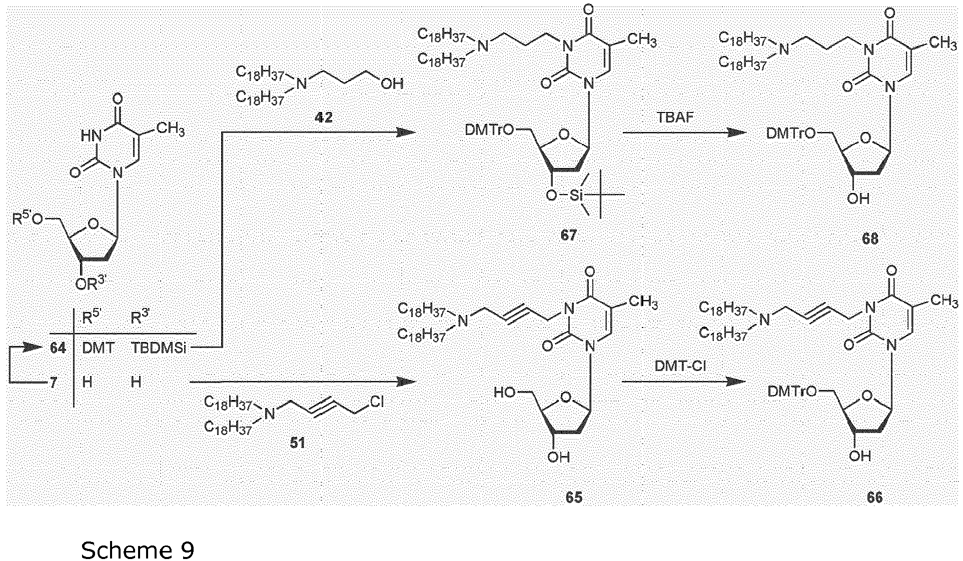

Due to the finding that the direct alkylation of thymidine **(7)** with compound **51** gave only a moderate yield of **65** (46%), next, the 5'-O-DMT-protected thymidine derivative **69 -** prepared from **7 -** was subjected to the alkylation with **51** (Scheme 10). However, the yield of the alkylated product **66** was found to be nearly the same (51%). Therefore, the totally, orthogonal protected derivative **64** was prepared and alkylated. This reaction gave the product **70** in high yield (95%). It could then be deprotected with tetrabutyl-ammonium fluoride in THF to produce the desired compound **66** in high yield (95%). Compound **66** (which can be, therefore, prepared on three different ways: from **69,** from **65,** and from **70)** could be then reacted with 2-cyanoethyl N,N-diisopropylchlorophosphosphite in the presence of *Hanig"s* base to form the corresponding phosphoramidite **71** which is ready to use for the preparation of oligonucleotides lipophilized at any position within the sequence.

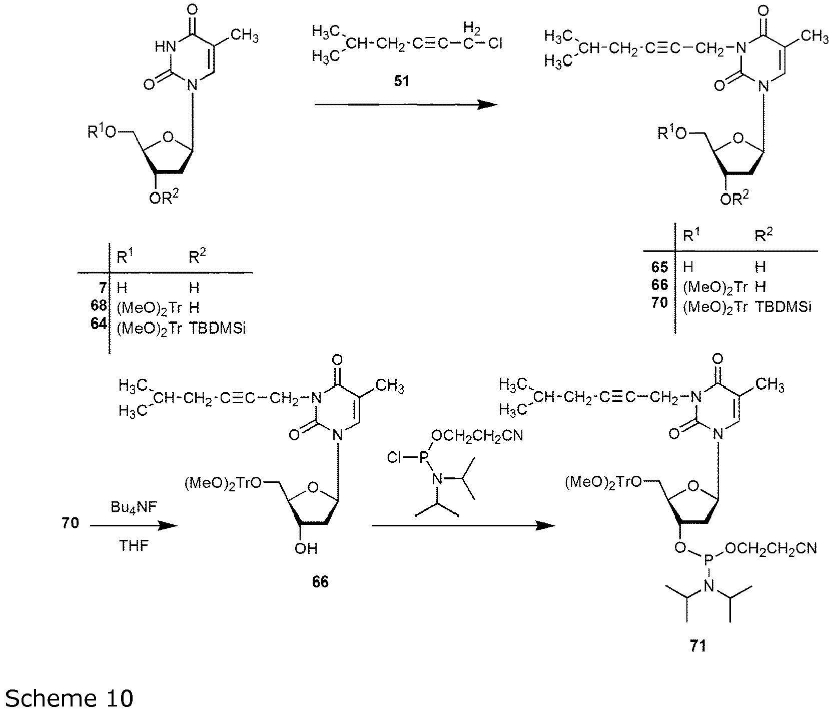

In a further aspect alkylation of thymidine **(7)** can be performed by a *Mitsunobu* reaction. This type of alkylation is somewhat more versatile because alcohols which are precursors of halides can be used. However, a protection of the nucleoside OH-groups is advantegous. For this purpose 5'-dimethoxytritylated thymidine **(68)** for a *Mitsunobu* reaction with the alcohol **42** can be used which, however, may lead to by-products. Therefore, also the 3'-hydroxyl of 5'-DMT-thymidine by a tert-butyl-dimethylsilyl group is protected (→ **64).** Reaction of compound **64** with the alcohole **42** in the presence of triphenylphosphine and diisopropylazo dicarboxylate (DIAD) gave in 70% yield the product **67** which was subsequently desilylated with tetrabutylammonium fluoride to give compound **68.**

Nucleolipids are synthesised using the compounds according to the present invention according to known methods. Preferred embodiments of the nucleolipids according to the present invention contain those that were produced with preferred embodiments of reactive lipids according to the invention. An especially preferred embodiment has the lipophilic moiety connected to the 5' end of the oligo- or poly-nucleotide via the linker, spacer and a phosphoric acid diester group.

A further embodiment of the inventin is a method for synthesising modified nucleotides, oligonucleotides or polynucleotides and comprising the step of the reaction of the reactive lipids according to the present invention with nucleosides, oligonucleotides or polynucleotides which are protected, except at one OH group.

The lipid/sample nucleic acid-conjugates are prepared by preparing the single strands of sample nucleic acids using methods well known to the artisan. Preferably, an automatic solid phase synthesis using the phosphoramidite- or the phosphonate-method, is applied. The lipid moiety is the last component used during the routine automatic synthesis using a compound according to the invention and is for example a phosphoramidite derivative or also an appropriate phosphonate derivative.

A further embodiment of the present invention is a method for the identification of nucleic acids. This method includes the steps of providing a sample potentially containing nucleic acids, providing nucleolipids according to the present invention which hybridise with the nucleic acid to be determined under hybridising conditions and contacting the nucleolipids with the sample under hybridising conditions, thus, forming a hybridised product of a nucleic acid contained in the sample and the nucleolipid and detecting said hybridisation product. In this context, the term "hybridisation" or "hybridising conditions" means the hybridisation under conventional hybridising conditions, especially under stringent conditions as described for example by Sambrook and Russell (Molecular cloning: a laboratory manual, CSH Press. Cold Spring Harbor, N.Y., USA, 2001). The term "hybridisation" means in an especially preferred embodiment that the hybridisation takes place under the following conditions: Hybridisation buffer: 2.times.SSC; 10 times.Denhardt-solution (Ficoll 400+PEG+BSA; ratio 1:1:1); 0.1% SDS; 5 mmol EDTA; 50 mmol Na₂HPO₄; 250 µ g/ml herring-sperm DNA; 50 .mu.g/ml tRNA; or 0.25 mol sodium phosphate buffer, pH 7.2; 1 mmol EDTA, 7% SDS.

Hybridising temperature: T=60°C.Washing buffer: 2 times SSC; 0.1% SDS; Washing temperature: T=60°C.

In a further preferred embodiment the term "under hybridising conditions" means formation of multiple stranded hybridisation products under the following conditions: Hybridisation buffer: 10 mM Na-Cacodylate, 10 mM MgCl₂, 100 mM NaCl or 10 mM Na-Cacodylate, 100 mM MgCl₂, 1 M NaCl (the latter for increase low Tm-values) Hybridisation temperature: room temperature, individually between room temperature and 60°C depending on the length and the composition of the target and sample sequences to be hybridized washing buffer: see above washing temperature: room temperature (25°C).

In a particular preferred embodiment of the present invention, the sample-sequence only hybridises with the target-sequence under hybridising conditions in which the sample-sequence is complementary to the target-sequence. This is of great importance when single mutations are to be identified like for example in the pharmacogenetic field.

A further embodiment of the invention also includes a method for detecting the presence or absence of nucleic acids containing specific sequences within a sample and includes the following steps: bringing the sample in contact with the nucleolipids (compound) according to the present invention having oligo- or polynucleotide moieties. At least one of these oligo- or polynucleotides must show a sequence substantially complementary to a specific sequence of nucleic acids contained in the sample, and detecting the formation of hybridising products of the nucleolipids (compounds) according to the present invention and a specific sequence of nucleic acids within the sample (target-sequence) if contained within the sample.

The nucleic acid containing a specific sequence (target sequence) can be marked with a reporter group before using conventional, well known techniques. The skilled person is aware of many of those marker molecules, such as fluorescence dyes, radioactive markers, biotin etc.

In a preferred embodiment fluorescence dyes are used as reporter groups, for example fluorescein, a member of the Alexa- or Cy-dye-group.

In another embodiment, the method according to the present invention for determining the absence or presence of nucleic acids containing specific sequence within a sample is conducted in a way that the step of bringing into contact is conducted in multiple compartments which are separated from each other, whereby in each of said compartments a nucleic lipid having identical nucleic acid sequences is present, thus, the probe sequences present in a single compartment is identical and, in addition, different sequences are present in each of the separated compartments. Alternatively, in one compartment various nucleic lipids having different predetermined nucleic acid sequences may be present. It allows to analyse multiple samples.

During equilibration of the chemical equilibrium hybridization of the target nucleic acid with the corresponding sequence of a multitude of probe sequences occur. Optionally, the kinetics of hybridisation may be optimized by adjusting the temperature of the liquid phase. Optionally, non bonded target sequences may be removed by washing. Stirring of the solution containing the target sequences is preferred.

According to the invention, the identification of the hybridising products can be effected by testing for the reporter groups. When using a fluorescence dye as a reporter group, the measurement of the hybridising products is done by measuring the emitted fluorescence of this marker. In a preferred embodiment, the measurement of the reporter group is effected in the area of the liquid-gas boundary only. In another embodiment using two liquid phases (both liquids are only limited miscible or immiscible and they must form a phase boundary) allows the measurement of the reporter group at the liquid-liquid boundary between the two fluids.

The nucleolipids (compounds) according to the present invention cannot only be used to identify nucleic acids within a sample but also to isolate nucleic acids from a sample containing nucleic acids. Therefore, this invention also covers a method for the isolation of nucleic acids from samples containing nucleic acids and includes the following steps a) bringing the nucleic acids containing sample into contact with the nucleolipid(s) comprising a lipophilic moiety and an oligo- or polynucleotide moiety, whereby the oligo- or polynucleotides allow the hybridisation with at least a part of the nucleic acids contained within the sample, and b) separation of the hybridising products from the other ingredients contained in the sample and, optionally, washing the hybridising products.

Preferably, the bringing into contact occurs in a first liquid phase. By adding a second liquid phase which builds a liquid-liquid boundary with the first liquid phase allowing a spreading of the nucleolipids in a mono-molecular layer in such a way that the lipophilic moiety reaches into the more lipophilic liquid phase, while the other part of the nucleolipid, hybridised with the complementary nucleic acid sequence, extends into the other fluid, the hybridising products can be separated from single stranded nucleic acids from the sample. If desired, the hybridising products can also be separated from the other ingredients contained within the sample and, optionally, be washed.

In a preferred embodiment of the present invention the nucleolipids are used for the isolation of RNA-molecules, especially siRNA, miRNA or mRNA. When isolating mRNA-molecules, the nucleotide moiety of the nucleolipid has a polydT-sequence. Of course, the isolation can also be based on other known sequences contained within the target-sequence.

In another preferred embodiment of the present invention certain types of nucleic acids, namely aptameres, may be used for the isolation method. In case aptameres are used as the nucleic acid moiety of the nucleolipids, purification of other molecules than nucleic acids, like for example proteins, is possible.

This invention also concerns kits for identifying nucleic acids which contain one or more of the nucleolipids according to the present invention. These kits contain instructions for the detection of nucleic acids and, if required, a second liquid phase which builds a liquid-liquid boundary with the liquid sample.

The nucleotides according to the present invention may also be used to produce arrays of nucleic acids. This means that the nucleolipids containing a lipid moiety and an oligonucleotide moiety can be utilised in nucleic acid arrays, so called DNA-chips. These DNA-chips can be used for example for the analysis of genes or sections of genes, in particular, in pharmacogenetic analyses. Those microarrays may now be produced with the help of the nucleolipids, according to the present invention, which in contrast to the conventional DNA-chips no longer requires complicated chemical procedures for activation of the solid surface and chemical fixation of the sample sequences on those surfaces.

Furthermore, the arrays according to the present invention have another advantage compared to the conventional arrays with permanent, i.e. covalently bound nucleic acid moieties, since they show spacial flexibility. When a hybridising product is formed, they require more space which requires a lateral displacement of the sample sequences. Since the nucleolipids are not connected to the solid plate by covalent bonds and the arrangement of the molecules at the boundary layer of a liquid-liquid system, respectively, the nucleolipids can move laterally thus enabling an optimum density for hybridisation at all times.

Therefore, this invention also concerns a system for the analysis of nucleic acids comprising a device like for example a DNA-chip or an array, comprising a lower section which may contain a liquid phase and an upper section which is not permanently attached to the lower part and which is insertable into the lower part, whereby the upper part has at least two compartments separated from each other and these compartments are formed from the upper to the lower side of the upper part. This makes it possible that e.g. the liquid phase in the lower section is able to exchange target nucleic acids contained within the phase of the upper section.

Preferably, the upper section has at least 4, 8, 16, 25, 64, 256, 384, etc. separated compartments.

In a preferred embodiment of the device, the upper part is designed in such a way that when placed within the lower part the lower section of the lower part contains a joined liquid subphase which is not divided in single compartments.

A specific detection of nucleic acids is possible by the use of nucleolipids and the device according to the present invention. In the following an example is given for the analysis of nucleic acids using the system according to the present invention comprising the nucleolipids and the device described above.

The highly lipophilic single-stranded oligonucleotides of different nucleic acid sequences according to the present invention are being inserted separately into the compartments of the device e.g. with the help of a spotter, where they form a monolayer with properties of a liquid-analogue phase. The spreading is such that the lipid molecules point towards the gas phase while the oligonucleotides point into the liquid phase. If required, the aqueous phase may also be covered with a thin layer of oil thus creating a liquid-liquid phase boundary, in which the lipid chains point towards the lipophilic phase.

Now, the target sequence to be identified--marked with reporter groups such as fluorescence dye using methods known in the art in advance--has been injected into the subphase, common to all sample sequences, and spreaded by gentle mechanical stirring. During the adjustment of the chemical equilibrium, the target-DNA will hybridise with the corresponding sequence. The kinetics of the hybridising process may be optimized by adjusting the temperature of the lower section of the device containing the subphase and/or by flow of a buffer solution through the subphase (washing). In a compartment of the device containing the marked target-sequence and the optimum fitting and known sample sequence will form the hybridisation product which can easily be identified by well known methods, like for example fluorescence detectors.

As discussed above, the reactive lipids according to the present invention are particularly useful for the use in conventional DNA-synthesisers where they may be applied as 5' end building blocks. This allows a simple synthesis of sample sequences and reduces the problems accompanied with the neosynthesis of oligonucleotides on the array itself and the difficult chemical fixation of nucleic acid probes functionalized in advance on the activated surface of the area, respectively.

When selecting a sample nucleic acid, various possibilities are given.

Not only can nature derived DNA- and RNA-molecules be used. Rather oligomeres which can be modified in multiple ways may be used. For example, a PNA (peptide-nucleic acid), complementary to the target nucleic acid to be examined, can be used for hybridisation. Furthermore, nucleic acids with modifications in their sugar moiety, i.e. hexose or hexitole nucleic acids, have been prepared in recent years which are capable of hybridising with natural nucleic acids. Said sample nucleic acids can be used also in the analytic methods according to the present invention. It has been shown that modifying a nucleobase of a sample oligonucleotide, i.e. incorporation of purin-isosteric 8-Aza-7-deaza-7-halogenopurine-base, significantly increases the stability of a duplex with a common DNA-oligomer which leads to a harmonisation of otherwise differently stable base-pairs guanine-cytosine and adenine-thymine. Also those modified sample nucleic acids can be used in this analytic procedure according to this invention.

According to the present invention, the gaseous phase or the gas is air or an inert gas such as nitrogen, argon etc. According to the present invention, using a system of two fluids which are generally immiscible produces a boundary layer, called liquid-liquid boundary layer. The result is a lipophilic phase as well as a hydrophilic phase. Usually, the target sequence will be in the hydrophilic phase which is generally an aqueous phase, like a buffer solution. The lipophilic phase is for example made up of an organic solvent or oil. The skilled person knows many of those systems.

Another application of the nucleolipids is using them as marker of different compositions, like crude oil or other processed products. Adding nucleolipids with a known nucleic acid sequence specifically marks the products. This specific marking allows for a later identification of the compound's origin. This could be an easy way to identify the polluter of an oil spill. These nucleic acids are soluble in oils and other lipophilic fluids due to their lipophilic section.

### Experimental Part

*General.* All chemicals were purchased from *Sigma-Aldrich* (D-Deisenhofen) or from *TCI -* Europe (B-Zwijndrecht). Solvents were of laboratory grade and were distilled before use. TLC: aluminum sheets, silica gel 60 F₂₅₄, 0.2 mm layer *(Merck,* Germany). M.p. *Büchi* SMP-20, uncorrected. UV Spectra: Cary 1E spectrophotometer *(Varian,* D-Darmstadt). NMR Spectra (incl. ¹H-DOSY spectra): AMX-500 spectrometer *(Bruker,* D-Rheinstetten); ¹H: 500.14 MHz, ¹³C: 125.76 MHz, and ³¹P: 101.3 MHz. Chemical shifts are given in ppm relative to TMS as internal standard for ¹H and ¹³C nuclei and external 85 % H₃PO₄; J values in Hz. ESI MS Spectra were measured on a *Bruker Daltronics* Esquire HCT instrument *(Bruker Daltronics,* D-Leipzig); ionization was performed with a 2% aq. formic acid soln. Elemental analyses (C, H, N) of crystallized compounds were performed on a VarioMICRO instrument (Fa. *Elementar,* D-Hanau). Gel permeation chromatography (GPC) was performed on three columns with a light scattering detector (Dawn Helios) and an RI detector (Optilab rEX, Wyatt). The results were evaluated and displayed with the program ASTRA 5.3.4, version 14. logP Values were calculated using the program suite *Chemsketch* (version 12.0, provided by *Advanced Chemistry Developments Inc.; Toronto,* Canada; http://www.acdlabs.com. Oligonucleotides were synthesized, purified, and characterized (MALDI-TOF MS) by Eurogentec (Eurogentec S.A., Liege Science Park, B-Seraing).

### Oligonucleotide incorporation into artificial bilayers.

The incorporation of the oligonucleotides **18 - 22** into artificial lipid bilayers was performed using a lipid mixture of 1-palmitoyl-2-oleyl-sn-glycero-3-phosphoethanolamine (POPE) and 1-palmitoyl-2-oleyl-sn-glycero-3-phosphocholine (POPC) (8:2, w/w, 10 mg/ml of n-decane). The horizontal bilayers were produced automatically within the *"Bilayer Slides"* using an *"Ionovation Explorer"* (Ionovation GmbH, Osnabrück, Germany). After pre-filling with buffer (250 mM KCI, 10 mM MOPS/Tris, pH 7), the *"Bilayer Slide"* is inserted into the stage unit of the *"Ionovation Explorer".* The Ag/AgCl electrodes are mounted, and after addition of 0.2 µl of POPE /POPC lipid to the cis-compartment, the automated bilayer production is started. The *"Ionovation Explorer"* uses a modified painting technique, where the air-water interface paints the lipid across the aperture. The bilayer formation is monitored via capacitance measurements. When a stable bilayer is established (> 50 pF) the corresponding oligonucleotide solution was injected into the cis compartment of the *"Bilayer Slide".* During the incubation time of 25 min the bilayer integrity was monitored by the *"Ionovation Explorer"* through continuous capacitance measurements.

A confocal laser scanning microscope (Insight Cell 3D, Evotec Technologies GmbH, Hamburg, Germany), equipped with a 635 nm emitting laser diode (LDH-P-635, PicoQuant GmbH, Berlin, Germany), a 40x water-immersion objective (UApo 340, 40x, NA = 1.15, Olympus, Tokyo, Japan), and an Avalanche photodiode detector (SPCM-AQR-13-FC, Perkin-Elmer Optoelectronics, Fremont, CA, USA) was used for the optical measurements. Fluorescence irradiation was obtained with an excitation laser power of 200 ± 5 µW. 2D- and 3D scans were performed by scanning the confocal laser spot in XY direction with a rotating beam scanner and movement of the objective in Z direction. The movement in all directions was piezo-controlled which allows a nano-meter precise positioning. For the 2D pictures (Z-scans, Figures 7, 9, and 10) the confocal plane was moved in 100 nm steps.

From the fluorescence signals of single molecules which pass the laser spot, the diffusion constants can be calculated by means of fluorescence correlation analysis. In order to determine the diffusion times of the fluorescent oligonucleotides within and in the proximity of the bilayer, they were measured at overall five different positions above, below and within the layer (Figure 2-A). At each point five 30 s - measurements, were taken. In summary, each measuring protocol was as follows: (i) a reference scan of the stable (empty) bilayer; (ii) addition of the sample with 30 min of incubation, followed by a scan series; (iii) additional scan series, each after a 1.and 2. perfusion (60 s, each).

Subsequently, the cyanine-5 - labelled oligonucleotide **23** (50 nM, 6 µl) was injected into the cis compartment of the "Bilayer Slides" containing either membrane-bound **21** or membrane-bound **22.** After an equilibration time of 60 min the cis channel was perfused repeatedly for 30 sec (1.1 ml/min) and the bilayers were inspected by confocal fluorescence microscopy.

*RP-18 HPLC. RP-18* HPLC was carried out on a 250 x 4 mm *RP-18* column (Merck, Germany) on a Merck-Hitachi HPLC apparatus with one pump (Model 655A-12) connected with a proportioning valve, a variable wavelength monitor (Model 655 A), a controller (Model L-5000), and an integrator (Model D-2000). Solvent: MeCN/0.1 M Et₃NH⁺OAc- (35:65, v/v, pH 7.0).

### Synthesis of inosine derivatives

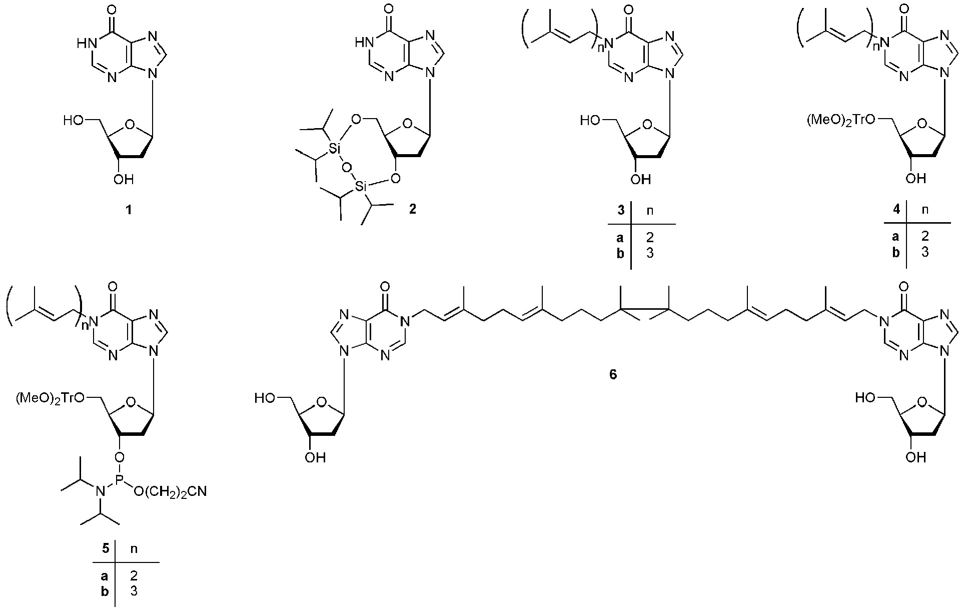

*9-((6aR,8R, 9aS)-2,2,4,4-Tetraisopropyltetrahydro-6H-furo[3,2-f]-[1,3,5,2,4]trioxadisilocin-8-yl)-1H-purin-6(9H)one* (2). Anhydr. 2'-deoxyinosine **(1,** 1.01 g, 4 mmol) was suspended in dry pyridine (40 ml), and 1,3-dichloro-1,1,3,3-tetraisopropyldisiloxane (1.39 g / 4.4 mmol) was added under moisture exclusion. After stirring for 24 h at ambient temperature the solvent was evaporated, and the residue was partitioned between EtOAc and water (80 ml, 1:1, v/v). The organic layer was washed twice with 1 M hydrochloric acid (80 ml), followed by H₂O, conc. aq. HaHCO₃ and brine (80 ml, each). After drying (Na₂SO₄, 1 h) the solvent was evaporated, and the residue was chromatographed (silica gel, column: 6 x 10 cm, CHCl₃-MeOH, 9:1, v/v). From the main zone compound **2** (1.96 g, 99 %) was isolated as colourless amorphous material. M.p. 210 °C. TLC (silica gel 60, CHCl₃-MeOH, 9:1, v/v): R_{f}, 0.56. UV (MeOH): λₘₐₓ = 244 nm (ε = 12.250 M⁻¹cm⁻¹); ε₂₆₀ = 7.500 M⁻¹cm⁻¹. ¹H-NMR ((D₆)DMSO): 12.33 (s, NH); 8.19 (s, H-C(2)); 7.97 (s, H-C(8)); 6.27 (t, ³J(H-C(1'), H-C(2')) = 5.5, H-C(1')); 4.94 (q, H-C(3')); 3.92 (m, H-C(4')); 3.81 (H₂-C(5')); 2.80 (m, H_{β}-C(2')); 2.56 (m, H_{c},-C(2')); 1.08 - 1.01 (m, 28 H, H-C(1'')); i-Pr).
¹³C-NMR ((D₆)DMSO): 156.47 (C(6)); 147.41 (C(4)); 145.49 (C(2)); 138.72 (C(8)); 124.74 (C(5)); 84.47 (C(1')); 82.10 (C(4')); 71.22 (C(3')); 62.40 (C(5')); 38.70 (C(2')); 17.06 (8 x CH₃); 12.33 (4 x CH). Anal. calc. for C₂₂H₃₈N₄O₅Si₂ (494.732): C 53.41 %, H 7.74 %, N 11.32 %; found: C 53.21 %, H 7.78 %, N 11.23 %.

*9-((2R, 4S, 5R)-4-Hydroxy-5-(hydroxymethyl)tetrahydro-furan-2-yl)-1-((2E, 6E)-3,7,11-trimethyldodeca-2,6,10-trienyl)-1H-purin-6(9H)-on* **(3b).** Anhydrous 2'-deoxyinosine **(1,** 1.01 g, 4 mmol) was suspended in anhydr., amine-free DMF and heated on a water bath (55 °C). Then, anhydr. K₂CO₃ (1.44 g, 10.4 mmol) was added, and the mixture was stirred for 10 min. After cooling to ambient temperature, farnesyl bromide (1.32 g, 4.4 mmol) was added drop-wise under N₂ atmosphere. After stirring for 24 h at room temp. the solvent was evaporated, and the residue dried in high vacuo. Chromatography (silica gel, column: 6 x 14 cm, CHCl₃-MeOH, 9:1, v/v) gave one main zone from which after evaporation of the solvent compd. **3b** (1.28 g, 74 %) was isolated as an amorphous solid. TLC (silica gel 60, CHCl₃-MeOH, 95:5, v/v): R_{f} 0.42. logP, 3,40 ± 0,94. UV (MeOH): λₘₐₓ = 250 nm (ε = 10.150 M⁻¹cm⁻¹) ε₂₆₀ = 7.000 M⁻¹cm⁻¹. ¹H-NMR ((D₆)DMSO): 8.34 (s, H-C(2)); 8.30 (s, H-C(8)); 6.29 (t, 3J(H-C(1'), H-C(2') = 7.0, H-C(1')); 5.35 (d, ³J(HO-C(3'), H-C(3') = 7.5, HO-C(3')); 5.35 (t, ³J(H-C(2''), H-C(1'') = 7.5, H-C(2'')); 5.10 (H-C(6'')); 5.10 (H-C(10'')); 4.92 (t, ³J(HO-C(5'), H-C(5') = 6,5, HO-C(5')); 4.70 (d, ³J(H-C(1''), H-C(2'') = 7,5, H-C(1'')); 4.39 (H-C(3')); 3.86 (H-C(4')); 3.55 (H-C(5')); 2.61 (H_{β}-C(2')); 2.27 (H_{α}-C(2')); 2.04 (H-C(8'')); 1.98 (H-C(9'')); 1.93 (H-C(5'')), 1.86 (H-C(4")); 1.77 (H-C(13'')); 1.60 (H-C(12'')); 1.51 (H-C(14'')); 1.51 (H-C(15'')). ¹³C-NMR ((D₆)DMSO): 155.71 (C(6)); 147.98 (C(4)); 146.99 (C(2)); 140.05 (C(3'')); 138.96 (C(8)); 134.67 (C(7'')); 130.56 (C(11'')); 124.02 (C(6'')); 123.80 (C(5)); 123.44 (C(10'')); 119.43 (C2')); 87.97 (C(1')); 83.60 (C(4')); 70.67 (C(3')); 61.61 (C(5')); 43.22 (C(1'')); 39.48 (C(2')); 39.11 (C(8'')); 38.81 (C(4''); 26.11 (C(5'')); 25.62 (C(12'')); 25.41 (C(9'')); 17.45 (C(15'')); 16.21 (C(14'')); 15.78 (C(13'')). Anal. calc. for C₂₅H₃₆N₄O₄ (456.578):
C 65.76 %, H 7.95 %, N 1227 %; found: C 65.42 %, H 8.06 %, N 12.04 %.

*1-((E)-3, 7-Dimethylocta-2, 6-dienyl)-9-((2R, 4S, 5R)-4-hydroxy-5-(hydroxymethyl)tetrahydrofuran-2-yl)-1H-purin-6(9H)-on* **(3a).** Compound **3a** was prepared and worked up from 2'-deoxyinosine **(1,** 1.01 g, 4 mmol) and geranyl bromide (0.96 g, 4.4 mmol) as described for **3b.** Yield: 0.80 g (51 %). TLC (silica gel 60, CHCl₃-MeOH, 95:5, v/v): R_{f} 0.41. UV (MeOH): λₘₐₓ = 250 nm (ε = 10.450 M⁻¹cm⁻¹) ε₂₆₀ = 7.800 M⁻¹cm⁻¹. log*P*: 1,37 ± 0.93. ¹H-NMR ((D₆)DMSO): 8.35 (s, H-C(2)); 8.31 (s, H-C(8)); 6.31 (t, ³J(H-C(1'), H-C(2') = 7.0, H-C(1')); 5.30 (HO-C(3')); 5,28 (t, ³J(H-C(2''), H-C(1') = 7.0, H-C(2'')); 5.03 (t, 33 (H-C(6''), H-C(5'') = 6.5, H-C(6'')); 4.94 (HO-C(5')); 4.62 (d, ³J(H-C(1''), H-C(2'') = 6.5, H-C(1'')); 4.39 (H-C(3')); 3.88 (H-C(4')); 3.57 (H-C(5')); 2.63 (H_{β}-C(2')); 2.30 (H_{α}-C(2')); 2.04 (H-C(5'')); 2.00 (H-C(4'')); 1.79 (H-C(13'')); 1.59 (H-C(8'')); 1.53 (H-C(14'')). ¹³C-NMR ((D₆)DMSO): 156.19 (C(6)); 148.45 (C(4)); 147.46 (C(2)); 140.56 (C(3'')); 139.49 (C(8)); 131.47 (C(7'')); 124.25 (C(6'')); 124.16 (C(5)); 119.87 (C2'')); 88.42 (C(1')); 84.07 (C(4')); 71.12 (C(3')); 62.07 (C(5')); 43.71 (C(1'')); 39.65 (C(2')); 39.32 (C(8'')); 38.98 (C(4''); 26.22 (C(5'')); 25.82 (C(8'')); 17.95 (C(14'')); 16.65 (C(13'')). Anal. calc. for C₂₀H₂₈N₄O₄ (388.461): C 61.84 %, H 7.27 %, N 14.42 %; found: C 61.72 %, H 7.31 %, N 14.31 %.

*9-((2R,4S,5R)-5-((Bis(4-methoxyphenyl)(phenyl)methoxy)- methyl)-4-hydroxytetrahydrofuran-2-yl)-1-((2E, 6E)-3,7,11-trimethyldodeca-2,6,10-trienyl)-1H-purin-6(9H)-on* **(4b).** Compound **3b** (0.46 g, 1.0 mmol) was co-evaporated twice from dry pyridine (1 ml, each) and then dissolved in anhydr. pyridine (5 ml). After addition of 4,4'-dimethoxytriphenylmethylchlorid (0.40 g, 1.15 mmol) the reaction mixture was stirred for 24 h at ambient temperature under N₂ atmosphere. Then, the reaction was quenched by addition of MeOH (3 ml). After addition of aq. 5 % NaHCO₃ (30 ml) the aqueous phase was extracted three times with CH₂Cl₂ (30 ml, each), and the combined organic layers were dried (Na₂SO₄, 1 h) and filtered. Chromatography (silica gel, column: 6 x 10 cm, CHCl₃-MeOH: 96:4, v/v) gave one main zone from which compound **4b** (0.46 g, 61 %) was isolated as a slightly yellowish glass; m.p. 68 °C. TLC (silica gel 60, CHCl₃-MeOH: 96:4, v/v): R_{f} 0.13. UV (MeOH): λₘₐₓ = 235 nm (ε = 38.200 M⁻¹cm⁻¹); ε₂₆₀ = 14.150 M⁻¹cm⁻¹. log P: 9,81 ± 0.96. ¹H-NMR ((D₆)DMSO): 8.23 (s, H-C(2)); 8.16 (s, H-C(8)); 7.32 (H-C(10'''))_{J} 7.31 (H-C(8''')); 7.20 (H-C(3''')); 7.18 (H-C(9''')); 6.80 (H-C(4''')); 6.31 (t, ³J(H-C(1'), H-C(2') = 6,5, H-C(1')); 5.33 (HO-C(3')); 5.24 (t, ³J(H-C(2''), H-C(1') = 7.0, H-C(2'')); 5.02 (t, ³J(H-C(10''), H-C(9'') = 6.0, H-C(10'')); 4.99 (t, ³J(H-C(6''), H-C(5'') = 6.5, H-C(6'')); 4.59 (H-C(1'')); 4.40 (H-C(3')); 3.98 (H-C(4')); 3.71 (H-C(6''')); 3.15 (H-C(5')); 2.74 (H_{β}-C(2')); 2.32 (H_{α}-C(2')); 2.05 (t, ³J(H-C(8''), H-C(9'') = 6.5, H-C(8'')); 1.99 (H-C(9'')); 1.93 (H-C(5'')); 1.85 (t, ³J(H-C(4''), H-C(5'') = 7.5, H-C(4'')); 1.77 (H-C(13'')); 1.59 (H-C(12'')); 1.50 (H-C(14'')); 1.50 (H-C(15'')). ¹³C-NMR ((D₆)DMSO): 157.93 (C(5'')); 155.65 (C(6)); 147.65 (C(2)); 146.93 (C(4)); 144.72 (C(7''')); 140.02 (C(3'')); 139.07 (C(8)); 135.48 (C(2''')); 134.59 (C(7'')); 130.47 (C(11'')); 129.54 (C3''')); 129.54 (C(9''')); 127.57 (C(8''')); 126.46 (C(10''')); 123.94 (C(6'')); 123.94 (C(10'')); 123.38 (C(5)); 119.29 (C(2'')); 112.97 (C(4'''); 85.93 (C(1''')); 85.35 (C(1')); 83.44 (C(4')); 70.54 (C(3')); 64.03 (C(5')); 54.89 (C(6''')); 43.09 (C(2')); 39.02 (C(8'')); 38.85 (C(4'')); 38.75 (C(1'')); 26.02 (C(9'')); 25.56 (C(5'')); 25.32 (C(12'')); 17.36 (C(15'')); 16.12 (C(14'')); 15.67 (C(13'')). Anal. calc. for C₄₆H₅₄N₄O₆ (758.944): C 72.80 %, H 7.17 %, N 738 %; found: C 72.53 %), H 714 %, N 7.27 %.

*9-((2R,4S,5R)-5-((Bis(4-methoxyphenyl)(phenyl)methoxy)-methyl)-4-hydroxytetrahydrofuran-2-yl)-1-((E)-3,7-di-methylocta-2,6-dienyl)-1H-purin-6(9H)-one* **(4a).** Compound **4a** was prepared from **3a** (0.39 g, 1.0 mmol) and worked up as described for compound **4b.** Yield: 0.48 g, 69 %) of a colourless glass; m.p. 74 °C. TLC ( silica gel 60, CHCl₃-MeOH: 96:4, v/v): R_{f} 0.19. UV (MeOH): λₘₐₓ = 235 nm (ε = 32.820 M⁻¹cm⁻¹); ε₂₆₀ = 12.791 M⁻¹cm⁻¹. logP: 7.77 ± 0.94. ¹H-NMR ((D₆)DMSO): 8.25 (s, H-C(2)); 8.18 (s, H-C(8)); 7.33 (H-C(10''')); 7.31 (H-C(8''')); 7.20 (H-C(3''')); 7.19 (H-C(9''')); 6.81 (H-C(4''')); 6.32 (t, ³J(H-C(1'), H-C(2') = 6.5, H-C(1')); 5.35 (HO-C(3')); 5.23 (t, ³J(H-C(2''), H-C(1'') = 6.5, H-C(2'')); 5.02 (t, ³J(H-C(6''), H-C(5'') = 6.0, H-C(6'')); 4.60 (H-C(1'')); 4.40 (H-C(3')); 3.98 (H-C(4')); 3.72 (H-C(6''')); 3.16 (H-C(5')); 2.75 (H_{β}-C(2')); 2.34 (H_{α}-C(2')); 2.03 (H-C(5'')); 2.00 (H-C(4'')); 1,77 (H-C(13'')); 1.57 (H-C(8'')); 1.51 (H-C(14'')). ¹³C-NMR ((D₆)DMSO): 157.95 (C(5''')); 155.67 (C(6)); 147.67 (C(2)); 146.95 (C(4)); 144.72 (C(7''')); 140.07 (C(3'')); 139.11 (C(8)); 135.48 (C(2''')); 130.93 (C(11'')); 129.56 (C3''')); 129.55 (C(9''')); 127.68 (C(8''')); 126.48 (C(10''')); 123.95 (C(6'')); 123.61 (C(5)); 119.27 (C(2'')); 112.98 (C(4'''); 85.92 (C(1''')); 85.35 (C(1')); 83.44 (C(4')); 70.53 (C(3')); 64.03 (C(5')); 54.88 (C(6''')); 43.11 (C(2')); 38.74 (C(4'')); 38.74 (C(1''); 25.70 (C(5'')); 25.26 (C(8'')); 17.39 (C(14'')); 16.10 (C(13'')). Anal. calc. for C₄₁H₄₆N₄O₆ (690.827): C 71.28 %, H 6.71 %, N 8.11%); found: C 70.94 %, H 6.67 %, N 7.93%.

*(2R,3S,5R)-2-((Bis(4-methoxyphenyl)(phenyl)methoxy)-methyl)-5-(6-oxo-1-((2E,6E)-3,7,11-trimethyldodeca-2,6,10-trienyl)-1H-purin-9(6H)-yl)tetrahydrofuran-3-yl 2-cyanoethyldiisopropyl phosphoramidite (***5b***)*. Compound **4b** (100 mg, 0.13 mmol) was co-evaporated twice with CH₂Cl₂ and then dissolved in CH₂Cl₂ (5 ml). After addition of N,N-diisopropylethylamine (42 µL, 0.24 mmol) and (chloro)(2-cyanoethoxy)(diisopropylamino)phosphine (52 µL, 0.24 mmol) the reaction mixture was stirred for 15 min (!) at room temp. under N₂ atmosphere. Then, ice-cold 5 % aq. NaHCO₃ was added (4 ml), and the mixture was extracted three times with CH₂Cl₂ (8 ml, each). The combined organic layers were dried (Na₂SO₄, 10 min), filtered and the solvent evaporated (< 25 °C). Flash chromatography (0.5 bar, silica gel, column: 2 x 8 cm, CH₂Cl₂-acetone: 8:2, v/v) gave compd. **5b** (98 mg, 78%) as amorphous material. TLC (silica gel, CH₂Cl₂-acetone, 8:2, v/v): R_{f} 0.64, 0.76 (diastereoisomers). log*P* = 12.86 ± 1.11. ³¹P-NMR (CDCl₃):148.86, 149.02.

*(2R,3S,5R)-2-((Bis(4-methoxyphenyl)(phenyl)methoxy)-methyl)-5-(1-((E)-3,7-dimethylocta-2,6-dienyl)-6-oxo-1H-purin-9(6H)-yl)-tetrahydrofuran-3-yl-2-cyanoethyl-diisopropylphosphoramidite* (**5a**). Compound **5a** was prepared and worked up from **4a** (100 mg, 0.14 mmol) as described for compd. **5b.** Yield: 79 mg (61 %) of amorphous material. TLC (silica gel, CH₂Cl₂-acetone, 8:2, v/v): R_{f} 0.64, 0.76 (diastereoisomers). logP = 10.82 ± 1.10. ³¹P-NMR (CDCl₃):148.87, 149.02.

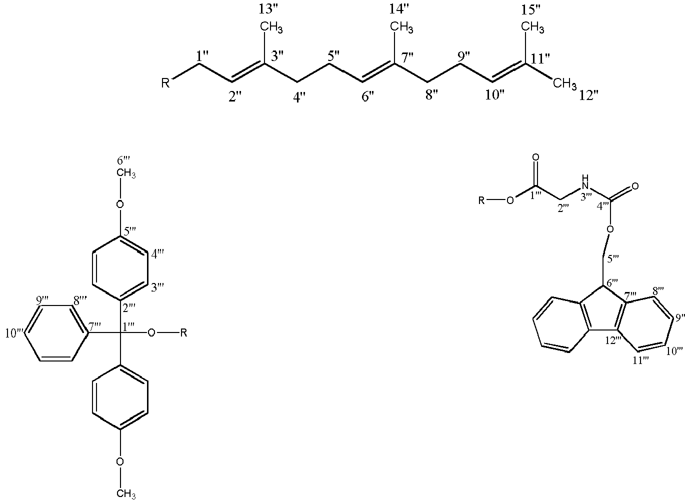

Numbering of the terpenyl-, the 4,4'-dimethoxytriphenylmethyl- (DMTr), and of the fluorenylmethoxycarbonyl- (Fmoc) residues throughout the Exp. Part.

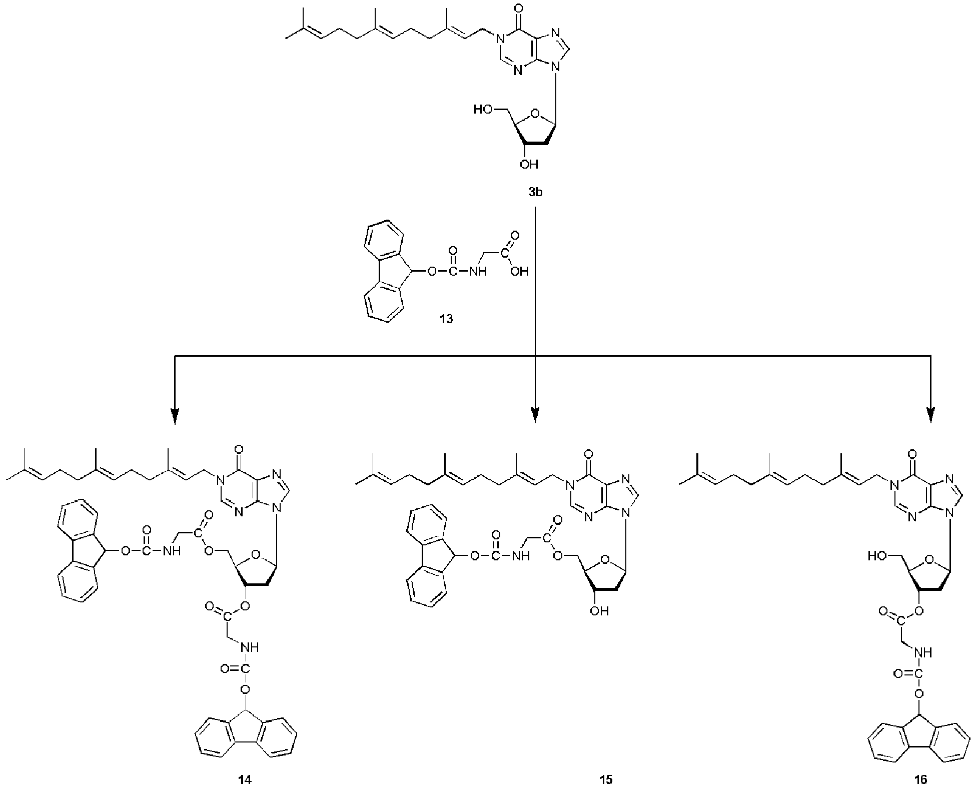

*Small-Scale Coupling of Compound **3b** with (i) N-[(9H-Fluoren-9-ylmethoxy)-carbonyl]-glycine (*→ **14** - **16***) and (ii) Sulforhodamin-sulfonylchloride* (→ **17**).(i) N-[(9H-Fluoren-9-ylmethoxy)-carbonyl]-glycine (13, 65.4 mg, 0.22 mmol) was dissolved in CH₂Cl₂ (20 ml) and 4-(dimethylamino)pyridine (5 mg) and compound **3b** (100 mg, 0.22 mmol) were added. The reaction mixture was cooled to 0 °C, and dicyclohexylcarbodiimide (45.5 mg, 0.22 mmol) in CH₂Cl₂ (2 ml) were added drop-wise. After 5 min the mixture was allowed to warm up to room temp., and stirring was continued over night. Then, further portions of N-[(9H-fluoren-9-ylmethoxy)-carbonyl]-glycine, 4-(dimethylamino)pyridine, and dicyclohexylcarbodiimide (30 mole-%, each) were added, and stirring was continued. After a total reaction time of 48 h the suspension was filtered, and the filtrate was evaporated to dryness. Chromatography (silica gel, column: 2 x 15 cm, CH₂Cl₂-acetone, 6:4, v/v) afforded three main zones from which the following fluorene-labelled nucleolipids were obtained upon evaporation of the solvent.

*(2R,3S,5R)-3-(2-(((9H-Fluoren-9-yl)methoxy)carbonyl-amino)acetoxy)5-(6-oxo-1-((2E, 6E)-3,7,11-trimethyl-dodeca-2,6,10-trienyl)-1H-purin-9(6H)-yl)-tetrahydrofuran-2-yl)methyl-2-(((9H-fluoren-9-yl)methoxy)carbonylamino)acetate (***14***)*. TLC (silica gel, CHCl₃-MeOH, 96:4, v/v): R_{f} 0.56. UV (MeOH): λₘₐₓ = 263 nm (ε = 45.200 M⁻¹cm⁻¹); ε₂₆₀ = 44.200 M⁻¹cm⁻¹˙ log*P* = 11.67 ± 1.22. ¹H-NMR ((D₆)DMSO): 8.29 (s, H-C(2)); 8.27 (s, H-C(8)); 7.87 (H-C(11''')); 7.68 (H-C(8''')); 7.40 (H-C(10''')); 7.31 (H-C(9''')); 6.28 (t, ³J(H-C(1'), H-C(2') = 7,0, H-C(1')); 5.43 (H-C(3')); 5.25 (H-C(2'')); 5.01 (H-C(6'')); 5.01 (H-C(10'')); 4.60 (H-C(1'')); 4.34 (H-C(5''')); 4.30 (H-C(4')); 4.25 (H-C(6''')); 3.86 (H-C(2''')); 3.80 (H-C(5')); 2.99 (H_{β}-C(2')); 2.03 (H-C(8'')); 1.98 (H-C(9'')); 1.93 (H-C(5'')); 1.85 (H-C(4'')); 1.77 (H-C(13")); 1.60 (H-C(12'')); 1.50 (H-C(14'')); 1.50 (H-C(15'')). ¹³C-NMR ((D₆)DMSO): 169.88 (C(1''')); 156.47 (C(4''')); 155.60 (C(6)); 148.06 (C(2)); 147.06 (C(4)); 143.71 (C(7''')); 140.66 (C(12''')); 140.05 (C(3'')); 138.99 (C(8)); 134.61 (C(7'')); 130.49 (C(11'')); 127.53 (C10''')); 126.96 (C(11''')); 125.05 (C(9''')); 123.96 (C(6'')); 123.38 (C(10'')); 119.99 (C(5)); 119.92 (C(8''')); 119.25 (C(2'')); 83.31 (C(1'); 81.46 (C(4')); 74.69 (C(3')); 65.76 (C(5''')); 55.76 (C(5')); 47.44 (C(6''')); 46.53 (C(2''' )); 43.26 (C(1'')); 38.98 (C(2')); 38.70 (C(8'')); 35.85 (C(4'')); 26.04 (C(5'')); 25.34 (C(12'')); 24.36 (C(9'')); 17.37 (C(15'')); 16.15 (C(14'')); 15.69 (C(13'')).

*((2R,3S,5R)-3-Hydroxy-5-(6-oxo-1-((2E,6E)-3,7,11-tri-methyldodeca-2,6,10-trienyl)-1H-purin-9(6H)-yl)tetra-hydrofuran-2-yl)methyl-2-(((9H-fluoren-9-yl)methoxy)-carbonylamino)acetate (***15***)*. TLC (silica gel, CHCl₃-MeOH, 96:4, v/v): R_{f} 0.31. UV (MeOH): λₘₐₓ = 263 nm (ε = 27.800 M⁻¹cm⁻¹); ε₂₆₀ = 27.100 M⁻¹cm-¹. log P: 7,57 ± 1.15. ¹H-NMR ((D₆)DMSO): 8.33 (s, H-C(2)); 8.25 (s, H-C(8)); 7.88 (H-C(11''')); 7.69 (H-C(8''')); 7.41 (H-C(10''')); 7.32 (H-C(9''')); 6.29 (t, ³J(H-C(1'), H-C(2')) = 7,5, H-C(1')); 5.49 (H-C(3')); 5.26 (H-C(2'')); 5.01 (H-C(6'')); 5.01 (H-C(10'')); 4.60 (H-C(1'')); 4.31 (H-C(5''')); 4.22 (H-C(4')); 4.02 (H-C(6''')); 3.77 (H-C(2''')); 3.77 (H-C(5')); 2.72 (H_{β}-C(2')); 2.34 (H_{α}-C(2')); 2.03 (H-C(8'')); 1.99 (H-C(9'')); 1.94 (H-C(5'')); 1.86 (H-C(4'')); 1.77 (H-C(13'')); 1.60 (H-C(12'')); 1.51 (H-C(14'')); 1.51 (H-C(15'')). ¹³C-NMR ((D₆)DMSO):169.95 (C(1''')); 156.41 (C(4''')); 155.66 (C(6)); 147.95 (C(2)); 146.99 (C(4)); 143.69 (C(7''')); 140.64 (C(12''')); 140.03 (C(3'')); 139.02 (C(8)); 134.61 (C(7'')); 130.49 (C(11'')); 127.52 (C10''')); 126.96 (C(11''')); 125.06 (C(9''')); 123.96 (C(6'')); 123.84 (C(10'')); 123.38 (C(5)); 119.99 (C(8''')); 119.31 (C(2'')); 84.13 (C(1'); 83.27 (C(4')); 70.41 (C(3')); 65.75 (C(5''')); 64.00 (C(5')); 46.51 (C(6''')); 43.18 (C(2''')); 42.00 (C(1'')); 39.90 (C(2')); 39.73 (C(8'')); 39.56 (C(4'')); 26.04 (C(5'')); 25.57 (C(9'')); 25.34 (C(12'')); 17.38 (C(15'')); 16.14 (C(14'')); 15.69 (C(13'')).

*(2R,3S,5R)-2-(hydroxymethyl)-5-(6-oxo-1-((2E,6E)-3,7,11-trimethyl-dodeca-2,6,10-trienyl)-1H-purin-9(6H)-yl)tetra-hydrofuran-3-yl-2-(((9H-fluoren-9-yl)methoxy)carbonyl-amino)acetate (***16***)*.TLC (silica gel, CHCl₃-MeOH, 96:4, v/v): R_{f} 0.20. UV (MeOH): λₘₐₓ = 263 nm (ε = 27.730 M⁻¹cm⁻¹); ε₂₆₀ = 27.100 M⁻¹cm-¹. log P: 7.73 ± 0.98. ¹H-NMR ((D₆)DMSO): 8.31 (s, H-C(2)); 8.29 (s, H-C(8)); 7.88 (H-C(11''')); 7.71 (H-C(8''')); 7.41 (H-C(10''')); 7.33 (H-C(9''')); 6.28 (t, ³J(H-C(1'), H-C(2')) = 8,0, H-C(1')); 5.40 (H-C(3')); 5.26 (H-C(2'')); 5.12 (H-C(6'')); 5.01 (H-C(10'')); 4.60 (H-C(1'')); 4.34 (H-C(5''')); 4.25 (H-C(4')); 4.08 (H-C(6''')); 3.85 (H-C(2''')); 3.60 (H-C(5')); 2.88 (H_{β}-C(2')); 2.04 (H-C(8'')); 2.00 (H-C(9'')); 1.93 (H-C(5'')); 1.86 (H-C(4'')); 1.78 (H-C(13'')); 1.60 (H-C(12'')); 1.51 (H-C(14'')); 1.51 (H-C(15'')). ¹³C-NMR ((D₆)DMSO): 169.70 (C(1''')); 156.48 (C(4''')); 155.61 (C(6)); 148.04 (C(2)); 147.01 (C(4)); 143.72 (C(7''')); 140.66 (C(12''')); 140.03 (C(3'')); 138.84 (C(8)); 134.62 (C(7'')); 130.51 (C(11'')); 127.54 (C10''')); 127.19 (C(11''')); 125.06 (C(9''')); 123.96 (C(6'')); 123.76 (C(10'')); 121.28 (C(5)); 120.02 (C(8''')); 119.31 (C(2'')); 85.09 (C(1'); 83.50 (C(4')); 75.45 (C(3')); 65.76 (C(5''')); 61.35 (C(5')); 46.54 (C(6''')); 43.25 (C(2''')); 42.33 (C(1'')); 39.01 (C(2')); 38.72 (C(8'')); 36.77 (C(4'')); 26.01 (C(5'')); 24.54 (C(9'')); 25.31 (C(12'')); 17.35 (C(15'')); 16.13 (C(14'')); 15.67 (C(13'')).

*(ii) 9-((2R,4S,5R)-4-hydroxy-5-(hydroxymethyl)tetrahydro-furan-2-yl)-1-((2E,6E)-3,7,11-trimethyldodeca-2,6,10-trienyl)-1H-purin-6(9H)-on-5'-sulforhodaminsulfonic ester (***17***)*. Compound **3b** (2.74 mg, 6.0 µmol) was dissolved in anhydr. Pyridine (3 ml), and sulforhodaminsulfonyl chloride (2.5 mg, 4.0 µmol) was added. The mixture was stirred under N₂ atmosphere for 48 h. Then, H₂O (10 ml) was added, and the mixture extracted once with CH₂Cl₂. The aqueous layer was separated and evaporated to dryness. Chromatography (silica gel, column: 2 x 10 cm, CH₂Cl₂-MeOH, 1:1, v/v) gave after evaporation of the solvent compound **17** as black, amorphous material. TLC (silica gel 60, CHCl₃-MeOH, 96:4, v/v): R_{f} 0.56. UV (MeOH): λₘₐₓ = 252 nm (ε = 18.200 M⁻¹cm⁻¹); ε₂₆₀ = 16.400 M⁻¹cm⁻¹_{.}

### Thymidine Derivatives

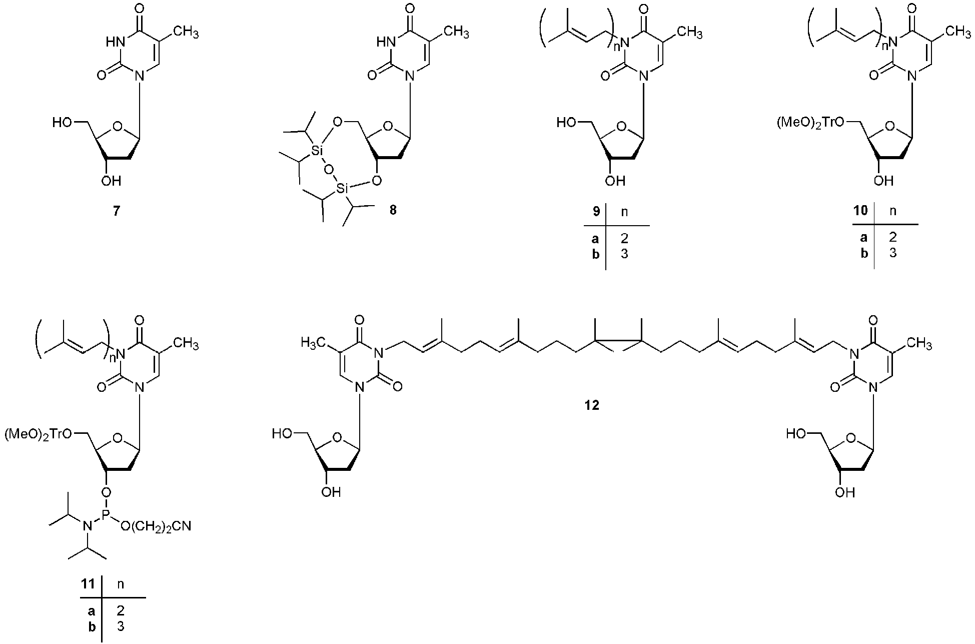

*5-Methyl-1-((6aR,8R,9aR)-2,2,4,4-tetraisopropyltetrahydro-6H-furo[3,2-f][1,3,5,2,4]trioxadisilocin-8-yl)pyrimidine-2,4(1H,3H)-dione (***8***)*. Anhydr. thymidine (**7**, 0.242 g, 1 mmol) was dissolved in dry pyridine (10 ml), and 1,3-Dichlor-1,1,3,3-tetraisopropyl-disiloxane (0.347 g, 1.1 mmol) was added. The reaction mixture was stirred for 24 h at ambient temp.. After evaporation of the solvent the residue was partitioned between EtOAc and H₂O (80 ml, 1:1, v/v). The organic layer was washed twice with cold 1M aq. HCl and H₂O (20 ml, each), followed by sat. aq. NaHCO₃ and brine. After drying (anhyd. Na₂SO₄) and filtration, the soln. was evaporated to dryness. Chromatography (silica gel, column: 2 x 10 cm, CHCl₃-MeOH, 9:1, v/v) gave, after evaporation of the main zone compd. **8** (0.476 g, 98 %) as a colourless solid. M.p. 174 °C. TLC (silica gel, CHCl₃-MeOH, 9:1, v/v): R_{f}, 0.9. UV(MeOH): λₘₐₓ = 265 nm (ε = 12.040 M-¹cm⁻¹). ¹H-NMR ((D6)DMSO): 11.33 (s, NH); 7.40 (*d*, ³J(CH₃, H-C(6)) = 1.0, H-C(6)); 6.00 (*dd*, ³J(H-C(1'), H_{α}-C(2')) = 5.0, ³J(H-C(1'),H_{β} -C(2')) = 5.0, C-H(1')); 4.56 (Ψ*q*, ³J(H-C(3'), H_{α}-C(2')) = 7.5, ³J(H-C(3'), H_{β} -C (2')) = 7.5, ³J(H-C(3'), H-C(4') = 7.5, C-H(3')); 3.96 (*ddd,* ³J(H_{b} -C(5'), H-C(4')) = 5.5, ³J(Hₐ -C(5'), H-C(4')) = 3.25, ²J(Hₐ -C(5'), H_{b} -C(5')) = -12, ²J(H_{b} -C(5'), Hₐ -C(5') = - 12.2, H₂-C(5')); 3.70 (*ddd,* ³J(H-C(4'), H-C(3')) = 7.5, ³J(H-C(4'), H_{b} -C(5')) = 5.5, ³J(H-C(4'), Hₐ-C(5')) = 3.25, C-H(4')); 2.42 (m, H_{β}-C(2')); 2.30 (m, H_{α}-C(2')); 1.76 (*d*, ³J(CH₃, H-C(6)) = 0.5, CH₃)); 1.04 (m, 28H, *i*-Pr). ¹³C-NMR: ((D₆)DMSO): 163.65 (C(4)); 150.14 (C(2)); 136.16 (C(6)); 109.25 (C(5)); 84.19 (C(1')); 83.23 (C(4')); 70.29 (C(3')); 61.71 (C(5')); 38.47 (C(2')); 17.31, 17.18, 17.16, 17.14, 17.01, 16.86, 16.83, 17.31-16.76 (m, 8 x CH₃, iPr), 12.73-11.95 (4 x CH, iPr), 12.11 (CH₃). Anal. calc. for C₂₂H₄₀N₂O₆Si₂ (484.73): C 54.51, H 8.32, N 5.78; found: C 54.54, H 8.21, N 5.68.

*1-((2R,4R,5R)-4-Hydroxy-5-(hydroxymethyl)tetrahydrofuran-2-yl)-5-methyl-3-((2E,6E)-3,7,11-trimethyldodeca-2,6,10-trienyl)pyrimidin-2,4(1H,3H)-dione* (**9b**). Anhydr. thymidine (**7**, 0.97 g, 4 mmol) was dissolved in amine-free, anhydr. DMF (20 ml), and dry K₂CO₃ (1.2 g, 10.4 mmol) was added. Then, trans,trans-farnesylbromide (0.87 ml, 4.4 mmol) was added drop-wise within 10 min under N₂ atmosphere, and the reaction mixture was stirred for 48 h at 40 °C. After filtration the mixture was then partitioned between CH₂Cl₂ and H₂O (100 ml, 1:1, v/v), the organic layer was separated and dried (anhydr. Na₂SO₄). After filtration and evaporation of the solvent the residue was dried in high *vacuo.* Subsequent gradient chromatography (silica gel 60, column: 6 x 10 cm, (i) CH₂Cl₂-MeOH, 95:5, v/v; (ii) CH₂Cl₂-MeOH, 9:1, v/v) gave after evaporation of the main zone compd. **9b** (0.76 g, 44 %). TLC (silica gel, CH₂Cl₂-MeOH, 9:1, v/v): R_{f}, 0.5; TLC (silica gel, CH₂Cl₂-MeOH, 95:5, v/v): R_{f}, 0.3. UV (MeOH): λₘₐₓ = 266 nm (ε = 9.660 M⁻¹cm⁻¹). log *P*: 6.60+/-0.64. ¹H-NMR (D₆)DMSO): 7.75 (*d*, ³J(CH₃, H-C(6)) = 1.26, H-C(6)); 6.20 (Ψ*t*, ³J(H-C(1'), H_{α}-C(2')) = 7.0, ³J(H-C(1'), H_{β} -C(2')) = 7.0, C-H(1')); 5.20 (*d*, ³J(OH-C(3'), H-C(3')) = 4.5, OH-C(3')); 5.10 (Ψ*t*, ³J(H-C(2''), H₂-C(1'') = 6.5, H-C(2")); 5.03 (*m*, (H-C(6", 10")); 5.0 (Ψ*t*, ³J(OH-C(5'), H_{b}-C(5')) = 5.2, ³J(OH-C(5'), Hₐ-C(5')) = 5.2, OH-C(5')); 4.39 (*d*, ³J(H₂-C(1''), H-C(2")) = 7.0, H₂-C(1'')); 4.24 (*ddd,* ³J(H-C(4'), H-C(3')) = 3.78, ³J(H-C(4'), H_{b}-C(5')) = 4.0, ³J(H-C(4'), Hₐ-C(5')) ₌ 4.0, H-C(4'));3.78 (Ψ*q*, ³J(H-C(3'), H_{α}-C(2')) = 3.78, ³J(H-C(3'), H_{β} -C(2')) = 3.78, ³J(H-C(3'), H-C(4')) = 3.78, H-C(3')); 3.60 (*ddd,* ³J(H_{b}-C(5'), H-C(4')) = 4.0, ³J(H_{b}-C(5'), OH-C(5')) = 5.2, ²J(H_{b}-C(5'), Hₐ-C(5')) = -12.0, H_{b}-C(5')); 3.55 (*m*, ²J(Hₐ-C(5'), H_{b}-C(5')) = -12.0, Hₐ-C(5')); 2.09 (*dd*, ³J(H_{α}-C(2'), H-C(1')) = 7.0, ³J(H_{β}-C(2'), H-C(1')) = 7.0, ³J(H_{α}-C(2'), H-C(3')) = 4.8, ³J(H_{β}-C(2'), H-C(3')) = 4.8, ²J(H_{α}-C(2'), H_{β}-C(2')) = -12.0, H₂-C(2')); 1.95 (*m,* (H₂-C(4'',5'',8'',9''), 4×CH₂); 1.82 (*d*, ³J(CH₃, H-C(6) = 1.0, CH₃)); 1.74 (s, H₃-C(13'')); 1.63 (*s*, H₃-C(12'')); 1.55 (*s*, H₃-C(14'')); 1.52 (s, H₃-C(15'')). ¹³C-NMR ((D₆) DMSO): 162.35 (C(4)); 150.24 (C(2)); 138.72 (C(3"); 134.66 (C(6)); 134.51 (C(7")); 130.57 (C(11")); 124.08 (C(6")); 123.57 (C(10")); 118.95 (C(2")); 108.51 (C(5)); 87.36 (C(1')); 84.75 (C(4')); 70.31 (C(3')); 61.24 (C(5')); 40.06 (C(2')); 39.17 (C(1")); 38.88 (C(8")); 38.58 (C(4")); 26.15 (C(5")); 25.67 (C(9")); 25.41 (C(12")); 17.48 (C(15")); 16.11 (C(14")); 15.75 (C(13")); 12.84 (CH₃). Anal. calc. for C₂₅H₃₈N₂O₅ (446.58): C 67.24, H 8.59, N 6.27; found: C 67.39, H 8.56, N 5.90.

*3-((E)-3,7-Dimethylocta-2,6-dienyl)-1-((2R,4R,5R)-4-hydroxy-5-(hydroxymethyl)tetrahydrofuran-2-yl)-5-methylpyrimidine-2,4(1H,3H)-dione (***9a***)*. Anhydr. thymidine (**7**, 0.97 g, 4 mmol) was reacted and worked up with geranylbromide (0.87 ml, 4.4 mmol) as described for compd. **9b.** Chromatography (silica gel, column: 6 x 10 cm, CH₂Cl₂-MeOH, 9:1, v/v) gave after evaporation of the main zone compd. **9a** (0.86 g, 2.27 mmol) as a yellowish amorphous solid. TLC (silica gel 60, CH₂Cl₂-MeOH 9:1, v/v): R_{f} 0.4. UV (MeOH): λₘₐₓ = 266 nm (ε = 7579 M⁻¹cm⁻¹). log *P*: 4.57+/-0.61. ¹H-NMR ((D₆)DMSO): 7.75 (*d*, ³J(CH₃, H-C(6)) = 1.3, H-C(6)); 6.21 (Ψ*t*, ³J(H-C(1'), H_{α}-C(2')) = 7.0, ³J(H-C(1'), H_{β} -C(2') = 7.0, H-C(1')); 5.20 (*d*, ³J(OH-C(3'), H-C(3')) = 4.5, OH-C(3')); 5.11 (Ψ*t*, ³J(H-C(2''), H₂-C(1'') = 6.75, H-C(2")); 5.03 (*m,* (H-C(6")); 4.99 (Ψ*t*, ³J(OH-C(5'), H_{b}-C(5')) = 5.2, ³J(OH-C(5'), Hₐ-C(5')) = 5.2, OH-C(5')); 4.40 (*d*, ³J(H2-C(1''), H-C(2")) = 6.5, H₂-C(1'')); 4.25 (*ddd,* ³J(H-C(4'), H-C(3')) = 3.5, ³J(H-C(4'), H_{b}-C(5')) = 4.0, ³J(H-C(4'), Hₐ-C(5')) = 4.0, H-C(4')); 3.78 (Ψ*q*, ³J(H-C(3'), H_{α}-C(2')) = 3.78, ³J(H-C(3'), H_{β} -C(2')) = 3.78, ³J(H-C(3'), H-C(4')) = 3.78, H-C(3')); 3.61 (*ddd,* ³J(Hb-C(5'), H-C(4')) = 4.0, ³J(H_{b}-C(5'), OH-C(5')) = 4.0, ²J(H_{b}-C(5'), Hₐ-C(5')) = -12, H_{b}-C(5')); 3.55 (*ddd,* ³J(Hₐ-C(5'),H-C(4')) = 4.0, ³J(Hₐ-C(5'), OH-C(5')) = 5.0, ²J(Hₐ-C(5'), H_{b}-C(5')) = -12, Hₐ-C(5')); 2.09 (*dd*, ³J(H_{α}-C(2'), H-C(1')) = 7.0, ³J(H_{β}-C(2'), H-C(1')) = 7.0, ³J(H_{α}-C(2'), H-C(3')) = 4.8, ³J(H_{β}-C(2'), H-C(3')) = 4.8, ²J(H_{α}-C(2'), H_{β}-C(2)) = - 12.0, H₂-C(2')); 1.94 *(m,* (H₂-C(4'',5''), 2xCH₂); 1.82 (*d*, ³J(CH₃, H-C(6) = 1.0, CH₃)); 1.74 (s, H₃-C(9'')); 1.61 (s, H₃-C(8'')); 1.53 (s, H₃-C(10'')). ¹³C-NMR ((D₆)DMSO): 162.29 (C(4)); 150.17 (C(2)); 138.71 (C(3")); 134.61 (C(6)); 130.77 (C(7")); 123.73 (C(6")); 118.84 (C(2")); 108.44 (C(5)); 87.28 (C(1')); 84.67 (C(4')); 70.21 (C(3')); 61.16 (C(5')); 40.08 (C(2')); 39.41 (C(4")); 39.07 (C(1'')); 25.79 (C(5")); 25.32 (C(8")); 17.41 (C(10")); 16.05 (C(9")); 12.79 (CH₃). Anal. calc. for C₂₀H₃₀N₂O₅ (378,463): C 63.47, H 7.99, N 7.40; found: C 63.26, H 7.98, N 7.19.

*1-((2R,4R,5R)-5-((Bis(4-methoxyphenyl)(phenyl)methoxy)methyl)-4-hydroxytetrahydrofuran-2-yl)-5-methyl-3-((2E,6E)-3,7,11-trimethyldodeca-2,6,10-trienyl)pyrimidin-2,4(1H,3H)-dione (***10***b*). Compd. **9b** (0.45 g, 1 mmol) was co-evaporated twice with anhydr. pyridine (1 ml, each) and then dissolved in anhydr. pyridine (5 ml). 4,4'-Dimethoxytriphenylmethyl chloride (0.39 g, 1.15 mmol) was added under N₂ atmosphere, and the mixture was stirred for 24 h at ambient temperature. Then, the reaction was quenched by addition of MeOH (3 ml). After 10 min ice-cold 5 % aq. NaHCO₃ was added, and the soln. was extracted with CH₂Cl₂. The organic layer was dried (Na₂SO₄), filtered, and the solvent evaporated. The residue was dried in high *vacuo* until a yellowish foam formed. Chromatography (silica gel, column: 6 x 10 cm, CH₂Cl₂-MeOH, 99:1, v/v) gave after evaporation of the main zone compd. **10b** (0. 48 g, 65 %) as a yellowish glass. TLC (silica gel 60, CH₂Cl₂-MeOH, 99:1, v/v): R_{f} 0,4. UV (MeOH): λₘₐₓ 232 nm (ε = 26.900 M⁻¹cm⁻¹), λ 268 nm (ε = 13.400). log P: 12.17+/-0.64. ¹H-NMR ((D₆)DMSO): 7.55 (*d*, ³J(CH₃, H-C(6)) = 0.9, H-C(6)); 7.38 (*d*, ³J(H-C(8"'), H-C(9''')) = 7.25, H-C(8''')); 7.30 (*t*, ³J(H-C(10'''), H-C(9''')) = 7.41, ³J(H-C(10'''), H-C(9''')) = 7.41; 7.25 (*m*, ³J(H-C(3''',9''')); 6.88 (*d*, ³J(H-C(4'''), H-C(3''')) = 8.20, H-C(4''')); 6.24 (Ψ*t*, ³J(H-C(1'), H_{α}-C(2')) = 6.62, ³J(H-C(1'), H_{β} -C(2') = 6.62, H-C(1')); 5.30 (*d*, ³J(OH-C(3'), H-C(3')) = 4.4, OH-C(3')); 5.11 *(m,* (H-C(2")); 5.03 *(m,* (H-C(6"), H-C(10'''), 2xCH); 4.40 (*d*, ³J(H₂-C(1''), H-C(2")) = 5.0, H₂-C(1'')); 4.32 (*m*, (H-C(4')); 3.90 (*m*, (H-C(3')); 3.73 (s, H₃-C(6'''), 2×OCH₃); 3.21 (*m*, (H₂-C(5')); 2.21 (*m*, (H₂-C(2')); 2.03 (*m*, (H₂-C(5''')); 1.96 (*m*, H₂-C(8''), H₂-C(9'')); 1.89 (*m*, H₂-C(4'')); 1.74 (*s*, H₃-C(5)); 1.61 (*s*, H₃-C(13'')); 1.53 (*s*, H₃-C(12'')); 1.52 (*s*, H₃-C(14'')); 1.49 (s, H₃-C(15'')). ¹³C-NMR ((D₆)DMSO): 161.23 (C(4)); 157.11 (C(5'''); 149.10 (C(2)); 143.61 (C(7''')); 137.74 (C(3")); 134.37 (C(2''')); 133.44 (C(6)); 129.50 (C(7")); 128.64 (C(3"')); 126.79 (C(9''')); 126.61 (C(8''')); 125.70 (C(11''')); 123.02 (C(6")); 122.51 (C(10")); 117.82 (C(2")); 112.17 (C(4''')); 107.71 (C(5)); 84.80 (C(1''')); 84.52 (C(1')); 83.70 (C(4')); 69.36 (C(3')); 62.63 (C(5')); 53.98 (C(6''')); 39.10 (C(2')); 37.81 (C(4")); 37.59 (C(1'')); 25.07 (C(5")); 24.61 (C(9")); 25.34 (C(8")); 16.42 (C(15")); 15.07 (C(14")); 14.69 (C(13")); 11.26 (CH₃). Anal. calc. for C₄₆H₅₆N₂O₇ (748,946): C 73.77, H 7.54, N 3.74; found: C 73.39, H 7.38, N 3.74.

*1-((2R,4R,5R)-5-((Bis(4-methoxyphenyl)(phenyl)methoxy)methyl)-4-hydroxytetrah ydrofuran-2-yl)-3-((E)-3, 7-dimethylocta-2, 6-dien-1-yl)-5-methylpyrimidin-2,4(1H,3H)-dione (***10***a*). Compd. **9a** (0.38 g, 1 mmol) was dried with anhydr. pyridine, reacted with 4,4'-dimethoxytriphenylmethyl chloride (0.39 g, 1.15 mmol), and worked up as described for compd. **10b.** Yield: 0.39 g (57 %) of 5b as a yellowish foam. TLC (silica 60, CH₂Cl₂-MeOH 99:1, v/v): R_{f} 0.57. UV (MeOH): λₘₐₓ = 231 nm (ε = 24.770 M⁻¹cm⁻¹), λ = 268 nm (ε = 12.200 M⁻¹cm⁻¹). log P: 10.14+/-0.61.
¹H-NMR ((D₆)DMSO-*d*₆): 7.55 (d, ³J(CH₃, H-C(6)) = 0.95, H-C(6)); 7.38 (d, ³J(H-C(8"'), H-C(9''')) = 7.57, H-C(8''')); 7.31 (*t*, ³J(H-C(10'''), H-C(9''')) = 7.72, ³J(H-C(10'''), H-C(9''')) = 7.72; 7.25 (*m*, ³J(H-C(3''',9'''), 4 x CH); 6.89 (*d*, ³J(H-C(4"'), H-C(3''')) = 8.98, H-C(4''')); 6.25 (Ψ*t*, ³J(H-C(1'), H_{α}-C(2')) = 6.78, ³J(H-C(1'), H_{β} -C(2') = 6.78, H-C(1')); 5.30 (*d*, ³J(OH-C(3'), H-C(3')) = 4.41, OH-C(3')); 5.11 (*m*, (H-C(2'')); 5.02 (*m*, (H-C(6'')); 4.40 (*d*, ³J(H₂-C(1''), H-C(2'')) = 5.0, H₂-C(1'')); 4.32 (*m*, (H-C(4')); 3.90 (Ψ*q*, ³J(H-C(3'), H_{α}-C(2')) = 3.90, ³J(H-C(3'), H_{β} -C(2')) = 3.90, ³J(H-C(3'), H-C(4')) = 3.90, H-C(3')); 3.74 (s, H₃-C(6'''), 2xOCH₃); 3.21 (*m,* (H₂-C(5')); 2.22 (*m,* (H₂-C(2')); 2.02 (*m,* (H₂-C(5'')); 1.93 (*m*, H₂-C(4'')); 1.74 (*s*, H₃-C(5)); 1.60 (*s*, H₃-C(9'')); 1.53 (*s*, H₃-C(8'')); 1.50 (*s*, H₃-C(10'')). ¹³C-NMR (D₆)DMSO-*d*₆): 162.28 (C(4)); 158.12 (C(5'''); 150.13 (C(2)); 144.62 (C(7''')); 144.62 (C(3")); 135.39 (C(2''')); 134.25 (C(6)); 130.82 (C(7")); 129.67 (C(3"')); 127.83 (C(9''')); 127.64 (C(8''')); 126.74 (C(10''')); 123.76 (C(6")); 118.80 (C(2")); 113.20 (C(4''')); 108.74 (C(5)); 85.83 (C(1''')); 85.54 (C(1')); 84.73 (C(4')); 70.38 (C(3')); 63.64 (C(5')); 55.01 (C(6''')); 40.14 (C(2')); 30.87 (C(4")); 38.62 (C(1'')); 25.80 (C(5")); 25.34 (C(8")); 17.43 (C(10")); 16.09 (C(9")); 12.29 (CH₃). Anal. calc. for C₄₁H₄₈N₂O₇ (680,829): C 72.33, H 7.11, N 4.11; found: C 72.16, H 7.00, N 3.84.

*(2R,3S,5R)-2-((Bis(4-methoxyphenyl)(phenyl)methoxy)methyl)-5-(5-methyl-2,4-dioxo-3-((2E, 6E)-3, 7,11-trimethyldodeca-2, 6,10-trien-1-yl)-3,4-dihydropyrimidin-1 (2H)-yl)tetrahydrofuran-3-yl (2-cyanoethyl) diisopropylphosphoramidite (***11b***)*. Compd. **10b** (0.32 g, 0.3 mmol) was co-evaporated twice from dry CH₂Cl₂ and dissolved in CH₂Cl₂ (15 ml). Then, N,N-diisopropylethylamine (126 µl, 0.72 mmol) and (chloro)(2-cyanoethoxy)(diisopropylamino)phosphine (156 µl, 0.72 mmol) were added under N₂ atmosphere. The reaction was stirred for 20 min at ambient temperature, and the reaction was then quenched by addition of ice-cold 5 % aq. NaHCO₃ (12 ml). The raw product was extracted with CH₂Cl₂, the solution was dried (Na₂SO₄) for 2 min, filtered and evaporated to dryness (bath temperature: < 25 °C), followed by drying in high vacuo for 5 min. Flash chromatography (0.5 bar, silica gel, column: 2 x 10 cm, CH₂Cl₂-acetone, 8:2, v/v, with 8 drops of Et₃N per I, total time of chromatography < 15 min) afforded the phosphoramidite **11b** (0.27 g, 67 %) as a colourless foam which was stored at -20 °C. TLC (silica gel 60, CH₂Cl₂-acetone, 8:2, v/v) R_{f} 0.87, 0.97 (diasteoisomers). ³¹P-NMR (CDCl₃): 149.055 (P_{R}), 148.528 (P_{S}).

*(2R, 3S, 5R)-2-((Bis(4-methoxyphenyl)(phenyl)methoxy)methyl)-5-(3-((E)-3, 7-dimethylocta-2, 6-dien-1-yl)-5-methyl-2,4-dioxo-3,4-dihydropyrimidin-1 (2H)-yl)tetrahydrofuran-3-yl (2-cyanoethyl) diisopropylphosphoramidite* (**11a**). Compound **11a** was prepared from **10a** (0.2 g, 0.3 mmol) as described for **11b.** Yield: 0.25 g (97 %) of a colourless foam. TLC (silica gel 60, CH₂Cl₂-acetone, 8:2, v/v) R_{f} 0.84, 0.94 (diastereoisomers). ³¹P-NMR (CDCl₃): 149.024 (P_{R}), 148.497 (P_{S}).

### Uridine- and Ribothymidine Derivatives

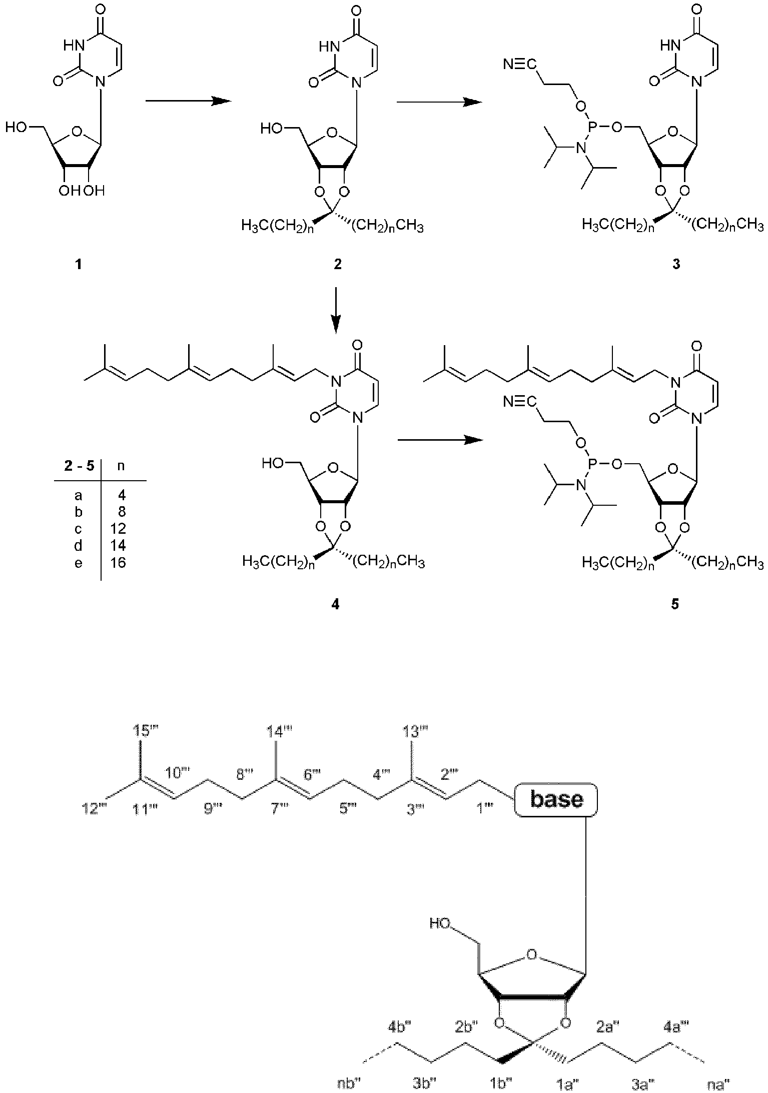

*General.* Starting compounds and solvents were purchased from the appropriate suppliers and were used as obtained. Chromatography: silica gel 60 (Merck, Germany). TLC: aluminum sheets, silica gel 60 F₂₅₄, 0.2 mm layer (*Merck,* Germany). NMR Spectra: AMX-500 spectrometer (*Bruker,* D-Rheinstetten); ¹H: 500.14 MHz, ¹³C: 125.76 MHz, and ³¹P: 101.3 MHz. Chemical shifts are given in ppm relative to TMS as internal standard for ¹H and ¹³C nuclei and external 85 % H₃PO₄; *J*
values in Hz. Elemental analyses (C, H, N) of crystallized compounds were performed on a VarioMICRO instrument (Fa. *Elementar,* D-Hanau). M.p.: *Stuart-*SMP3 apparatus (Fa. *Bibby Scientifis Limited,* UK-Staffordshire); uncorrected. UV Spectra: Cary 6000i spectrophotometer (*Varian,* D-Darmstadt).

*1-((3aR,4R,* 6R, *6aR)-6-(hydroxymethyl)-2,2-dipentyltetrahydrofuro[3,4-d][1,3]dioxol-4-yl)pyrimidin-2,4(1H,3H)-dione* (**25a**). Uridine (**24**; 0.76 g, 3.1 mmol) was dissolved in anhydr. DMF (10 ml), and undecan-6-one (0.80 ml, 3.9 mmol) as well as 4 M HCl in 1,4-dioxane (4 ml) and triethylorthoformate (1 ml) were added. The mixture was stirred for 24 h at room temp.. Subsequently, the solution was partitioned between CH₂Cl₂ (75 ml) and a sat. aq. NaHCO₂ soln. (50 ml). The organic phase was washed with dist. H₂O (100 ml) and separated. After drying over Na₂SO₄ the solvent was evaporated. Purification was performed by column chromatography (silica gel 60; column: 2 x 22 cm). A stepwise elution with 750 ml CH₂Cl₂/MeOH (99:1, v/v), followed by 250 ml CH₂Cl₂/MeOH (95:5, v/v) gave one main zone from which compd. **25a** (1.1 g, 2.69 mmol, 87 %) was isolated as a colourless foam, obtained upon evaporation and drying in high vacuo. UV(MeOH): λₘₐₓ = 260 nm (ε = 9.200 M⁻¹cm⁻¹). TLC (silica gel 60; CH₂Cl₂/MeOH, 95:5 (v/v)): R_{f}: 0.19. Anal. calc. for C₂₀H₃₂N₂O₆ (396.48): C, 60.59; H, 8.14; N, 7.07. Found: C, 60.20; H, 8.11; N, 6.97. ¹H-NMR (500.13 MHz, DMSO-*d₆*): 11.32 (s, H-N(3)); 7.77 (d, ³J(H-C(6), H-C(5)) = 8.2, H-C(6)); 5.83 (*d*, ³J(H-C(1'), H-C(2')) = 2.5, H-C(1')); 5.62 (dd, ³J(H-C(5), H-C(6)) = 8.0, ⁴J(H-C(5), H-N(3)) = 1.7, H-C(5)); 5.01 (*t*, ³J (OH-C(5')), H_{b}-C(5') = 5.04, ³J (HO-C(5'), Hₐ-C(5')) = 5.04, OH-C(5')); 4.89 (*dd*, ³J(H-C(2'), H-C(1')) = 2.8, ³J(H-C(2'), H-C(3')) = 6.6, H-C(2')); 4.74 ((*dd*, ³J(H-C(3'), H-C(4')) = 3.5, ³J(H-C(3'), H-C(2')) = 6.6, H-C(3')); 4.06 (q, 2x³J(H-C(4'), H₂-C(5')) = 4.4, ³J(H-C(4'), H-C(3')) = 4.4, H-C(4')); 3.56 (*m*, H₂-C(5')); 1.67 (*m*, H_{2(endo)}-C(1a")); 1.52 (*m*, H₂₍ₑₓₒ₎-C(1b")); 1.44-1.20 (*m*, 6xH_{2(endo)}-C(2a"- 4a"), 6xH₂₍ₑₓₒ₎-C(2b"- 4b"), 12H); 0.86 (*m*, 2xH₃-C(5a", 5b"), 6H). ¹³C-NMR (125.76 MHz, DMSO-*d₆*): δ. 163.06 (C(4)); 150.26 (C(2)); 141.94 (C(6)); 116.631 (C(acetal)); 101.65 (C(5)); 91.20 (C(1')); 86.66 (C(4')); 83.79 (C(3')); 80.73 (C(2')); 61.34 (C(5')); 36.34 (C(1")); 31.34 (C(3a")); 31.25 (C(3b")); 23.19 (C(2a")); 22.51 (C(2b")); 21.91 (C(4a")); 21.89 (C(4b")); 13.76 (C(5")).

*1-((3aR,4R,6R,6aR)-6-(hydroxymethyl)-2,2-dinonyltetrahydrofuro[3,4-d][1,3]dioxol-4-yl)pyrimidin-2,4(1H,3H)-dione* (***25b***)*.* Uridine (**24**; 0.76 g, 3.1 mmol) was dissolved in anhydr. DMF (10 ml), and nonadecan-10-one (1.11 g, 3.9 mmol), 4 M HCl in 1,4-dioxane (4 ml) and triethylortho formate (1 ml) as well as CH₂Cl₂ (6 ml) were added consecutively. The reaction mixture was stirred for 24 h at room temp. Subsequently, the mixture was partitioned between an aq. sat. NaHCO₃ soln (100 ml) and CH₂Cl₂ (100 ml). The organic layer was washed with dest. H₂O (100 ml), separated, dried over Na₂SO₄, and then evaporated to dryness. Purification of the raw product was performed by stepped gradient column chromatography (silica gel 60, column: 6.5 x 10 cm). A stepwise elution with 800 ml CH₂Cl₂/MeOH (99:1, v/v), followed by 200 ml of CH₂Cl₂/MeOH (95:5, v/v) gave one main zone from which compd. **25b** (1.50 g, 2.95 mmol, 95%) was isolated as a colourless foam, obtained upon evaporation and drying in high vacuo. TLC (silica gel 60; CH₂Cl₂/MeOH 95:5 (v/v)): *R*_{f}, 0.21. UV(MeOH): λₘₐₓ = 260 nm (ε = 9.600 M⁻¹cm⁻¹). M.p.: 69.8°C. Anal. calc. for C₂₈H₄₈N₂O₆ (508.69) C, 66.11; H, 9.51; N, 5.51. Found:C, 65.86; H, 9.50; N, 5.21. ¹H-NMR (500.13 MHz, DMSO-d₆): 11.33 (*s*, H-N(3)); 7.76 (*d*, ³J(H-C(6), H-C(5)) = 8.0, H-C(6)); 5.82 (*d*, ³J(H-C(1'), H-C(2')) = 2.5, H-C(1')); 5.62 (*d*, ³J(H-C(5), H-C(6)) = 8.0, H-C(5)); 5.02 (*m*, HO-C(5')); 4.89 (*dd*, ³J(H-C(2'), H-C(1')) = 2.5, ³J(H-C(2'), H-C(3')) = 6.5, H-C(2')); 4.73 (*dd*, ³J(H-C(3'), H-C(4')) = 3.5, ³J(H-C(3'), H-C(2')) = 6.5, H-C(3')); 4.05 (*q*, 2x³J(H-C(4'), H₂-C(5')) = 4.2, ³J(H-C(4'), H-C(3')) = 4.2, H-C(4')); 3.57 (*m*, H₂-C(5')); 1.66 (*m*, H_{2(endo)}-C(1a")); 1.51 (m, H₂₍ₑₓₒ₎-C(1b")); 1.30-1.20 (*m*, 7xH_{2(endo)}-C(2a"-8a"), 7xH₂₍ₑₓₒ₎-C(2b"-8b"), 28H); 0.85 (*m*, 2xH₃-C(9a", 9b"), 6H). ¹³C-NMR (125.76 MHz, DMSO-d₆): δ. 163.11 (C(4)); 150.28 (C(2)); 141.98 (C(6)); 116.63 (C(acetal)); 101.67 (C(5)); 91.23 (C(1')); 86.69 (C(4')); 83.82 (C(3')); 80.70 (C(2')); 61.35 (C(5')); 36.38 (C(1a")); 36.29 (C(1b")); 31.21 (C(7a")); 31.19 (C(7b")); 29.09 (C(3a")); 29.04 (C(3b")); 28.86 (C(4a")); 28.83 (C(4b")); 28.80 (C(5")); 28.59 (C(6a")); 28.58 (C(6b")); 23.52 (C(2a")); 22.88 (C(2b")); 22.01 (C(8a")); 22.00 (C(8b")); 13.85 (C(9")).

*1-((3aR,4R, 6R, 6aR)-6-(hydroxymethyl)-2,2-ditridecyltetrahydrofuro[3,4-d][1,3]dioxol-4-yl)pyrimidin-2,4(1H,3H)-dione* (***25c***)*.* Heptacosan-14-one (0.40 g, 1 mmol) was added to a soln. of anhydr. THF (14 ml), uridine (**24**; 1.22 g, 5 mmol), TsOH (0.19 g, 1 mmol) and triethylortho formate (0.85 ml, 5.1 mmol). The reaction mixture was refluxed for 24 h (75°C), and then triethylamine (0.6 ml) was added. To this mixture ice-cold 4% aq. NaHCO₃ (50 ml) was added and stirred for 15 min at room temp.. The mixture was washed with 100 ml of CH₂Cl₂ and 100 ml of dist. H₂O. The organic layer was dried (Na₂SO₄), filtered, and the solvent was evaporated. Compound **2c** was precipitated by addition of ice-cold MeOH on ice; the material was filtered and dried in high vacuo overnight. Yield: colourless solid (0.41 g, 0.66 mmol, 65.6%). TLC (silica gel 60; CH₂Cl₂/MeOH 95:5 (v/v): *R*_{f}: 0.24. UV(CH₂Cl₂): λₘₐₓ = 260 nm (ε = 9.340 M⁻¹cm⁻¹). M.p.: 90.1 °C. Anal. calc. for C₃₆H₆₄N₂O₆ (620.90): C, 69.64; H, 10.39; N, 4.51. Found: C, 69.77; H, 10.74; N, 3.92. ¹H-NMR (500 MHz, DMSO-*d₆*): 11.32 (*d*, ⁴J(H-N(3), (OH-C(5')) = 1.89, H-N(3)); 7.76 (*d*, ³J(H-C(6), H-C(5)) = 8.2, H-C(6)); 5.82 (*d*, ³J(H-C(1'), H-C(2')) = 2.5, H-C(1')); 5.61 (*dd,* ³J(H-C(5), H-C(6)) = 8.2, ⁴J(H-C(5'), H-N(3)) = 2.2, H-C(5)); 5.01 (*t*, 2x³J (OH-C(5'), H₂-C(5')) = 5.4, OH-C(5')); 4.88 (*dd*, ³J (H-C(2'), H-C(1')) = 2.5, ³J (H-C(2'), H-C(3')) = 6.6, H-C(2')); 4.73 (*dd*, ³J (H-C(3'), H-C(4')) = 3.5, ³J (H-C(3'), H-C(2')) = 6.6, H-C(3')); 4.05 (*q*, 2x³J(H-C(4'), H₂-C(5')) = 4.4 , ³J(H-C(4'), H-C(3')) = 4.4, H-C(4')); 3.55 (*m*, H₂-C(5')); 1.66 (*m*, H_{2(endo)}-C(1a")); 1.51 (*m*, H₂₍ₑₓₒ₎-C(1b")); 1.42-1.19 (*m*, 11xH_{2(endo)}-C(2a"-12a"), 11xH₂₍ₑₓₒ₎-C(2b"-12a"), 44H); 0.85 (*m*, 2xH₃-C(13a", 13b"), 6H). ¹³C-NMR (125.76 MHz, DMSO-*d₆*): δ. 163.07 (C(4)); 150.26 (C(2)); 141.94 (C(6)); 116.62 (C(acetal)); 101.66 (C(5)); 91.22 (C(1')); 86.68 (C(4')); 83.81 (C(3')); 80.68 (C(2')); 61.34 (C(5')); 36.38 (C(1a")); 36.22 (C(1b")); 31.21 (C(11")); 29.05 (C(3a")); 28.99 (C(3b")); 28.96-28.59 (C(4")-C(10")); 22.48 (C(2a")); 22.86 (C(2b")); 22.00 (C(12")); 13.84 (C(13")).

*1-(6-Hydroxymethyl-2,2-dipentadecyl-tetrahydro-furo[3,4-d][1,3]dioxol-4-yl)-1H-pyrimidin-2,4-dione* (***25d***). Hentriacontan-16-one (0.45 g, 1.0 mmol) was added to a soln. of anhydr. THF (14 ml), uridine (**24**; 1.22 g, 5 mmol), TsOH (0.19 g, 1.0 mmol) and triethylortho formate (0.85 ml, 5.1 mmol). This mixture was refluxed for 24 h (75°C), and triethylamine (0.6 ml) was added. The resulting mixture was poured into an aq., ice-cold 4% NaHCO₃ soln (50 ml) and stirred for 15 min at room temp.. The mixture was washed with CH₂Cl₂ (100 ml) and dist. H₂O (100 ml), dried over Na₂SO₄, filtered, and the solvent evaporated. The residue was triturated with ice-cold MeOH on ice which gave the solid product **25d** (0.36 g, 0.54 mmol, 53.7%) as a slightly yellowish solid which was dried in high vacuo. TLC (silica gel 60; CH₂Cl₂/MeOH 95:5 (v/v): R_{f}, 0.26. UV(CH₂Cl₂): λₘₐₓ = 260 nm (ε = 8.350 M⁻¹cm⁻¹). M.p.: 93°C. Anal. calc. for C₄₀H₇₂N₂O₆ (677.01): C, 70.96; H, 10.72; N, 4.14. Found: C, 71.08; H, 11.06; N, 3.74. ¹H-NMR (500 MHz, DMSO-d₆): 11.32 (s, H-N(3)); 7.77 (*d*, ³J(H-C(6), H-C(5)) = 8.0, H-C(6)); 5.83 (d, ³J(H-C(1'), H-C(2')) = 2.5, H-C(1')); 5.62 (*d*, ³J(H-C(5), H-C(6)) = 8.0, H-C(5)); 5.01 (t, 2x³J(OH-C(5'), H₂-C(5')) = 5.0, OH-C(5')); 4.88 (*dd*, ³J(H-C(2'), H-C(1')) = 2.5, ³J(H-C(2'), H-C(3')) = 6.5, H-C(2')); 4.73 (*dd*, ³J(H-C(3'), H-C(4')) = 3.5, ³J(H-C(3'), H-C(2')) = 6.3, H-C(3')); 4.05 (*q*, 2x³J(H-C(4'), H₂-C(5')) = 4.4, ³J(H-C(4'), H-C(3')) = 4.4, H-C(4')); 3.55 (*m*, H₂-C(5')); 1.66 (*m*, H_{2(endo)}-C(1a")); 1.51 (*m*, H₂₍ₑₓₒ₎-C(1b")); 1.41-1.16 (*m*, 13xH_{2(endo)}-C(2a"-14a"), 13xH₂₍ₑₓₒ₎-C(2b"-14a"), 52H); 0.85 (*m*, 2xH₃-C(15a", 15b"), 6H). ¹³C-NMR (125.76 MHz, DMSO-*d₆*): δ. 162.69 (C(4)); 150.03 (C(2)); 141.49 (C(6)); 116.52 (C(acetal)); 101.45 (C(5)); 91.02 (C(1')); 86.44 (C(4')); 83.62 (C(3')); 80.50 (C(2')); 61.18 (C(5')); 36.32 (C(1a")); 36.06 (C(1b")); 30.90 (C(13")); 28.77 (C(3a")); 28.73 (C(3b")); 28.66-28.28 (C(4")-C(12")); 23.18 (C(2a")); 22.63 (C(2b")); 21.66 (C(14")); 13.46 (C(15")).

*1-(2,2-Diheptadecyl-6-hydroxymethyl-tetrahydro-furo[3,4-d][1,3]dioxol-4-yl)-1H-pyrimidin-2,4-dione* (***25e***). Pentatriacontan-18-one (0.5 g, 0.99 mmol) was added to a soln. of anhydr. THF (14 ml), uridine (**24**; 1.22 g, 5 mmol), TsOH (0.19 g, 1.0 mmol), and triethylortho formate (0.85 ml, 5.1 mmol). The mixture was refluxed for 24 h at 75°C. Then, triethylamine (0.6 ml) was added, and the resultant mixture was poured into an ice-cold aq. 4% NaHCO₃ soln. (50 ml). This soln. was stirred for 15 min at room temp. The organic layer was washed with CH₂Cl₂ (100 ml) and dist. H₂O (100 ml), dried over Na₂SO₄, filtered, and the solvent was evaporated. The residue was triturated with ice-cold MeOH on ice which gave the solid product. The colourless product **25e** was dried over night in high vacuo. Yield: 0.45 g (0.61 mmol, 61.4%). TLC (silica gel 60; CH₂Cl₂/MeOH 95:5 (v/v)): R_{f}: 0.21. UV(CH₂Cl₂): λₘₐₓ = 260 nm (ε = 9.320 M⁻¹cm⁻¹). M.p.: 89.7°C. Anal. calc. for C₄₄H₈₀N₂O₆ (733.12) C, 72.09; H, 11.00; N, 3.82. Found: C, 71.70; H, 11.14; N, 3.81. ¹H-NMR (500 MHz, DMSO-*d₆*, 60°C): 11.16 (*s*, H-N(3)); 7.74 (*d*, ³J(H-C(6), H-C(5)) = 8.0, H-C(6)); 5.84 (*d*, ³J(H-C(1'), H-C(2')) = 2.5, H-C(1')); 5.60 (*d*, ³J(H-C(5'), H-C(6)) = 8.2, H-C(5)); 4.88 (*m*, H-C(2') & OH-C(5'), 2H); 4.75 (*dd*, ³J(H-C(3'), H-C(4')) = 3.5, ³J(H-C(3'), H-C(2')) = 6.5, H-C(3')); 4.07 (*q*, 2x³J(H-C(4'), H₂-C(5')) = 4.3, ³J(H-C(4'), H-C(3')) = 4.3, H-C(4')); 3.53-3.63 (*m*, H₂-C(5')); 1.68 (*m*, H₂-C(1a")); 1.53 (*m*, H₂-C(1b")); 1.43-1.19 (*m*, 15xH_{2(endo)}-C(2a"-16a"), 15xH₂₍ₑₓₒ₎-C(2b"-16b"), 60H); 0.86 (*m,* 2xH₃-C(17a", 17b"), 6H). ¹³C-NMR (125.76 MHz, DMSO-*d₆*): δ. 162.69 (C(4)); 150.03 (C(2)); 141.50 (C(6)); 116.52 (C(acetal)); 101.45 (C(5)); 91.02 (C(1')); 86.44 (C(4')); 83.62 (C(3')); 80.50 (C(2')); 61.18 (C(5')); 36.32 (C(1a")); 36.05 (C(1b")); 31.19 (C(15")); 28.76 (C(3a")); 28.72 (C(3b")); 28.79-28.25 (C(4")-C(14")); 23.17 (C(2a")); 22.62 (C(2b")); 21.66 (C(16")); 13.46 (C(17")).

*(((3aR,4R,6R,6aR)-6-(2,4-dioxo-3,4-dihydropyrimidin-1(2H)-yl)-2,2-dipentyltetrahydrofuro[3,4-d][1,3]dioxol-4-yl)methyl) 2-Cyanoethyl N,N-diisopropylphosphoramidite* (***26a***). Compound **25a** (0.214 g, 0.3 mmol) was dissolved in distilled CH₂Cl₂ (15 ml). Under Ar-atmosphere N,N-diisopropylethylamine (125 µl, 0.72 mmol) and 2-cyanoethyldiisopropylchlorophosphoramidite (156 µl, 0.6 mmol) were then added, and the mixture was stirred for 17 min at room temp.. The reaction was quenched by addition of an ice-cold aq. 5% NaHCO₃ soln. (12 ml), and the mixture was extracted with CH₂Cl₂ (15 ml). The combined organic layers were dried (1 min, Na₂SO₄), filtered, evaporated to dryness (25°C), and the raw product was further dried in high vacuo at room temp.. Column chromatography (silica gel 60, column: 2 x 10 cm, CH₂Cl₂/acetone 8:2 (v/v)), containing 8 drops of triethylamine per I) gave one main zone which was pooled, evaporated and dried in high vacuo. Yield: 0.13 g (0.22 mmol, 71%) of a colourless oil which was stored at -20°C. TLC (silica gel 60; CH₂Cl₂/acetone 8:2 (v/v)): *R*_{f}: 0.66. ³¹P-NMR (202.45 MHz, CDCl₃): δ. 149.40 (P_{R}), 149.30 (P_{S}).

*(((3aR,4R,6R,6aR)-6-(2,4-dioxo-3,4-dihydropyrimidin-1(2H)-yl)-2,2-dinonyltetrahydrofuro[3,4-d][1,3]dioxol-4-yl)methyl) 2-Cyanoethyl N,N-diisopropylphosphoramidite* (***26b***). Compound **25b** (0.153 g, 0.3 mmol) was reacted to the phosphoramidite **26b** as described for **25a.** Yield: 0.157 g (0.22 mmol, 73 %) of a colourless oil which was stored at -20°C. TLC (silica 60; CH₂Cl₂/acetone 8:2 (v/v)): *R*_{f}: 0.89. ³¹P-NMR (202.45 MHz, CDCl₃): δ. 149.46 (P_{R}), 149.37 (P_{S}).

*(((3aR,4R,6R,6aR)-6-(2,4-dioxo-3,4-dihydropyrimidin-1(2H)-yl)-2,2-diheptadecyltetrahydrofuro[3,4-d][1,3]dioxol-4-yl)methyl) 2-Cyanoethyl N,N-diisopropylphosphoramidite* (***26e***). Compound **25e** (0.22 g, 0.3 mmol) was reacted to the phosphoramidite **26e** as described for **26a.** Yield: 0.1 g (0.17 mmol, 57%) of a colourless oil which was stored at -20°C. TLC (silica gel 60; CH₂Cl₂/acetone 8:2 (v/v)): *R*_{f}: 0.81. ³¹P-NMR (202.45 MHz,. CDCl₃): δ. 149.46 (P_{R}), 149.38 (P_{S}).

*1-((3aR,4R, 6R, 6aR)-6-(hydroxymethyl)-2,2-dinonyltetrahydrofuro[3,4-d][1,3]dioxol-4-yl)-3-((2E,6E)-3,7,11-trimethyldodeca-2,6,10-trien-1-yl)pyrimidin-2,4(1H,3H)-dione (27b).* Compound **25b** (0.5 g, 0.98 mmol) was dissolved in anhydr. DMF (14 ml), and K₂CO₃ (1 g, 7.24 mmol) was added. Subsequently, under Ar-atmosphere trans-trans-farnesylbromide (0.35 ml, 1.1 mmol) was added dropwise within 10 min. The reaction mixture was stirred for 24 h at room temp. under the exclusion of light. Then, the mixture was filtered and partitioned between dest. H₂O (225 ml) and CH₂Cl₂ (150 ml). The organic phase was separated, dried over Na₂SO₄, and filtered. The soln. was evaporated to dryness and further dried in high vacuo. Column chromatography (silica gel 60, column: 2 x 27 cm, CH₂Cl₂ and MeOH, 99:1 (v/v)) and evaporation of the main fractions gave compd. **27b** (0.533 g, 0.75 mmol, 77%) as colourless oil. TLC (silica gel 60; CH₂Cl₂/MeOH 95:5 (v/v)): *R*_{f}, 0.59. UV(MeOH): λₘₐₓ = 260 nm (ε = 7010 M⁻¹cm⁻¹). Anal. calc. for C₄₃H₇₂N₂O₆ (713.04): C, 72.43; H, 10.18; N, 3.93. Found: C, 72.59; H, 10.58; N, 3.96. ¹H-NMR (500.13 MHz, DMSO-*d₆*): 7.81 (*d*, ³J(H-C(6), H-C(5)) = 8.0, H-C(6)); 5.87 (*d*, ³J(H-C(1'), H-C(2')) = 2.2, H-C(1')); 5.74 (*d*, ³J(H-C(5), H-C(6)) = 8.0, H-C(5)); 5.11 (*t*, 2x³J (OH-C(5'), H₂-C(5')) = 6.5, OH-C(5')); 5.08-5.00 (*m*, H-C(2"', 6"', 10"'), 3H); 4.87 (*dd*, ³J(H-C(2'), H-C(1')) = 2.5, ³J(H-C(2'), H-C(3')) = 6.6, H-C(2')); 4.73 (*dd*, ³J(H-C(3'), H-C(4')) = 3.0, ³J(H-C(3'), H-C(2')) = 6.5, H-C(3')); 4.41-4.38 (*m*, H₂-C(1"')); 4.09 (*q*, 2x³J(H-C(4'), H₂-C(5')) = 4.2, ³J(H-C(4'), H-C(3')) = 4.2, H-C(4')); 3.61-3.51 (*m*, H₂-C(5')); 2.03 (*m*, H₂-C(5"')); 2.00-1.92 (*m*, H₂-C(8"', 9"'), 4H); 1.92-1.87 (*m*, H₂-C(4'")); 1.73 (*s*, H₃-C(13"')); 1.69-1.66 (*m,* H₂-C(1a")); 1.63 (s, H₃-C(12"')); 1.54 (s, H₃-C(14"')); 1.51 (*m*, H₂-C(1b")); 1.42-1.18 (*m*, 7xH_{2(endo)}-C(2a"-8a"), 7xH₂₍ₑₓₒ₎-C(2b"-8b"), 28H); 0.85 (*m*, 2xH₃-C(9a", 9b"), 6H). ¹³C-NMR (125.76 MHz, DMSO-*d₆*): δ. 161.55 (C(4)); 150.20 (C(2)); 140.18 (C(6)); 138.81 (C(3"')); 134.45 (C(7"')); 130.47 (C(11"')); 124.01 (C(6"')); 123.49 (C(10"')); 118.69 (C(2"')); 116.56 (C(acetal)); 100.87 (C(5)); 92.09 (C(1')); 86.79 (C(4')); 83.99 (C(3')); 80.72 (C(2')); 61.28 (C(5')); 39.04 (C(8"')); 38.74 (C(4"')); 38.17 (C(1"')); 36.36 (C(1a")); 36.31 (C(1b")); 31.19 (C(7a")); 31.16 (C(7b")); 29.07 (C(3a")); 29.02 (C(3b")); 28.83 (C(4a")); 28.78 (C(4b")); 28.58 (C(5a")); 28.56 (C(5b")); 28.58 (C(6a"); 28.59 (C(6b")); 26.09 (C(5"')); 25.62 (C(9"')); 25.34 (C(12"')); 23.48 (C(2a")); 22.83 (C(2b")); 21.99 (C(8")); 17.41 (C(15"')); 16.06 (C(14"')); 15.66 (C(13"')); 13.81 (C(9")).

*(((3aR, 4R, 6R, 6aR)-6-(2,4-dioxo-3-((2E,6E)-3,7,11-trimethyldodeca-2,6,10-trien-1-yl)-3,4-dihydropyrimidin-1(2H)-yl)-2,2-dinonyltetrahydrofuro[3,4-d][1,3]dioxol-4-yl)methyl) (2,4-dimethylpentan-3-yl) 2-Cyanoethyl N,N-diisopropylphosphoramidite* (***28b***). Compound **27b** (0.214 g, 0.3 mmol) was reacted to the phosphoramidite **28b** as described for **26a.** Yield: 0.253 g (0.26 mmol, 87 %) of a colorless oil which was stored at -20°C. TLC (silica gel 60; CH₂Cl₂/acetone 8:2 (v/v)): *R*_{f}: 0.97. ³¹P-NMR (202.45 MHz,. CDCl₃): δ. 149.34 (P_{R}), 149.31 (P_{S}).

### Ribothymidine Derivatives

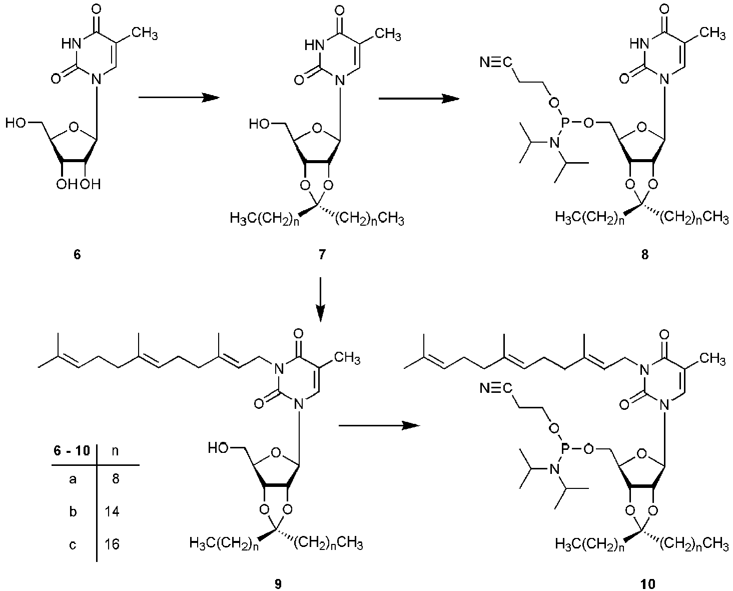

*1-(6-Hydroxymethyl-2,2-dinonyl-tetrahydro-furo[3,4-d][1,3]dioxol-4-yl)-5-methyl-1H-pyrimidin-2,4-dione (**30a**).* Anhydrous methyluridine (**29**; 0.77 g, 3 mmol) was dissolved in dry DMF (10 ml). Then, nonadecan-10-one (1.13 g, 4 mmol), dissolved in CH₂Cl₂ (10 ml), triethylorthoformate (1 ml), and 4 M HCl in 1,4-dioxane (4 ml) were added. The mixture was stirred at room temp. for 24 h. Subsequently, the mixture was partinioned between an aq. sat. Na₂CO₃ soln. (100 ml) and CH₂Cl₂ (100 ml). The organic phase was separated, dried over Na₂SO₄, filtered and evaporated to dryness. Traces of DMF were removed by repeated evaporation from CH₂Cl₂. The residue was dried in high vacuo overnight. The resulting colourless foam was purified by chromatography (silica gel 60, column: 6.5 x 10 cm). Elution with CH₂Cl₂/MeOH, 95:5 (v/v) gave one main zone which was pooled, the solvent was evaporated, and the residue was dried in high vacuo. Colourless oil (1.3 g, 83%). TLC (silica gel 60, CH₂Cl₂/MeOH, 95:5, (v/v)): R_{f}, 0.31. UV(MeOH): λₘₐₓ = 265 nm (ε = 10600 M⁻¹ cm⁻¹). Anal. calc. for C₂₉H₅₀N₂O₆ (522,73): C, 66.63; H, 9.64; N, 5.36. Found: C, 66.47; H, 9.272; N, 5.25. ¹H-NMR (DMSO-d₆): 11.34 (*s*, H-N(3)); 7.63 (s, H-C(6)); 5.83 (*d*, ³J(H-C(1'),H-C(2') = 2.5, H-C(1')); 5.02 (*t*, ³J(OH-C(5'), H₂-C(5')) = 5.0, (OH-C(5')); 4.88 (*dd*, ³J (H-C(2'),H-C(1') = 3.0, ³J (H-C(2'), H-C(3') = 6.5, H-C(2')); 4.75 (*dd*, ³J(H-C(3'), H-C(2') = 6.5; ³J(H-C(3'),H-C(4') = 3.5, H-C(3')); 4.02 (*m*, ³J(H-C(4'), H-C(3') = 3.5; ³J (H-C(4'), H₂C(5') = 4.5, H-C(4')); 3.56 *(m,* H₂-C(5')); 1.76 (s, 3H, CH₃); 1.66 (*m*, H_{2(endo)}-C(1a")); 1.52 (*m*, H₂₍ₑₓₒ₎-C(1b")); 1.38 (*m*, H_{2(endo)}-C(2a")); 1.24 (*m*, 6xH_{2(endo)}-C(3a"-8a"), 7xH₂₍ₑₓₒ₎-C(2b"-8b"), 26H); 0.85 (*m*, 2xH₃-C(9a", 9b"), 6H). ¹³C-NMR (DMSO-d₆): δ. 163.66 (C(4)); 150.27 (C(2)); 137.50 (C(6)); 116.77 (C(acetal)); 109.38 (C(5)); 90.52 (C(1')); 86.24 (C(4')); 83.50 (C(3')); 80.58 (C(2')); 61.31 (C(5')); 36.33 (C(1a")); 36.28 (C(1b")); 31.17 (C(7a")); 31.16 (C(7b")); 29.06 (C(3a")); 29.01 (C(3b")); 28.83 (C(4a")); 28.81 (C(4b")); 28.80 (C(5")); 28.77 (C(6a")); 28.55 (C(6b")); 23.48 (C(2a")); 22.86 (C(2b")); 21.97 (C(8a")); 21.96 (C(8b")); 13.82 (C(9a")); 13.81 (C(9b")); 11.94 (CH₃-(base)).

*1-(6-Hydroxymethyl-2,2-dipentadecyl-tetrahydro-furo[3,4-d][1,3]dioxo-4-yl)-5-methyl-1H-pyrimidin-2,4-dione* (***30b***)*.* Hentriacontan-16-one (0.45 g, 1 mmol) was added to a soln. of methyluridine (**29**; 1.29 g, 5 mmol), TsOH (0.19 g, 1 mmol), triethylorthoformate (0.83 ml, 5 mmol) in tetrahydrofurane (14 ml). This reaction mixture was heated to 75°C under reflux for 24 h. Then, triethylamine (0.6 ml) was added and the resultant mixture was poured into an ice-cold aq. 4% NaHCO₃ soln. (50 ml). After stirring for 15 min at room temperature, the reaction mixture was partinioned between CH₂Cl₂ (100 ml) and H₂O (100 ml). The organic layer was separated, dried over Na₂SO₄, filtered, and the solvent was evaporated. The resulting oil was triturated with ice-cold MeOH on an ice bath, which caused precipitation of compd. **30b** as a colourless solid. The latter was filtered off, and the filtrate was evaporated yielding another portion of solid **30b.** Total yield: 0.509 g, 74% of a yellowish solid. TLC (silica gel 60, CH₂Cl₂/MeOH 95:5 (v/v)): *R*_{f}, 0.24. UV(CH₂Cl₂): λₘₐₓ = 263 nm (ε = 12250 M⁻¹ cm⁻¹). M.p.: < 70°C. Anal. calc. for C₄₁H₇₄N₂O₆ (691.04): C, 71.26; H, 10.79; N, 4.05. Found: C, 72.39; H, 11.48; N, 3.33. ¹H-NMR (DMSO-d₆): 11.09 (*s*, H-N(3)); 7.58 (*s*, H-C(6)); 5.83 (*d*, ³J(H-C(1'),H-C(2') = 2.5, H-C(1')); 5.01 (*t*, 2x³J (OH-C(5'), H₂-C(5')) = 5.0, (OH-C(5')); 4.89 (*dd*, ³J (H-C(2'),H-C(1') = 2.8, ³J (H-C(2'), H-C(3') = 6.6, H-C(2')); 4.77 (*dd,* ³J(H-C(3'), H-C(2') = 6.6, ³J(H-C(3'),H-C(4') = 3.5, H-C(3')); 4.05 (*q*, 2x ³J(H-C(4'), H₂-C(5') = 4.4, ³J(H-C(4'), H-C(3') = 4.4, H-C(4')); 3.60 (*m*, H₂-C(5')); 1.79 (*s*, H₃-C(base)); 1.66 *(m,* H_{2(endo)}-C(1a")); 1.52 (*m*, H₂₍ₑₓₒ₎-C(1b")); 1.43-1.19 (*m*, 13xH_{2(endo)}-C(2a"-14a"), 13xH₂₍ₑₓₒ₎-C(2b"-14b"), 52H); 0.85 (*m*, 2xH₃-C(15a", 15b"), 6H). ¹³C-NMR (DMSO-*d₆*): δ. 163.14 (C(4)); 149.91 (C(2)); 136.94 (C(6)); 116.59 (C(acetal)); 109.07 (C(5)); 90.50 (C(1')); 86.03 (C(4')); 83.31 (C(3')); 80.38 (C(2')); 61.12 (C(5')); 36.32 (C(1a")); 36.03 (C(1b")); 30.74 (C(13")); 28.63 (C(3a")); 28.61 (C(3b")); 28.44-28.10 (C(4")-C(12")); 23.02 (C(2a")); 22.52 (C(2b")); 21.48 (C(14")); 13.24 (C(15")); 11.33 (CH₃-(base)).

*1-(2,2-Diheptadecyl-6-hydroxymethyl-tetrahydro-furo[3,4-d][1,3]dioxo-4-yl)-5-methyl-1H-pyrimidin-2,4-dione* (***30c***)*.* Pentatriacontan-18-one (0.50 g, 1 mmol) was added to a soln. of methyluridine (**29**; 1.29 g, 5 mmol), TsOH (0.19 g, 1 mmol), triethylorthoformate (0.83 ml, 5 mmol) in tetrahydrofurane (10 ml). This reaction mixture was heated to 75°C under reflux for 24 h. Then, triethylamine (0.6 ml) was added and the mixture was poured into an ice-cold aq. 4% NaHCO₃ soln. (50 ml). After stirring for 15 min at room temperature, the reaction mixture was partinioned between CH₂Cl₂ (100 ml) and H₂O (100 ml). The organic layer was separated, dried over Na₂SO₄, filtered, and the solvent was evaporated. The resulting oil was triturated with cold MeOH which caused precipitation of raw **30c.** The product was filtered off, and the filtrate was evaporated to yield a further crop of **30c.** Total yield: (0.5 g, 68%). TLC (silica gel 60, CH₂Cl₂/MeOH 95:5 (v/v)): *R*_{f}, 0.19. UV(CH₂Cl₂): λₘₐₓ = 263 nm (ε = 14380 M⁻¹ cm⁻¹). M.p.: 72°C. Anal. calc. for C₄₅H₈₂N₂O₆ (747.14): C, 72.34; H, 11.06; N, 3.75. Found: C, 72.39; H, 11.48; N, 3.33. ¹H-NMR (DMSO-*d₆*): 11.31 (*s*, H-N(3)); 7.62 (*s*, H-C(6)); 5.84 (*d*, ³J(H-C(1'),H-C(2') = 2.5, H-C(1')); 5.02 (*t*, 2x³J(OH-C(5'), H₂-C(5')) = 6.5, (OH-C(5')); 4.88 (*dd,* ³J (H-C(2),H-C(1') = 2.5, ³J (H-C(2'), H-C(3') = 6.6, H-C(2')); 4.75 (*dd*, ³J(H-C(3'), H-C(2') = 6.6, ³J(H-C(3'),H-C(4') = 3.5, H-C(3')); 4.02 (*dd*, 2x³J (H-C(4'), H₂-C(5') = 4.2, ³J(H-C(4'), H-C(3') = 4.2, H-C(4')); 3.57 (*m*, H₂-C(5')); 1.76 (*s*, H₃-C(base)); 1.66 (m, H_{2(endo)}-C(1a")); 1.51 (m, H₂₍ₑₓₒ₎-C(1b")); 1.37 (*m*, 2H_{endo}-C(2a")); 1.24 *(m,* 14xH_{2(endo)}-C(3a"-16a"), 15xH₂₍ₑₓₒ₎-C(2b"-16b"), 58H); 0.85 (*m*, 2 x H-C(17a", 17b"), 6H). ¹³C-NMR (DMSO-d₆): δ. 163.33 (C(4)); 150.04 (C(2)); 137.14 (C(6)); 116.64 (C(acetal)); 109.18 (C(5)); 90.48 (C(1')); 86.09 (C(4')); 83.38 (C(3')); 80.44 (C(2')); 61.18 (C(5')); 36.31 (C(1a")); 36.06 (C(1b")); 30.90 (C(15")); 28.75 (C(3a")); 28.71 (C(3b")); 28.59-28.27 (C(4")-C(14")); 23.17 (C(2a")); 22.63 (C(2b")); 21.66 (C(16")); 13.46 (C(17")); 11.56 (CH₃-(base)). *(((3aR,4R,6R,6aR)-6-(5-methyl-2,4-dioxo-3,4-dihydropyrimidin-1(2H)-yl)-2,2-dinonyltetrahydrofuro[3,4-d][1,3]dioxol-4-yl)methyl) 2-Cyanoethyl N,N-diisopropyl-phosphoramidite (**31a**)*. Compound **30a** (0.157 g, 0.3 mmol) was reacted to the phosphoramidite **31a** as described for **26a.** Yield: 0.155 g (71 %) of a colorless oil which was stored at -20°C. TLC (silica gel 60; CH₂Cl₂/acetone 8:2 (v/v)): *R*_{f}: 0.88. ³¹P-NMR (202.45 MHz, CDCl₃): δ,149.36 (P_{R}), 149.22 (P_{S}).

*(((3aR,4R,6R,6aR)-2,2-diheptadecyl-6-(5-methyl-2,4-dioxo-3,4-dihydropyrimidin-1(2H)-yl)tetrahydrofuro[3,4-d][1,3]dioxol-4-yl)methyl) 2-Cyanoethyl N,N-diisopropylphosphoramidite* (**31c**). Compound **30c** (0.214 g, 0.3 mmol) was reacted to the phosphoramidite **31c** as described for **26a.** Yield: 0.20 g (0.21 mmol, 70 %) of a colourless oil which was stored at -20°C. TLC (silica 60, CH₂Cl₂/acetone 8:2 (v,v)): R_{f}: 0.87. ³¹P-NMR (101.25 MHz, CDCl₃): δ. 149.31 (P_{R}), 149.17 (P_{S}).

*1-(6-Hydroxymethyl-2,2-dinonyl-tetrahydro-furo[3,4-d][1,3]dioxol-4-yl)-5-methyl-3-(3,7,11-trimethyl-dodeca-2,6,10-trienyl)-1H-pyrimidin-2,4-dione* (***32a***). Compound **30a** (0.52 g, 1 mmol) was dissolved in dry DMF (14 ml), and anhydr. K₂CO₃ (1 g, 7.24 mmol) was added. Then, *trans-trans*-farnesylbromide (0.35 ml, 1.1 mmol) was added drop-wise, and the mixture was stirred under Ar-atmosphere for 24 h under the exclusion of light. Then, the mixture was partitioned between H₂O (200 ml) and CH₂Cl₂ (100 ml). The organic layer was separated, dried over Na₂SO₄, filtered, and the solvent was evaporated. Traces of DMF were removed by drying in high vacuo overnight. Chromatography (silica gel 60, column: 2 x 21 cm, CH₂Cl₂/MeOH, 99:1 (v/v)) gave one main zone which was pooled and evaporated to dryness. Further drying in high vacuo gave compd. **32a** as a colourless oil (0.5 g, 68%). TLC (silica gel 60, CH₂Cl₂/MeOH, 95:5, (v/v)): R_{f}, 0.66. UV(MeOH): λₘₐₓ = 266 nm (ε = 8700 M⁻¹ cm⁻¹). Anal. calc. for C₄₄H₇₄N₂O₆ (727.07): C, 72.69; H, 10.26; N, 3.85. Found: C, 72.67; H, 9.925; N, 3.76. ¹H-NMR (DMSO-*d₆*): 7.68 (*s*, H-C(6)); 5.89 (*d*, ³J(H-C(1'),H-C(2') = 2.5, H-C(1'))); 5.12 (*t*, ³J(OH-C(5'), H₂-C(5')) = 5.0, OH-C(5')); 5.07-5.00 (*m*, H-C(2"_{'}, 6"', 10"'), 3H); 4.85 (*dd*, ³J (H-C(2'),H-C(1') = 2.8, ³J (H-C(2'), H-C(3') = 6.6, H-C(2')); 4.76 (*dd*, ³J(H-C(3'), H-C(2') = 6.6; ³J(H-C(3'),H-C(4') = 3.5, H-C(3')); 4,39 (*m*, H₂-C(1"')); 4.06 (*m*, H-C(4')); 3.62-3.52 (*m*, H₂-C(5')); 2.03 (m, H₂-C(5"')); 1.99-1.92 (*m*, H₂-C(8"', 9"'), 4H); 1.92-1.86 (*m*, H₂-C(4"')); 1.81 (s, H₃-C(base)); 1.74 (s, H₃-C(13"')); 1.69-1.64 (*m*, H_{2(endo)}-C(1a")); 1.63 (s, H₃-C(12"')); 1.54 (*s*, H₃-C(14"')); 1.52 (*m*, H₂₍ₑₓₒ₎-C(1b")); 1.43-1.17 *(m,* 7xH_{2(endo)}-C(2a"-8a"), 7xH₂₍ₑₓₒ₎-C(2b"-8b"), 28H); 0.85 (*m*, 2xH₃-C(9a", 9b"), 6H). ¹³C-NMR (DMSO-*d₆*): δ. 162.27 (C(4)); 150.09 (C(2)); 138.71 (C(6)); 135.71 (C(3"')); 134.42 (C(7"')); 130.45 (C(11"')); 123.98 (C(6"')); 123.46 (C(10"')); 118.75 (C(2"')); 116.74 (C(acetal)); 108.57 (C(5)); 91.42 (C(1')); 86.34 (C(4')); 83.64 (C(3')); 80.61 (C(2')); 61.25 (C(5')); 38.98 (C(8"')); 38.76 (C(4"')); 38.52 (C(1"')); 36.30 (C(1")); 31. 15 (C(7a")); 31.13 (C(7b")); 29.03 (C(3a")); 28.97 (C(3b")); 28.80 (C(4a")); 28.74 (C(4b")); 28.55 (C(5a")); 28.52 (C(5b")); 26.06 (C(6a")); 25.55 (C(6b")); 25.32 (C(5"')); 23.44 (C(9"')); 22.80 (C(12"')); 21.95 (C(2a")); 21.93 (C(2b")); 17.37 (C(8")); 16.03 (C(15"')); 15.64 (C(14")); 13.79 (C(13"')); 13.78 (C(9")); 12.59 (H₃C-(base)).

*(((3aR,4R,6R,6aR)-6-(5-methyl-2,4-dioxo-3-((2E,6E)-3,7,11-trimethyldodeca-2,6,10-trien-1-yl)-3,4-dihydropyrimidin-1(2H)-yl)-2,2-dinonyltetrahydrofuro[3,4-d][1,3]dioxol-4-yl)methyl) 2-Cyanoethyl N,N-diisopropylphosphoramidite* (***33a***)*.* Compound **32a** (0.22 g, 0.3 mmol) was reacted to the phosphoramidite **33a** as described for **26a.** Yield: 0.2 g (0.22 mmol, 77.9%) of a colourless oil which was stored at -20°C. TLC (silica gel 60; CH₂Cl₂/acetone 8:2 (v/v)): *R*_{f}: 0.97. ³¹P-NMR (202.45 MHz,. CDCl₃): δ. 149.28 (P_{R}), 149.26 (P_{S}).

### Lipidderivatives and Hydrophobization of Thymidine

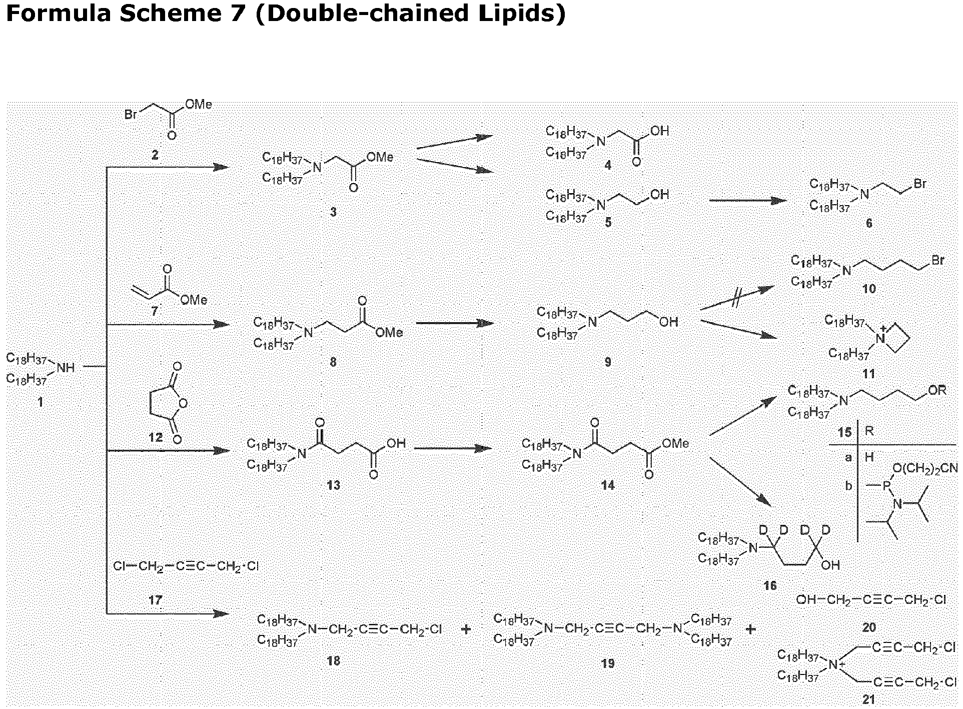

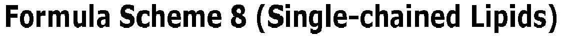

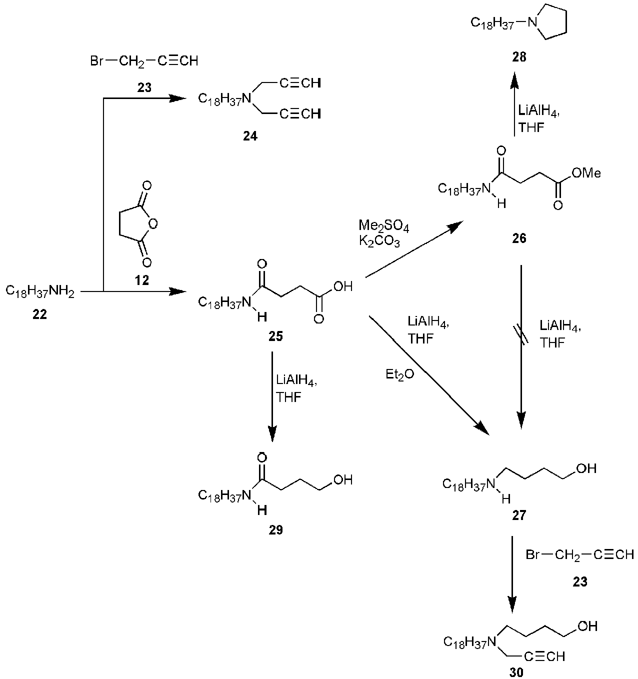

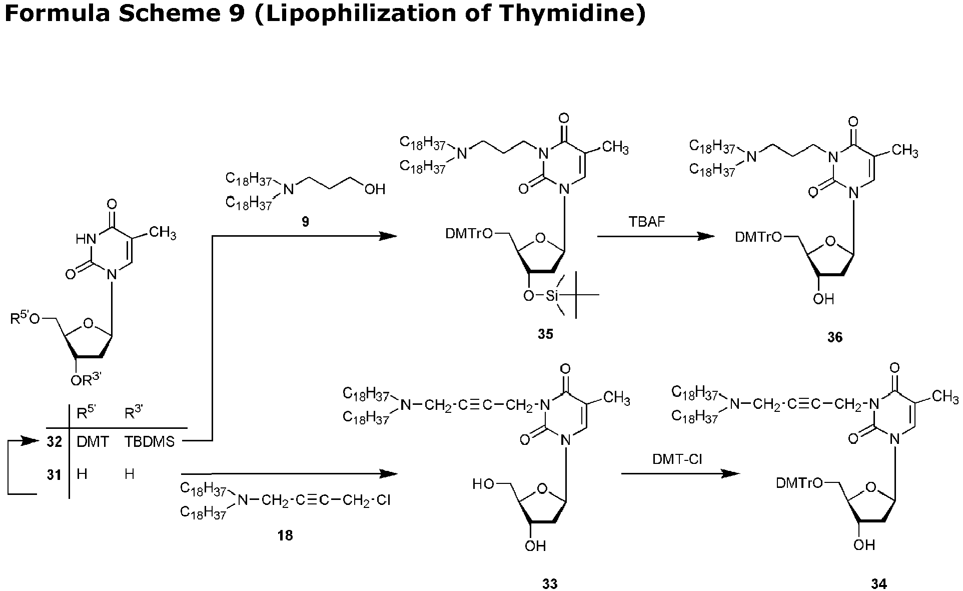

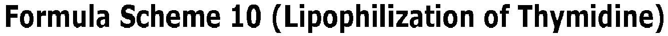

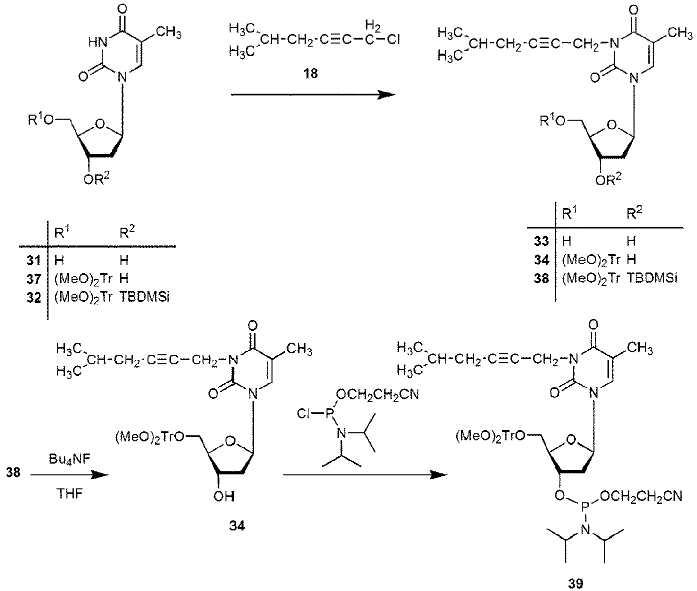

*General.* Starting compounds and solvents were purchased from the appropriate suppliers and were used as obtained. 1,4-Dichlorobut-2-yne **50** was prepared from but-2-yne-1,4-diol and thionyl chloride in pyridine according to the known procedure [6]. Reactions were carried out under argon atmosphere in a dry Schlenk flask. Chromatography: silica gel 60 (Merck, Germany). NMR Spectra: AMX-500 spectrometer *(Bruker,* D-Rheinstetten); ¹H: 500.14 MHz, ¹³C: 125.76 MHz, and ³¹P: 101.3 MHz. Chemical shifts are given in ppm relative to TMS as internal standard for ¹H and ¹³C nuclei and external 85 % H₃PO₄; *J* values in Hz. ESI MS Spectra were measured on a *Bruker Daltronics* Esquire HCT instrument *(Bruker Daltronics,* D-Leipzig); ionization was performed with a 2% aq. formic acid soln. Elemental analyses (C, H, N) of crystallized compounds were performed on a VarioMICRO instrument (Fa. *Elementar,* D-Hanau).

*Methyl N,N-(dioctadecyl)glycinate* **(36).** *N,N*-Dioctadecylamine **34** (1.90 g, 3.65 mmol), methyl bromoacetate **35** (1.62 g, 10.6 mmol), dibenzo-[18]-crown-6 (10 mg) and Na₂CO₃ (1.93 g, 18,3 mmol) were suspended in benzene (50 ml) at room temperature and stirred overnight under reflux (20 h). A second portion of methyl bromoacetate **35** (0.56 g, 3.65 mmol) was added and stirring under reflux was continued for further 10 h until the reaction was complete as monitored by ¹H-NMR analysis (amine **34** at 2.95 ppm, product **36** at 3.34 ppm). The white suspension was filtered through a silica gel layer (1 cm) to separate the unreacted amine **34,** washed with benzene (2 x 30 ml) and concentrated in *vacuo* resulting in the formation of compound **36** (2.10 g, 97%) as a slightly yellow crystalline mass [7]. TLC (hexane-Et₂O, 1 :1 v/v) : R*_{f}* 0.60. M.p. 60 - 61°C. ¹H-NMR (CDCl₃): 3.71 (s, 3H, CH₃O), 3.34 (s, 2H, CH₂COO), 2.58-2.55 (*m*, 4H, 2 CH₂CH₂N), 1.48-1.42 (*m*, 4H, 2 CH₂CH₂N), 1.28 *(br.* s, 60 H), 0.90 (*t*, 6H, *J* = 6.9, 2 CH₃). ¹³C-NMR (CDCl₃): 172.09 (C=O), 55.05 (NCH₂CH₂), 54.54 (NCH₂CO), 51.20, 51.16 (CH₃O), 31.90 (CH₂CH₂CH₃), 29.68, 29.61, 29.55, 29.31 (CH₂(CH₂)₃N), 27.47 (CH₂CH₂N), 27.37 (CH₂(CH₂)₂N), 22.65 (CH₂CH₃), 14.04, 14.03 (CH₂CH₃). ESI-MS (calculated mass: 593): 594.7 [M + H]⁺. Anal. calc. for C₃₉H₇₉NO₂ (594.07): C 78.85, H 13.40, N 2.36; found: C 78.59, H 13.50, N 2.24.

*N,N-dioctadecylglycine* **(37).** Powder of the amino-ester **36** (2.97 g, 5 mmol) was added at once to a freshly prepared solution of NaOH (0.40 g, 10 mmol) in H₂O (50 ml) and the resulting suspension was stirred at 95°C overnight. White precipitate was removed by filtration, washed with Et₂O (3 x 5 ml), suspended in H₂O (20 ml) and carefully made acidic (pH 6) by addition of HCl (5%). Precipitate was collected, washed with H₂O, pressed down and dried in vacuum resulting in the formation of acid **37** (2.84 g, 98%). TLC (CH₂Cl₂-MeOH, v/v 10:1): R*_{f}* 0.43. M.p. 102 - 103°C (Lit. m.p. 102 - 103°C, [8]). ¹H-NMR (CDCl₃): 8.63 *(br.s,* 0.5H, COOH), 8.15 (*br.s,* 0.5H, COOH), 3.46 (s, 2H, CH₂CO), 3.06 - 3.03 (*m*, 4H, 2 CH₂N), 1.70 - 1.65 (*m*, 4H, 2 CH₂CH₂N), 1.25 (*br.s,* 60H), 0.88 (*t*, 6H, *J* = 6.8, 2 CH₃). ¹³C-NMR (CDCl₃): 168.69 (COO), 54.17 (NCH₂), 31.94 (CH₃CH₂CH₂), 29.77, 29.70, 29.52, 29.38, 27.32 (CH₂CH₂N), 26.64 (CH₂(CH₂)₂N), 24.89, 22.69 (CH₃CH₂), 14.08 (CH₃) (¹H and ¹³C NMR are in agreement to those reported [8]).

*2-(Dioctadecylamino)ethanol* **(38).** Methyl *N,N-*dioctadecylglycinate **36** (2.24 g, 3.77 mmol) was dissolved in THF (150 ml), cooled in an ice-bath and a LiAlH₄ (0.57 g, 15 mmol) was added in portions with stirring within 3 min (gas evolution occurs). The cooling bath was removed, and stirring was continued overnight at room temp. The reaction mixture was cooled in an ice-bath, and MeOH (2.5 ml) was added drop-wise to destroy the excess of LiAlH₄. The mixture obtained was concentrated *in vacuo* (25 Torr), suspended in CH₂Cl₂ (100 ml), and carefully treated with H₂O (40 ml) until a solid precipitate has been formed. The organic layer was separated, washed with H₂O (50 ml), dried over anhydr. Na₂SO₄ and concentrated resulting in the formation of compd. **38** (2.0 g, 94%) as an off-white solid [7, 9]. TLC (silica gel, Et₂O): R*_{f}* 0.26. M.p. 43 - 44°C. ¹H-NMR (CDCl₃): 3.52 (*t*, 2H, *J* = 5.4, CH₂O), 3.1 *(br.* S, 1H, OH), 2.57 (*t*, 2H, *J* = 5.4, OCH₂CH₂N), 2.44 (t, 4H, *J* = 7.2, 2 CH₂CH₂CH₂N), 1.43 (*m*, 4H, CH₂CH₂CH₂N), 1.26 *(br.* s, 60H), 0.89 (*t*, 6H, *J* = 6,9, 2 CH₃). ¹³C-NMR (CDCl₃): 58.28 (CH₂O), 55.54 (CH₂CH₂O), 53.90 (NCH₂(CH₂)₁₆, 31.93 (CH₃CH₂CH₂), 29.70, 29.66, 29.60, 29.36 (CH₂(CH₂)₃N), 27.45 (CH₂CH₂CH₂N), 27.24 (CH₂(CH₂)₂N), 22.68 (CH₃CH₂), 14.08 (CH₃). ESI-MS (calculated mass: 565): 566.7 [M + H]⁺.

*N-(2-Bromoethyl)-N,N-dioctadecylamine* **(39).** PPh₃ (8.80 g, 33.6 mmol) was dissolved in a pre-cooled solution of compd. **38** (3.80 g, 6.71 mmol) in CH₂Cl₂ (180 ml) at 5°C followed by addition of CBr₄ (11.15 g, 33.6 mmol) in portions within 3 min. The resulting orange solution was stirred at ambient temperature for 30 h. The reaction mixture was concentrated, and the bromide **39** was isolated by chromatography on silica gel (100 g, eluted with a gradient mixture of hexane-CH₂Cl₂, v/v from 1:1 to 0:1) in low yield (0.43 g, 10%). TLC (silica gel, hexane-CH₂Cl₂, 1:1, v/v): R*_{f}* 0.58. M.p. 69 - 71°C. ¹H-NMR (CDCl₃): 3.38 (*t*, 2H, *J* = 7.5, CH₂Br), 2.88 (*t*, 2H, *J* = 7.5, BrCH₂CH₂N), 2.50 (*t*, 4H, *J* = 7.2, 2 (CH₂)₁₆CH₂N), 1.49 - 1.41 (*m*, 4H, 2 CH₂CH₂CH₂N), 1.27 *(br.* s, 60H), 0.90 (t, 6H, *J* = 6,9, 2 CH₃). ¹³C-NMR (CDCl₃): 56.16 (BrH₂CCH₂), 54.48 (NCH₂(CH₂)₁₆), 31.91 (CH₃CH₂CH₂). 29.68, 29.64, 29.61, 29.52 (CH₂Br), 29.33, 27.35 (NCH₂CH₂CH₂) 27.21, 22.65 (CH₂CH₃), 14.05 (CH₃). ESI-MS (calculated mass: 627 ⁷⁹Br]): 548,7 [M - HBr + H]⁺, 628.7 [M(⁷⁹Br) + H]⁺, 630.6 [M'(⁸¹Br) + H]⁺. Anal. calc. for C₃₈H₇₈BrN (628.96): C 72.57, H 12.50, N 2.23; found: C 72.18, H 12.38, N 2.04.

*Methyl N,N-dioctadecyl-2-aminopropionate* **(41).** *N,N*-Dioctadecylamine **(34,** 0.93 g, 1.78 mmol) was added to a solution of methyl acrylate **(40,** 1.75 g, 20.3 mmol) in a mixture of *i*-PrOH (14 ml) and CH₂Cl₂ (6 ml), and the resulting white suspension was stirred at 45°C overnight. The reaction mixture was filtered through a paper filter and concentrated *in vacuo* (10 Torr) resulting in the formation of the propionate **41** (1.04 g, 96%) as a white solid mass [7, 10]. TLC (silica gel, hexane-Et₂O, 1:1, v/v): R*_{f}* 0.58. M.p. 44 - 45°C. ¹H-NMR (CDCl₃): 3.67 (s, 3H, OCH₃), 2.78 (*t*, 2H, *J* = 5.4, CH₂CO), 2.47 - 2.36 (*m*, 6H, 3 CH₂N), 1.48 - 1.36 (*m*, 4H, 2 CH₂CH₂CH₂N), 1.26 *(br. s.,* 60H), 0.89 (*t*, 6H, *J* = 6,9, 2 CH₃) (in a good agreement with those reported [11]).¹³C-NMR (CDCl₃): 173.35 (C=O), 54.00 (NCH₂(CH₂)₁₆), 51.42 (OCH₃), 49.42 (CH₂CH₂CO), 32.30 (CH₂CO), 31.90 (CH₃CH₂CH₂), 29.68, 29.64, 29.60, 29.33 (N(CH₂)₃CH₂), 27.50 (NCH₂CH₂CH₂), 27.19 (N(CH₂)₂CH₂), 22.66 (CH₃CH₂), 14.07 (CH₃CH₂). ESI-MS (calculated mass: 607): 608.7 [M + 1]⁺. Anal. calc. for C₄₀H₈₁NO₂ (608.10): C 79.01, H 13.43, N 2.30; found: C 78.86, H 13.39, N 2.12.

*3-(Dioctadecylamino)propanol* **(42).** LiAlH₄ (0.26 g, 6.84 mmol) was added in portions within 2 min to a solution of propanoate **41** (1.04 g, 1.71 mmol) in THF (45 ml), cooled in an ice-bath. The bath was removed and stirring was continued overnight. The reaction mixture was carefully treated with a solution of MeOH (0.6 ml) in Et₂O (2 ml) with cooling in an ice-bath until the gas evolution ceased. Organic solvents were removed *in vacuo;* the residue was dissolved in CH₂Cl₂ (70 ml) and washed with H₂O (3 x 30 ml), dried (Na₂SO₄) and concentrated resulting in the formation of compd. **42** (0,98 g, 98%) as a white solid mass [7, 12]. TLC (silica gel, Et₂O): R*_{f}* 0.23. M.p. 48 - 49°C ¹H-NMR (CDCl₃): 5.68 (s, 1H, OH), 3.79 (*t*, 2H, *J* = 5.3, CH₂OH), 2.63 (*t*, 2H, *J* = 5.3, CH₂CH₂CH₂OH), 2.38 - 2.43 (*m*, 4H, 2 (CH₂)₁₆CH₂N), 1.67 (*quint,* 2H, *J* = 5.3, CH₂CH₂CH₂OH), 1.53 - 1.40 (*m*, 4H, 2 (CH₂)₁₅CH₂CH₂N), 1.26 *(br.* s, 60H), 0.89 (*t*, 6H, *J* = 6.5, 2 CH₃). ¹³C-NMR (CDCl₃): 64.82 (CH₂O), 55.36 (CH₂(CH₂)₂O), 54.22 (NCH₂(CH₂)₁₆), 31.90 (CH₃CH₂CH₂), 29.68, 29.64, 29.60, 29.33, 27.83 (CH₂CH₂O), 27.51 (NCH₂CH₂(CH₂)₁₅), 26.82 (N(CH₂)₂CH₂(CH₂)₁₄), 22.66 (CH₂CH₃), 14.06 (CH₃). ESI-MS (calculated mass: 579): 580.7 [M + H]⁺.

*1.1-Dioctadecylazetidinium bromide* **(44).** Crystalls of CBr₄ (320 mg, 1 mmol) were added to a pre-cooled (ice-bath) solution of aminopropanole **42** (116 mg, 0.2 mmol) and PPh₃ (260 mg, 1 mmol) in CH₂Cl₂ (13 ml) and the resulting mixture was stirred at the same temperature overnight. Yellow suspension was filtered through a SiO₂ layer (4 cm) and washed consecutively by CH₂Cl₂ (100 ml) and Et₂O (100 ml) to give in the second fraction light-yellow crystalline mass of **44** (16 mg, 13%). TLC (silica gel, CH₂Cl₂-Et₂O v/v 4:1): R*_{f}* 0.64. ¹H-NMR (CDCl₃): 4.52 - 4.49 (*m*, NCH₂), 3.57 - 3.54 (*m*, 2H, NCH₂), 3.51 - 3.48 *(m,* 2H), 2.87 - 2.79 (*m*, 2H), 1.56 (*br.s,* 2H), 1.34 - 1.26 (*m*, 60H), 1.89 *(t, J* = 6.8, 6H, 2 CH₃). ESI-MS (calculated mass: 641 [⁷⁹Br]): 562.7 [M - HBr + H]⁺, 642.6 [M(⁷⁹Br) + H]⁺, 644.6 [M'(⁸¹Br) + H]⁺.

*4-(Dioctadecylamino)-4-oxobutanoic acid* **(46).** Dioctadecylamine **(34)** (522 mg, 1 mmol) and triethylamine (202 mg, 2 mmol) were added consecutively to a stirred soln. of succinic anhydride **(45)** (150 mg, 1.5 mmol) in CH₂Cl₂ (10 ml), and the white suspension formed was stirred at 35°C overnight. The clear resulting soln. was concentrated *in vacuo* and recrystallized from acetone (3 ml) to give the acid **46** (600 mg, 96%) as a white powder. TLC (silica gel, CH₂Cl₂-AcOEt, v/v 1:1): R*_{f}* 0.62. M.p. 68 - 69°C (acetone) (Lit. m.p. 63 - 64°C (Et₂O), [8]). ¹H-NMR (CDCl₃): 3.36 - 3.33 (*m*, 2H, NCH₂), 3.26 - 3.23 (*m*, 2H, NCH₂), 2.70 (s, 4H, COCH₂CH₂CO), 1.62 - 1.52 (*m*, 4H, 2 NCH₂CH₂), 1.28 *(br.s,* 60H), 0.90 (*t*, 6H, *J* = 6.9, 2 CH₃). ¹³C-NMR (CDCl₃): 173.88 (COO), 172,55 (CON), 48.42, 46.80 (NCH₂), 31.90 (CH₃CH₂CH₂), 30.69 (NCOCH₂), 29.67, 29.63, 29.59, 29.57, 29.54, 29.52, 29.49, 29.33, 29.28, 28.81, 28.08, 27.61, 26.99, 26.87 (NCH₂CH₂CH₂), 22.65 (CH₃CH₂), 14.06 (CH₃) (¹H and ¹³C NMR are in agreement to those partly reported [13]). ESI-MS (calculated mass: 621): 622.7 [M + H]⁺. Anal. calc. for C₄₀H₇₉NO₃ (622.08): C 77.23, H 12.80, N 2.25; found: C 77.12, H 12.89, N 2.08.

*Methyl 4-(dioctadecylamino)-4-oxobutanoate* **(47).** Dimethyl sulfate (126 mg, 1 mmol) and K₂CO₃ (198 mg, 1.43 mmol) were added consecutively to a suspension of the acid **46** (311 mg, 0.5 mmol) in acetone (4 ml) and the reaction mixture was stirred at 55°C overnight. The resulting white suspension was cooled to room temperature, the precipitate was filtered off, washed with acetone (3 ml), and the filtrate was concentrated *in vacuo.* The residue was taken up in CH₂Cl₂ (5 ml), washed with aq. NH₃ (2 ml) to destroy the excess of dimethyl sulfate, and H₂O (2 x 3 ml), dried (Na₂SO₄) and concentrated resulting in the formation of the methyl ester **47** (291 mg, 91%) in a form of a colorless oil, which solidified upon standing [7]. TLC (silica gel, hexane-Et₂O, 1:1, v/v): R*_{f}* 0.55. M.p. 29 - 30°C; ¹H-NMR (CDCl₃): 3.70 (*s*, 3H, OCH₃), 3.31-3.29 (*mi*, 2H, NCH₂), 3.26-3.23 (*m*, 2H, NCH₂), 2.70-2.67 (*m*, 2H, COCH₂CH₂CO), 2.64-2.61 (*m*, 2H, COCH₂CH₂CO), 1.61-1.48 (*m*, 4H, 2 NCH₂CH₂), 1.27 (*br.s,* 60H), 0.90 (*t*, 6H, *J* = 6.9, 2 CH₃). ¹³C-NMR (CDCl₃): 173.72 (COO), 170,49 (CON), 51.62 (OCH₃), 47.85, 46.18 (NCH₂), 31.90, 29.67, 29.63, 29.58, 29.54, 29.42, 29.32, 28.94, 27.99, 27.79, 27.06, 26.92 (NCH₂CH₂CH₂), 22.65 (CH₃CH₂), 14.06 (CH₃). ESI-MS (calculated mass: 635): 1294.2 [2M + Na]⁺, 658.7 [M + Na]⁺, 636.7 [M + H]⁺.

*4-(Dioctadecylamino)butan-1-ol* **(48a).** Powdered LiAlH₄ (106 mg, 2.8 mmol) was added in portions during 2 min to a pre-cooled (ice-bath) soln. of the ester **47** (222 mg, 0.35 mmol) in THF (4 ml), and the resulting suspension was stirred at room temperature overnight. The reaction mixture was cooled on an ice-bath, and MeOH (1 ml) was added drop-wise to destroy the excess of LiAlH₄. Stirring was continued until the gas evolution had ceased. The precipitate formed was filtered off, washed with Et₂O (5 x 5 ml); the filtrate was concentrated and the crude product was purified by chromatography (preparative TLC, eluted with a mixture of CH₂Cl₂-MeOH, 15:1, v/v) resulting in the formation of the alcohol **48a** (122 mg, 76%) in a form of a colorless solid mass. TLC (silica gel, CH₂Cl₂-MeOH, 15:1, v/v): R*_{f}* 0.30. M.p. 57 - 58°C. ¹H-NMR (CDCl₃): 3.56 *(br.s,* 2H, CH₂O), 2.49 - 2.43 (m, 6H, (CH₂)₂NCH₂), 1.68 - 1.64 (*m*, 4H), 1.54 - 1.43 (*m*, 4H), 1.26 *(br.s,* 60H), 0.88 (*t*, 6H, *J* = 6.9, 2 CH₃). ¹³C-NMR (CDCl₃): 62.56 (OCH₂), 54.58 (NCH₂), 53.61 (NCH₂(CH₂)₁₆), 32.54 *(br.s,* CH₂CH₂OH), 31.30 (CH₃CH₂CH₂), 29.67, 29.63, 29.60, 29.50, 29.33, 27.62 (NCH₂CH₂(CH₂)₁₅), 26.05 *(br.s,* NCH₂CH₂), 25.71 (N(CH₂)₂CH₂(CH₂)₁₄), 22.65 (CH₃CH₂), 14.05 (CH₃). ESI-MS (calculated mass: 593): 522.7 [M - C₄H₈ + H]⁺, 594.8 [M + H]⁺.

*2-Cyanoethyl 4-(dioctadecylamino)butyl N,N-diisopropylphosphoramidite* **(48b).** A solution of dioctadecylaminobutanol **(48a,** 154 mg, 0.26 mmol) in CH₂Cl₂ (5 ml) under Argon atmosphere was treated with *Hünig's* base (101 mg, 0.78 mmol). The resulting mixture was cooled in an ice-bath, and (chloro)(2-cyanoethoxy)(diisopropylamino)phosphine (123 mg, 0.56 mmol) was added, and the reaction mixture was stirred for 20 min with cooling and then for 1 h at ambient temperature. The resulting colorless clear solution was diluted with CH₂Cl₂ (40 ml), washed with a, ice-cold aq. NaHCO₃ soln. and brine, dried (Na₂SO₄), and concentrated. The resulting oil was chromatographed (silica gel 60, eluted with benzene-Et₂O-Et₃N, 80:10:1, v/v/v); the product **(48b)** was obtained from the first three fractions upon evaporation as acolorless oil (191 mg, 93%). ¹H NMR (CDCl₃, 500 MHz) : 3.91 - 3.79 (*m*, 2H, OCH₂), 3.72 - 3.58 (*m*, 4H, OCH₂, 2 NCH), 2.65 (*t*, 2H, *J* = 6.55, NCCH₂), 2.44 - 2.41 (*m*, 2H, NCH₂), 2.40 - 2.37 (*m*, 4H, 2 NCH₂), 1.65 - 1.60 (*m*, 2H, OCH₂CH₂), 1.54 - 1.48 (*m*, 2H, NCH₂CH₂), 1.45 - 1.39 (*m*, 4H, NCH₂CH₂), 1.35 - 1.1.27 (*m*, 2H, CH₂), 1.27 (*br.s,* 2H, CH₂), 1.20 (*d*, 6H, *J* = 6.65, CH(CH₃)₂), 1.19 (*d*, 6H, *J* = 6.65, CH(CH₃)₂), 0.90 (*t*, 6H, *J* = 6.65, 2 CH₂CH₃). ¹³C-NMR (CDCl₃,125 MHz) : 117.53 (C≡N), 63.72 (*d*, ²*J*_{CP} = 17.1, CH₂OP), 58.32 (*d*, ²*J*_{CP} = 19.0, CH₂OP), 54.25 (2 CH₂N), 53.91 (CH₂N), 43.51 (*d*, ²*J*_{CP} = 12.4, 2 CHNP), 31.90 (2 CH₂CH₂CH₃), 29.68, 29.37, 29.33, 27.66 (2 CH₂CH₂N), 27.16 (2 CH₂(CH₂)₂N), 24.65 (CHCH₃), 24.59 (2 CHCH₃), 24.52 (CHCH₃), 23.59 (CH₂CH₂N), 22.66 (2 CH₂CH₃), 20.34 (d, ³*J*_{CP} = 6.7, CH₂CH₂OP), 14.06 (2 CH₃).
³¹P NMR (CDCl₃, 202.5 MHz) : 147.42. ESI-MS (calculated mass: 793): 711,7 [M - N*i*Pr₂ + OH + H]⁺, 741.8 [M - O(CH₂)₂CN + OH + H]⁺, 810.8 [M + O + H]⁺.

*[1,1,4,4-D_{4]}-4-(Dioctadecylamino)butan-1-ol* **(49).** Powdered LiAlD₄ (109 mg, 2.6 mmol) was added portions-wise during 2 min to a pre-cooled (ice-bath) soln. of the ester **47** (206 mg, 0.32 mmol) in THF (4 ml), and the resulting suspension was stirred at room temperature overnight. The reaction mixture was cooled in an ice-bath, diluted with Et₂O (10 ml), and MeOH (1 ml) was added drop-wise to destroy an excess of LiAlD₄. Stirring was continued until gas evolution had ceased (10 min). The precipitate formed was filtered off, washed with Et₂O (5 x 5 ml), the filtrate was concentrated and the crude product was suspended in CH₂Cl₂. The resulting precipitate was filtered off and washed with CH₂Cl₂ (5 x 1 ml). The filtrate was concentrated resulting in the formation of compd. **49** (145 mg, 75%) as a white solid [7]. TLC (silica gel. CH₂Cl₂-MeOH, 8:1, v/v): R*_{f}* 0.60. M.p. 59 - 60°C. ¹H-NMR (CDCl₃): 2.46 - 2.42 (*m*, 4H, (CH₂)₂NCD₂), 1.66 - 1.62 (*m*, 4H), 1.51 - 1.46 (*m*, 4H), 1.27 *(br.* s, 60H), 0.89(*t*, 6H, *J* = 6.9, 2 CH₃). ¹³C-NMR (CDCl₃) : 61.89 (*quint., J*_{C-D} = 20.5, OCD₂), 53.98 (*quint., J*_{C-D} = 21.1, NCD₂), 53.71 (NCH₂), 32.51 *(br.* s, CH₂CD₂OH), 31.90 (CH₃CH₂CH₂), 29.67, 29.63, 29.61, 29.53, 29.33, 27.66 (NCH₂CH₂), 26.13 (*br.s,* NCD₂CH₂), 25.93 (N(CH₂)₂CH₂(CH₂)₁₄), 22.66 (CH₃CH₂), 14.05 (CH₃). ESI-MS (calculated mass: 597): 522.7 [M - C₄H₄D₄ + H]⁺, 598.8 [M + H]⁺.

*4-Chloro-N,N-dioctadecylbut-2-yn-1-amine* **(51).** *N,N*-Dioctadecylamine **34** (2,08 g, 4,0 mmol), the dichloride **50** (1.48 g, 12 mmol), and Na₂CO₃ (1.69 g, 16 mmol) were suspended in benzene (40 ml) and stirred at 65 - 70°C (bath) overnight (16 h) until the reaction was completed (NMR analysis: amine **34** at 2.66 ppm, product **51** at 2.44 ppm). The light brown reaction mixture was concentrated, diluted with Et₂O, inorganic salts and residual starting amine **34** were filtered off and washed with pre-cooled Et₂O (+5°C, 20 ml). The filtrate was concentrated resulting in the formation of 2.1 g of a beige solid mass. The product **51** was isolated by chromatography on SiO₂ (100 g, eluted with a mixture CH₂Cl₂-Et₂O (4 :1, v/v, 400 ml) as a light beige mass (1.57 g, 60.5%) followed by other products (in order of their elution from the column): *N,N,N',N'-*tetraoctadecylbut-2-yne-1,4-diamine **(52),** 4-chlorobut-2-yn-1-ol **(53)** and di*N,N*-(4-chlorobut-2-ynyl)-*N,N*-dioctadecylammonium chloride **(54).** TLC (silica gel, CH₂Cl₂): R*_{f}* 0.45. M.p. 51 - 52°C. ¹H-NMR (CDCl₃): 4.18 (*t*, 2H, *J* = 1.83, CH₂Cl), 3.44 (*t,* 2H, *J* = 1.83, NCH₂C≡), 2.47 - 2.44 (m, 4H, 2 CH₂CH₂N), 1.48 - 1.41 (*m*, 4H, 2 CH₂CH₂N), 1.28 *(br. S,* 60H), 0.90 (*t*, 6H, *J* = 6,9, 2 CH₃). ¹³C-NMR (CDCl₃): 82.45 (ClC-C≡), 79.31 (ClC-C≡C), 53.83 (NCH₂CH₂), 42.19 (NCH₂C=), 31.92 (CH₂CH₂CH₃), 30.60 (CH₂Cl), 29.70, 29.65, 29.56 (CH₂CH₂CH₂CH₃), 29.35 (CH₂(CH₂)₃N), 27.52 (CH₂CH₂N), 27.46 (CH₂(CH₂)₂N), 22.66 (CH₂CH₃), 14.03 (CH₃). ESI-MS (calculated mass: 607 [³⁵Cl]): 608.7 [M(³⁵Cl) + H]⁺, 609.7, 610.7 [M'(³⁷Cl) + H]⁺, 611.7. Anal. calc. for C₄₀H₇₈ClN (608.53): C 78.95, H 12.92, N 2.30; found: C 78.73, H 12.97, N 2.15.

*N,N,N',N'-Tetraoctadecylbut-2-yne-1,4-diamine* **(52).** TLC (silica gel, CH₂Cl₂: R_{f} 0.40. M.p. 55 - 56°C. ¹H-NMR (CDCl₃): 3.43 (s, 4H, 2 NCH₂C≡), 2.47 - 2.44 (*m*, 8H, 4 CH₂CH₂N), 1.48 - 1.41 (*m*, 8H, 4 CH₂CH₂N), 1.27 *(br.* s, 120H), 0.90 (*t*, 12H, *J* = 6,9, 4 CH₃). ¹³C-NMR (CDCl₃): 79.36 (C=C), 53.95 (NCH₂CH₂), 41.97 (NCH₂C≡), 31.91 (CH₂CH₂CH₃), 29.70, 29.66, 29.64, 29.34, 27.61, 27.52, 22.66 (CH₂CH₃), 14.06 (CH₃). ESI-MS (calculated mass: 1092): 548.7 [M + 2H]⁺²

4*-Chlorobut-2-yn-1-ol* **(53).** [14] TLC (silica gel, CH₂Cl₂-Et₂O, v/v, 1:1): *R_{f}* 0.31. ¹H-NMR (CDCl₃): 4.34 (*t*, 2H, *J* = 1.75, CH₂O), 4.19 (*t*, 2H, *J* = 1.75, CH₂Cl).

*Di-N,N-(4-chlorobut-2-ynyl)-N,N-dioctadecylammonium chloride* **(54).** ¹H-NMR (CDCl₃): 4.97 (*s*, 4H, 2 ≡CCH₂N⁺), 4.21 (*s*, 4H, 2 CH₂Cl), 3.60-3.56 (*m*, 4H, 2 CH₂CH₂N⁺), 1.91-1.86 (*m*, 4H, 2 CH₂CH₂N⁺), 1.27 *(br.* s, 120H), 0.90 (*t*, 12H, *J* = 6,9, 4 CH₃).

*N-Octadecyl-N,N-diprop-2-ynylamine* **(57).** Propargyl bromide **(56,** 3.57 g, 30 mmol) and K₂CO₃ (4.14 g, 30 mmol) were added consecutively to a stirred suspension of octadecylamine **55** (2.69 g, 10 mmol) in MeOH (20 ml) in a bottle with a screwed up stopper which was finally closed. The resulting mixture was stirred at room temperature overnight. The brown suspension was filtered through a SiO₂ layer (1 cm), washed with EtOAc (100 ml), and the filtrate was concentrated to give the amine **24** (3.34 g, 96%) as viscose mass which solidified upon standing. The product is pure enough for further synthesis, however it could be easily purified for analytical purpose by filtration through a SiO₂ (5 cm, elution with a mixture hexane-AcOEt, v/v 15:1). TLC (hexane-EtOAct, 2:2, v/v): *R_{f}* 0.85. M.p. 43 - 44°C (MeOH). ¹H-NMR (CDCl₃): 3.45 (*d*, 2H, *J* = 2.3, NCH₂C≡); 2.54 - 2.51 (*m*, 2H, NCH₂(CH₂)₁₆); 2.22 (*t*, 1H, *J* = 2.3, HC≡); 1.51 - 1.44 (*m*, 2H); 1.27 *(br.* s, 30H); 0.90 (*t*, 3H, *J* = 6.5, CH₃). ¹³C-NMR (CDCl₃): 78.91 (HC≡C); 72.72 (HC≡); 53.05 (NCH₂(CH₂)₁₆); 42.08 (NCH₂C≡); 31.90, 29.66, 29.59, 29.55, 29.48, 29.32, 27.45, 27.32, 22.65 (CH₃CH₂); 14.05 (CH₃). ESI-MS (calculated mass: 345): 384.4 [M + K]⁺, 346.4 [M + H]⁺, 318.3 [M - C₂H₄ + H]⁺, 270.3 [M - C₆H₄ + H]⁺.

*4-(Octadecylamino)-4-oxobutanoic acid* **(58).** Powdered succinic anhydride **(45,** 0.440 g, 4.4 mmol) was added in portion to a stirred soln. of octadecylamine **55** (1.076 g, 4 mmol) in CH₂Cl₂ (20 ml) at r. t. followed by Et₃N (0.808 g, 8 mmol). The resulting white suspension was stirred for 3 h until the precipitate was dissolved. The clear colorless soln. was concentrated *in vacuo,* and the residue was crystallized from acetone resulting in the formation of the amide **58** (1.277 g, 87%) as white crystals. Chromatographic separation of the concentrated mother liquid on silica gel (10 g, CH₂Cl₂/MeOH, 1:1, v/v) gave a further amount of the amide **58** (0.088 g, 6%). TLC (silica gel, CH₂Cl₂-MeOH, 8:1 v/v): *R_{f}* 0.64. M.p. 124 - 125°C. ¹H-NMR (CDCl₃): 5.69 (*br.s,* 1H, NH), 3.31 - 3.27 (*m*, 2H, NCH₂), 2.73 - 2.71 (*m*, 2H, NCH₂), 2.56 - 2.54 (*m*, 2H, O=CCH₂), 1.56 - 1.51 (*m*, 2H, NCH₂CH₂(CH₂)₁₅), 1.31 (*br.s,* 2H), 1.28 (*br.s,* 28H), 0.90 (*t*, 3H, *J* = 6.9, CH₃), ¹³C-NMR: 173.02 (COO), 170,90 (CON), 40.05 (NCH₂), 31.90 (CH₃CH₂CH₂), 30.75, 30.08, 29.66, 29.63, 29.59, 29.54, 29.49, 29.39, 29.32, 29.21, 26.83 (NCH₂CH₂CH₂), 22.65 (CH₃CH₂), 14.05 (CH₃). ESI-MS (calculated mass: 369): 370.4 [M + H]⁺.

*Methyl 4-(octadecylamino)-4-oxobutanoate* **(59).** Dimethyl sulfate (0.454 g, 3.6 mmol) and K₂CO₃ (1.01 g, 7.4 mmol) were added consecutively to a stirred soln. of the acid **58** (0.680 mg, 1.8 mmol) in acetone (4 ml) at room temperature, and the resulting suspension was heated at 55°C overnight. The reaction mixture was cooled to room temperature, all solids were filtered off, washed with acetone (5 ml); the filtrate was concentrated, and the residue dissolved in CH₂Cl₂ (5 ml). The soln. was washed with H₂O (2 x 5 ml), dried (Na₂SO₄) and concentrated resulting in the formation of the ester **59** (0.502 mg, 72%) in a form of light-cream crystals. TLC (hexane-AcOEt, 1:1 v/v): R*_{f}* 0.50. M.p. 86 - 87°C (Lit. m.p. 86.5 - 87.5°C [15]). ¹H-NMR (CDCl₃): 5.60 *(br.* s, 0.8H, NH); 5.35 *(br.* s, 0.2H, NH); 3.69 (s, 3H, OCH₃); 3.23 (q, 2H, *J* = 6.75, NCH₂); 2.68 (*t*, 2H, *J* = 6.75, COCH₂); 2.46 (*t*, 2H, *J* = 6.75, COCH₂); 1.59 *(br.* s, 2H); 1.54 - 1.44 (*m*, 2H); 1.26 *(br.* s, 28H); 0.88 (*t*, 3H, *J* = 6.8, CH₂CH₃). ¹³C-NMR (CDCl₃): 173.54 (COO), 171,28 (CON), 51.79 (OCH₃), 39.72 (NCH₂), 31.92, 31.14 (NCOCH₂), 29.69, 29.65, 29.59, 29.54, 29.48, 29.34, 29.28, 26.88 (NCH₂CH₂CH₂), 22.67 (CH₃CH₂), 14.08 (CH₃). ESI-MS (calculated mass: 383): 406.3 [M + Na]⁺, 384.4 [M + H]⁺.

*1-Octadecylpyrrolidine* **(61).** Powdered LiAlH₄ (80 mg, 2.08 mmol) was added portions-wise to a pre-cooled (ice-bath) soln. of the ester **59** (100 mg, 0.26 mmol) in THF (3 ml), and the resulting suspension was stirred at room temperature over a period of 5 h. The resulting grey suspension was cooled on an ice-bath, diluted with Et₂O (6 ml), and MeOH (1 ml) was added drop-wise. The resulting mixture was stirred for 30 min until the formation of crystalline precipitate was completed. Solids were separated, washed with Et₂O (2 x 5 ml), and the filtrate was concentrated in vacuo resulting in the formation of the pyrrolidine derivative **61** (60 mg, 71%) as a yellowish solid mass. TLC (CH₂Cl₂-MeOH, 10:1 v/v): R*_{f}* 0.46. M.p. 25 - 27°C (Lit. m.p. 26 - 27°C [16]). ¹H-NMR (CDCl₃): 2.49 *(br.* s, 4H, 2 N(CH₂CH₂)₂); 2.43 - 2.40 (*m*, 2H, NCH₂(CH₂)₁₆); 1.78 *(br. s*, 4H, N(CH₂CH₂)₂); 1.54 - 1.46 (*m*, 2H, NCH₂CH₂(CH₂)₁₅); 1.30 - 1.26 *(m,* 30H); 0.89 (*t*, 3H, *J* = 6.8, CH₃). ¹³C-NMR (CDCl₃): 56.72 (NCH₂(CH₂)₁₆), 54.21 (N(CH₂CH₂)₂), 31.89 (CH₃CH₂CH₂), 29.66, 29.59, 29.32, 29.02, 27.72, 23.38 (N(CH₂CH₂)₂), 22.65 (CH₃CH₂), 14.05 (CH₃). ESI-MS (calculated mass: 323): 324.4 [M + H]⁺, 296.3 [M - C₂H₄ + H]⁺.

*4-Hydroxy-N-octadecylbutanamide* **(62).** Powdered LiAlH₄ (182 mg, 4.8 mmol) was added in portions during 3 min to a pre-cooled (ice-bath) stirred suspension of the acid **58** (222 mg, 0.6 mmol), dissolved in THF (10 ml). After 15 min the cooling bath was removed, and stirring was continued at ambient temperature for another 5 h. The reaction mixture was cooled on an ice-bath, diluted with Et₂O (20 ml), and MeOH (1 ml), followed by H₂O (1 ml) were added drop-wise until the gas evolution had ceased, and the violet suspension turned into a white precipitate. It was filtered off, washed with Et₂O; filtrates were concentrated, and the residue was separated on a TLC plate (silica gel, eluted with a mixture CH₂Cl_{2/}MeOH, 8:1, v/v) to give the amide 62 (175 mg, 82%) as colorless crystals. TLC (CH₂Cl₂-MeOH, 9:1 v/v): R*_{f}* 0.42. M.p. 86 - 87°C (Lit. m.p. 86 - 87°C [17]). ¹H-NMR (CDCl₃): 5.73 (*br.s,* 1H, NH), 3.72 - 3.70 (m, 2H, CH₂O), 3.25 (q, 2H, *J* = 6.7, NCH₂), 2.37 - 2.34 (m, 2H, CH₂C=O), 1.81 (*quint,* 2H, *J* = 6.2, CH₂CH₂C=O), 1.51 *(quint.,* 2H, *J* = 6.8, NCH₂CH₂), 1.27 (*br.s,* 30H), 0.89 (t, 3H, *J* = 6.8, CH₃). ¹³C-NMR (CDCl₃): 173.03 (C=O), 62.37 (COH), 39.71 (NCH₂), 34.06 (COCH₂), 31.89, 29.66, 29.62, 29.57, 29.52, 29.32, 29.26, 28.17 (NCH₂CH₂), 26.91 (N(CH₂)₂CH₂), 22.64 (CH₃CH₂), 14.06 (CH₃). ESI-MS (calculated mass: 355): 356.3 [M + H]⁺.

*4-(Octadecylamino)butan-1-ol* **(60).** Powdered LiAlH₄ (340 mg, 8 mmol) was added in portions during 10 min to a pre-cooled (ice-bath) stirred suspension of the acid **58** (371 mg, 1 mmol) in Et₂O (20 ml). After 5 min the cooling bath was removed, and stirring was continued at ambient temperature for another 1 h and than at 35°C overnight. The reaction mixture was cooled on an ice-bath, diluted with Et₂O (20 ml), and H₂O (0.5 ml), was added drop-wise until the gas evolution had ceased, and the gray suspension turned into a white precipitate. It was filtered off and washed with Et₂O. The filtrates were concentrated, and the white residue was separated on a TLC plate (silica gel, eluted with a mixture CH₂Cl_{2/}MeOH, 8:1, v/v) to give butanol **60** (288 mg, 84%) as colorless crystals followed by the amide **62** (22 mg, 6%). TLC (CH₂Cl₂-Et₂O, 1:1 v/v): R*_{f}* 0.42. M.p. 68 - 69°C (Lit. m.p. 68 - 70°C [18]). ¹H-NMR (CDCl₃): 3.60 - 3-58 (m, 2H, CH₂O); 2.67 - 2.65 (*m*, 2H, NCH₂); 2.63 - 2.60 (*m*, 2H, NCH₂); 1.72 - 1.67 (*m*, 2H, CH₂); 1.65 - 1.58 (*m*, 3H, CH₂, OH); 1.52 - 1.48 (*m*, 2H, CH₂), 1.26 *(br.* s, 30H); 0.88 (*t*, 3H, *J* = 6.8, CH₃). ¹³C-NMR (CDCl₃): 61.53 (COH), 47.90, 47.80 (NCH₂), 31.90 (CH₃CH₂CH₂), 29.68, 29.63, 29.60, 29.53, 29.44, 29.33, 29.07, 26.80, 25.96 (NCH₂CH₂), 23.66 (OCH₂CH₂CH₂), 22.65 (CH₃CH₂), 14.06 (CH₃). ESI-MS (calculated mass: 341): 270.4 [C₁₈NH₃]⁺, 314.4 [M - 28 + H]⁺, 342.7 [M + H]⁺.

*4-[Octadecyl(prop-2-ynyl)amino]butan-1-ol* **(63).** Propargyl bromide **(56,** 21 mg, 0.18 mmol) was added to a stirred suspension of K₂CO₃ (25 mg, 0.18 mmol) of a soln. of the amine **60** (30 mg, 0.09 mmol) in MeOH (1 ml) at room temperature. The resulting mixture was stirred overnight. The resulting precipitate was filtered off, washed with EtOAc (3 ml), the filtrate was concentrated in vacuo, and the propargylamine **63** was isolated by chromatography (silica gel, CH₂Cl₂/Et₂O, 1:1, v/v) as colorless crystals (20 mg, 61%). TLC (CH₂Cl₂/Et₂O, 1:1, v/v): R*_{f}* 0.32. M.p. 33 - 34°C. ¹H-NMR (CDCl₃): 3.59 *(br.s,* 2H, CH₂O); 3.48 *(br. s,* 2H, CH₂C≡); 2.61 - 2.59 (*m*, 2H, NCH₂); 2.58 - 2.55 (*m*, 2H, NCH₂(CH₂(₁₅); 2.21 *(br. s,* 1H, HC≡); 1.66 (*br.s,* 4H, CH₂CH₂CH₂O); 1.56 - 1.46 (*m*, 2H, NCH₂CH₂); 1.26 *(br. s,* 30H); 0.88 (*t*, 3H, *J* = 6.7, CH₃). ¹³C-NMR (CDCl₃): 73.80 (HC≡), 62.63 (CH₂OH), 53.87, 53.61 (NCH₂), 40.94 (NCH₂C≡), 31.90, 30.32 (OCH₂CH₂), 29.66, 29.62, 29.59, 29.53, 29.43, 29.32, 27.40, 26.83, 25.25 (OCH₂CH₂CH₂), 22.65 (CH₃CH₂), 14.06 (CH₃). ¹³C-NMR (C₆D₆): 78.88 (HC≡C), 73.97 (HC≡), 63.25 (CH₂OH), 54.65, 54.49 (NCH₂), 41.87 (NCH₂C≡), 32.93, 32.76 (OCH₂CH₂), 32.79, 30.72, 30.58, 30.41, 28.40, 28.29, 26.00, 23.70 (CH₃CH₂), 14.94 (CH₃). ESI-MS (calculated mass: 379): 308.4 [M - C₄H₈O + H]⁺, 362.4 [M - H₂O + H]⁺, 380,4 [M + H]⁺.

*5'-O-(4,4'-Dimethoxytrityl)-2'-deoxythymidine* **(69).** 2'-Deoxythymidine **(7,** 0.726 g, 3.0 mmol) was added portions-wise at room temperature to a yellowish clear solution of 4,4'-dimethoxytrityl chloride (1.220 g, 3.6 mmol) in pyridine (15 ml), and the resulting orange mixture was stirred overnight. It was diluted with EtOAc (80 ml), washed with H₂O (3 x 25 ml), dried (Na₂SO₄) and concentrated in *vacuo* resulting in the formation of an orange viscose mass (2.0 g). The product was isolated by chromatography on silica gel (120 ml), eluted with a gradient mixture of hexane-EtOAc, 2:1 to 0:1, v/v) as a light-yellow oil (1.52 g, 93.8%), which solidified on standing at room temperature. TLC (silica gel, EtOAc): *R_{f}* 0.5. M.p. 123 - 125°C (Lit. m.p. 122 - 124 °C, [19a]). ¹H-NMR (CDCl₃): 9.67 *(br.* s, 1H, NH), 7.60 (*s*, 1H, C(6)H), 7.41 (*d*, 2H, *J* = 7.9, C-CH ₐᵣ), 7.31 - 7.28 *(m,* 6H), 7.21 (*t*, 1H, *J* = 7.2, CH ₐᵣ), 6.83 (*d*, 4H, *J* = 8.7, 4 CH ₐᵣ), 6.45 - 6.41 (*m*, 1H, C(1')H), 4.58 - 4.56 (m, 1H, C(3')H), 4.11 - 4.07 (*m*, 1H, C(4')H), 3.77 (*s*, 6H, 2 OCH₃), 3.47 - 3.35 (*q_{AB},* 2H, H_{A} = 3.46, H_{B} = 3.37, *J*_{AB} = 10.5, *J*_{AX} = *J*_{BX} = 2.6, C(5')H₂), 2.47 - 2.43 (*m*, 1H, C(2')H₂), 2.33 - 2.28 (*m*, 1H, C(2')H₂), 1.47 (*s*, 3H, C(7)H₃). ¹³C-NMR (CDCl₃): 164.15 (C4), 158.69 (C_{Ar}OCH₃), 150.72 (C2), 144.38, 135.78 (C6), 135.48, 135.42, 130.08, 128.14, 127.95, 127.08, 113.27, 111.24 (C5), 86.88 (CH₂OC), 86.37 (C4'), 84.85 (C1'), 72.38 (C3'), 63.67 (C5'), 55.21 (OCH₃), 40.94 (C2'), 11.78 (C7) (¹H and ¹³C-NMR spectra are in a good agreement to those reported [19]). ESI-MS (calculated mass: 544): 567.3 [M + Na]⁺, 583.3 [M + K]⁺, 1111.5 [2M + Na]⁺.

*5'-O-(4,4'-Dimethoxytrityl)-3'-O-(t-butyldimethylsilyl)- 2'-deoxythymidine* **(64).** Imidazole (0.52 g, 7.6 mmol) was dissolved in a soln. of 5'-O-(4,4'-dimethoxytrityl)-2'-deoxythymidine **(69,** 1.36 g, 2.5 mmol) in DMF (20 ml) at room temperature. The resulting mixture was cooled in an ice-bath and a soln. of tert-butyldimethylsilyl chloride (0.57 g, 3.8 mmol) in DMF (3 ml) was added drop-wise during 5 min. The cooling bath was removed, and the reaction mixture was stirred at ambient temperature overnight. Methanol (10 ml) was added to destroy an excess of tert-butyldimethylsilyl chloride, and the resulting mixture was stirred for 30 min, diluted with EtOAc (200 ml), washed consecutively with aq.NaHCO₃ and H₂O, dried (Na₂SO₄) and concentrated to give crude **64** (1.95 g) as a colorless viscous oil. [7] It was purified by chromatography on silica gel (200 g), eluted with a gradient mixture of hexane-EtOAc-Et₃N, (15:15:1, v/v), resulting pure **64** (1.45 g, 88%) as a colorless viscose oil which turned to a solid foam on drying in high vacuum [20]. TLC (silica gel, hexane-EtOAc, 2:1, v/v): *R_{f}* 0.29. M.p. 87 - 88°C. ¹H-NMR (CDCl₃): 8.46 (is, 1H, NH), 7.64 (*s*, 1H, C(6)H), 7.43 (*d*, 2H, *J* = 7.9, CH ₐᵣ), 7.33 - 7.29 (*m*, 6H, C ₐᵣ), 7.27 - 7.24 *(m,* 1H, CH ₐᵣ), 6.85 (*d*, 4H, *J* = 8.8, CH ₐᵣ), 6.37 - 6.34 (*m*, 1H, C(1')H), 4.54 - 4.52 (*m*, 1H, C(3')H), 3.98 - 3.95 (m, 1H, C(4')H), 3.79 (s, 6H, 2 OCH₃), 3.50 - 3.24 (*q_{AB},* 2H, H_{A} = 3.46, H_{B} = 3.27, *J*_{AB} = 10.6, *J*_{AX} = *J*_{BX} = 2.8, C(5')H₂), 2.37 - 2.32 (*m*, 1H, C(2')H₂), 2.25 - 2.21 (*m*, 1H, C(2')H₂), 1.51 (*s*, 3H, C(7)H₃), 0.84 (s, 9H, SiC(CH₃)₃), 0.03 (s, 3H, SiCH₃), - 0.03 (s, 3H, SiCH₃). ¹³C-NMR (CDCl₃): 163.61 (C4), 158.76 (CH₃OC ₐᵣ), 150.18 (C2), 144.35, 135.58 (C6), 135.50, 135.46, 130.06, 130.04, 128.14, 127.95, 127.11, 113.28, 113.27, 110.98 (C5), 86.84 (CH₂OC), 86.80 (C4'), 84.90 (C1'), 72.11 (C3'), 62.94 (C5'), 55.23 (OCH₃), 41.54 (C2'), 25.70 (SiCCH₃), 17.92 (SiC), 11.86 (C7), - 4.69, - 4.88 (SiCH₃) (¹H and ¹³C-NMR spectra are in a good agreement to those reported [21]). ESI-MS (calculated mass: 658): 681.4 [M + Na]⁺, 697.4 [M + K]⁺.

*2'-Deoxy-3-[4-(dioctadecylamino)but-2-ynyl]thymidine* **(65).** 2'-Deoxythymidine **31** (32 mg, 0.132 mmol), DMSO (0.1 ml) and K₂CO₃ (36 mg, 0.264 mmol) were consecutively added to a stirred solution of chloride **51** (80 mg, 0.132 mmol) in THF (0.5 ml) at room temperature in a bottle with a screwed up stopper which was finally closed and the reaction mixture was stirred at 70°C during 48 h. The resulting brown mixture was cooled to room temperature, treated with H₂O (4 ml) and Et₂O (4 ml), organic phase was separated and water phase was extracted with Et₂O (4 ml). Combined organic phases were washed with H₂O, dried (Na₂SO₄), concentrated and the product **65** was isolated by preparative TLC (silica gel, eluted with EtOAc) to give 49 mg (46%) of yellow oil. TLC (EtOAc): *R_{f} 0.33.* M.p. 49 - 50°C. ¹H-NMR (CDCl₃): 7.49 (s, 1H, C(6)H); 6.24 (t, *J* = 6.7, 1H, C(1')H); 4.72 (s, 2H, CONCH₂C≡), 4.58 - 4.56 *(m,* C(3')H), 4.00 - 3.98 *(m,* 1H, C(4')H), 3.92 - 3.83 *(q_{AB},* 2H, H_{A} = 3.91, H_{B} = 3.84, *J*_{AB} = 11.8, *J*_{AX} = *J*_{BX} = 2.8, C(5')H), 3.33 (s, 2H, CH₂NCH₂C≡), 2.47 - 2.41 *(m,* 4H, N(CH₂)₂), 2.34 - 2.32 *(m,* 2H; C(2')H₂), 1.99 (s, 3H, C(7)H₃), 1.44 - 1.40 *(m,* 4H), 1.27 *(br. s,* 60H); 0.89 *(t,* 6H, *J* = 6.9, 2 CH₂CH₃). ¹³C-NMR (CDCl₃): 162.36 (C4), 150.24 (C2), 134.92 (C6), 110.30 (C5), 87.26 (C4'), 86.86 (C1'), 71.43 (C3'), 62.33 (C5'), 53.68 (NCH₂(CH₂)₁₆), 42.25 (NCH₂C≡), 40.25 (CHCH₂CH), 31.90 (CH₃CH₂CH₂), 30.74 (CONCH₂), 29.68, 29.63, 29.55, 29.33, 27.48 (NCH₂CH₂), 27.07 (N(CH₂)₂CH₂), 22.65 (CH₃CH₂), 14.06 (CH₃CH₂), 13.22 (C7). ¹³C-NMR (CD₃OD): 164.36 (C4), 151.64 (C2), 136.73 (C6), 110.69 (C5), 89.06 (C4'), 87.28 (C1'), 81.09 (C≡), 77.77 (C≡), 72.11 (C3'), 62.77 (C5'), 54.82 (NCH₂(CH₂)₁₆), 42.76 (NCH₂C≡), 41.49 (CHCH₂CH), 33.06 (CH₃CH₂CH₂), 31.52 (CONCH₂), 30.75, 30.66, 30.64, 30.51, 30.44, 28.53 (NCH₂CH₂), 27.81 (N(CH₂)₂CH₂), 23.71 (CH₃CH₂), 14.41 (CH₃CH₂), 13.13 (C7). ESI-MS (calculated mass: 813): 814.7 [M + H]⁺.

*5'-O-(4,4'-Dimethoxytrityl)-2'-deoxy-3-[4-(dioctadecylamino)but-2-ynyl]thymidine **(66)** from **65.*** A solution of 4,4'-dimethoxytrityl chloride (13.4 mg, 0.039 mmol) in pyridine (0.1 ml) was added to a pre-cooled (ice-bath) solution of butynylthymidine **65** (28 mg, 0.034 mmol), and the resulting orange solution was stirred at ambient temperature 48 h. The reaction mixture was diluted with CH₂Cl₂ (2 ml), concentrated in vacuum (0.05 Torr) and the residue was separated on an analytical TLC plate (20 x 20 cm, silica gel, eluted with a mixture CH₂Cl₂-EtOAc-Et₃N, 40:9:1, v/v/v) to give in the 3-d fraction protected thymidine **66** (28 mg, 73%) as yellowish oil. TLC (silica gel, CH₂Cl₂-EtOAc-Et₃N, v/v/v, 40:9:1): *R_{f}* 0.44. ¹H-NMR (CDCl₃): 7.55 *(s,* 1H, C(6)H); 7.42 - 7.41 *(m,* 2H, CH ₐᵣ), 7.32 - 7.30 *(m,* 6H, CH ₐᵣ), 7.26 - 7.24 *(m,* 1H, CH ₐᵣ), 6.86 - 6.84 *(m,* 2H, CH ₐᵣ), 6.43 (*t, J* = 6.6, 1H, C(1')H); 4.74 *(s,* 2H, CONCH₂C≡), 4.58 - 4.55 *(m,* C(3')H), 4.05 - 4.03 *(m,* 1H, C(4')H), 3.81 *(s,* 6H, 2 OCH₃), 3.52 - 3.39 *(q_{AB},* 2H, H_{A} = 3.45, H_{B} = 3.40, *J*_{AB} = 10.5, *J*_{AX} = 3.3, *J*_{BX} = 3.1, C(5')H₂), 3.36 (s, 2H, CH₂NCH₂C≡), 2.47 - 2.41 *(m,* 4H, N(CH₂)₂), 2.34 - 2.29 (m, 2H; NCHCH₂), 1.57 (s, 3H, C(7)H₃), 1.46 - 1.40 *(m,* 4H), 1.27 *(br. s,* 60H); 0.89 *(t,* 6H, *J* = 6.9, 2 CH₂CH₃). ¹³C-NMR (CDCl₃): 162.46 (C4), 158.78 (COCH₃), 150.21 (C2), 144.32 (OCC ₐᵣ), 135.40 (OCC ₐᵣ), 133.69 (C6), 130.06 (CH ₐᵣ), 128.12 (CH ₐᵣ), 127.14 (CH ₐᵣ), 113.31 (CH ₐᵣ), 110.38 (C5), 86.99 (OCC ₐᵣ), 85.84 (C4'), 85.30 (C1'), 72.36 (C3'), 63.43 (C5'), 55.23 (NCH₂(CH₂)₁₆), 53.72 (OCH₃), 42.35 (NCH₂C≡), 41.03 (C2'), 31.90 (CH₃CH₂CH₂), 30.76 (CONCH₂), 29.69, 29.65, 29.60, 29.33, 27.52 (NCH₂CH₂), 27.41, 22.66 (CH₃CH₂), 14.07 (CH₃CH₂), 12.60 (C7). ¹³C-NMR (C₆D₆): 161.89 (C4), 159.03 (COCH₃), 150.11 (C2), 144.88 (OCC ₐᵣ), 135.63 (OCC ₐᵣ), 133.40 (C6), 130.21 (CH ₐᵣ), 128.30 (CH ₐᵣ), 126.99 (CH ₐᵣ), 113.30 (CH ₐᵣ), 109.93 (C5), 86.87 (OCC ₐᵣ), 85.89 (C4'), 85.45 (C1'), 79.78 (C=), 77.59 (C=), 71.90 (C3'), 63.64 (C5'), 54.48 (NCH₂(CH₂)₁₆), 53.70 (OCH₃), 42.12 (NCH₂C≡), 40.72 (CHCH₂CH), 31.96 (CH₃CH₂CH₂), 30.64 (CONCH₂), 29.84, 29.75, 29.72, 29.44, 27.75 (NCH₂CH₂), 27.49, 22.72 (CH₃CH₂), 13.96 (CH₃CH₂), 12.56 (C7). ESI-MS (calculated mass: 1115): 1116,9 [M + H]⁺.

*5'-O-(4,4'-Dimethoxytrityl)-2'-deoxy-3-[4-(dioctadecylamino)but-2-ynyl]thymidine* **(66)** *from **69.** A* clear soln. of compounds **69** (72 mg, 0.132 mmol) and **51** (80 mg, 0.132 mmol) in THF (0.5 ml) was diluted with DMSO (0.2 ml); then, K₂CO₃ (36 mg, 0.264 mmol) was added, and the resulting mixture was stirred at 70 °C during 2 days. The resulting brownish reaction mixture was cooled and treated with H₂O (5 ml), extracted with Et₂O (2 x 5 ml), washed with H₂O (2 x 2 ml), dried (Na₂SO₄) and evaporated. The crude product was purified by column chromatography (silica gel 60, gradient elution with a mixture of CH₂Cl₂-MeOH, 500-30:1, v/v) resulting in isolation of the product **66** (75 mg, 51 %) and starting thymidine derivative **69** (28 mg, 39%) (in order of their elution from the column). TLC (silica gel, CH₂Cl₂-AcOEt-Et₃N, 40:9:1, v/v/v): *R_{f}* 0.46. ¹H NMR (CDCl₃, 500 MHz) : 7.55 (s, 1H, C(6)H), 7.41 (*d*, 2H, *J* = 7.65, CH ₐᵣ), 7.32 - 7.30 *(m,* 6H, 6 CH ₐᵣ), 7.25 (t, 1H, *J* = 7.3, CH ₐᵣ), 6.85 (*d*, 4H, *J* = 8.55, 4 CH ₐᵣ), 6.43 *(t,* 1H, *J* = 6.6, C(1')H), 4.77 - 4.70 *(m,* 2H, ≡CCH₂), 4.58 - 4.54 *(m,* 1H, C(3')H), 4.05 - 4.02 *(m,* 1H, C(4')H), 3.81 (s, 6H, 2 OCH₃), 3.52 - 3.38 *(q_{AB},* 2H, H_{A} = 3.50, H_{B} = 3.40, *J*_{AB} = 10.5, *J*_{AX} = *J*_{BX} = 3.3, C(5')H₂), 3.43 *(s,* 1H, OH), 3.36 *(s,* 2H, ≡CCH₂), 2.47 - 2.40 (m, 5H, CH₂NCH₂, C(2')HH), 2.35 - 2.28 *(m,* 1H, C(2')HH), 1.57 *(s,* 3H, C(7)H₃), 1.47 - 1.40 (m, 4H, 2 NCH₂CH₂(CH₂)₁₅), 1.28 (*br.s,* 60H), 0.90 (*t*, 3H, J = 6.8, CH₂CH₃).

*5'-O-(4,4'-Dimethoxytrityl)-3'-O-(t-butyldimethylsilyl)-2'-deoxy-3-[4-(dioctadecylamino)but-2-ynyl]thymidine* **(70).** A solution of compounds **64** (329 mg, 0.50 mmol) and **51** (304 mg, 0.50 mmol) in THF (4.0 ml) was diluted with DMF (5 ml); K₂CO₃ (276 mg, 2.0 mmol) and dibenzo-[18]-crown-6 (30 mg, 0.08 mmol) were added, and the resulting mixture was stirred at 60 °C for 2 days. Brown cooled reaction mixture was treated with Et₂O (100 ml), washed with H₂O (4 x 15 ml), brine, dried (Na₂SO₄) and concentrated to give the crude product **70** (589 mg, 95%). TLC (silica gel, hexane-CH₂Cl₂-acetone-Et₃N, 20:5:5:1, v/v/v/v): *R_{f}* 0.75. ¹H NMR (CDCl₃, 500 MHz) : 7.65 *(s,* 1H, C(6)H), 7.43 (*d*, 2H, *J* = 7.65, CH ₐᵣ), 7.33 - 7.29 *(m,* 6H, 6 CH ₐᵣ), 7.25 *(t,* 1H, *J* = 7.3, CH ₐᵣ), 6.85 (*d*, 4H, *J* = 8.55, 4 CH ₐᵣ), 6.40 (*t*, 1H, *J* = 6.5, C(1')H), 4.79 - 4.71 *(m,* 2H, ≡CCH₂), 4.53 - 4.51 *(m,* 1H, C(3')H), 4.00 - 3.98 *(m,* 1H, C(4')H), 3.81 (s, 6H, 2 OCH₃), 3.50 - 3.27 *(q_{AB},* 2H, H_{A} = 3.49, H_{B} = 3.29, *J*_{AB} = 10.6, *J*_{AX} = *J*_{BX} = 2.6, C(5')H₂), 3.37 (s, 2H, ≡CCH₂), 2.45 - 2.42 *(m,* 4H, CH₂NCH₂), 2.38 - 2.34 *(m,* 1H, C(2')H₂), 2.24 - 2.18 *(m,* 1H, C(2')H₂), 1.57 (s, 3H, C(7)H₃), 1.45 - 1.39 *(m,* 4H, 2 NCH₂CH₂(CH₂)₁₅), 1.28 (*br.s,* 60H), 0.90 (t, 3H, *J* = 6.5, CH₂CH₃), 0.86 *(s,* 9H, SiC(CH₃)₃), 0.04 (s, 3H, SiCH₃), - 0.02 (s, 3H, SiCH₃). ¹³C NMR (CDCl₃, 125 MHz) : 162.52 (C(4)), 158.75 (2 OC ₐᵣ), 150.21 (C(2)), 144.36 (OCC ₐᵣ), 135.49 (2 OCC ₐr), 133.73 (C(6)), 130.06, 130.05 (4 CH ₐᵣ), 128.14 (2 CH ₐᵣ), 127.93 (2 CH ₐᵣ), 127.09 (CH ₐᵣ), 113.27, 113.26 (4 CH ₐᵣ), 110.22 (C(5)), 86.83 (OCC ₐᵣ), 86.74 (C(1')), 85.58 (C(4')), 78.94 (≡C), 77.56 (≡C), 72.06 (C(3')), 62.92 (C(5')), 55.22 (2 OCH₃), 53.74 (CH₂NCH₂), 42.42 (CH₂C≡), 41.64 (C(2')), 31.91 (CH₂C≡), 30.71, 29.69, 29.64, 29.33, 27.52, 25.69 (C(CH₃)₃), 22.66 (2 CH₂CH₃), 17.90 (SiC), 14.07 (2 CH₂CH₃), 12.62 (C(7)), -4.69, -4.89 (2 SiCH₃). ESI-MS (calculated mass: 1229): 1230.9 [M + H]⁺.

*5'-O-(4,4'-Dimethoxytrityl)-2'-deoxy-3-[4-(dioctadecylamino)but-2-ynyl]thymidine* **(66)** *from **70.*** A solution of compd. **70** (192 mg, 0.156 mmol) in THF (0.4 ml) was diluted with H₂O (0.05 ml), and a soln. of tetrabutylammonium fluoride (0.17ml, 0.156 mmol) in THF was added in one portion. The resulting clear reaction mixture was stirred at 50 °C overnight. It was cooled, diluted with CH₂Cl₂ (10ml), The aqueous phase was separated, the solution was dried (Na₂SO₄ and concentrated. The pure product **66** was isolated by column chromatography on silica gel (eluted with a mixture hexane-CH₂Cl₂-acetone-Et₃N, 40:10:5:1 v/v/v/v) as beige mass (135 mg, 78%). TLC (silica gel, hexane-EtOAc, v/v, 2: 1): *R_{f}* 0.69.

*5'-O-(4,4'-Dimethoxytrityl)-2'-deoxy-3-[4-(dioctadecylamino)but-2-ynyl]thymidine 2-Cyanoethyl-N,N-diisopropylphosphoramidite* **(71).** *Hünig's* base (47 mg, 0.36 mmol) was added to a soln. of compd. **66** (135 mg, 0.12 mmol) in CH₂Cl₂ (3 ml) under Argon atmosphere; the resulting mixture was cooled in an ice-bath, (chloro)(2-cyanoethoxy)(diisopropylamino)phosphine was added, and the reaction mixture was stirred for 10 min with cooling and 1h at ambient temperature. The resulting light yellow clear solution was diluted with CH₂Cl₂ (30 ml), washed with a cold aq. NaHCO₃ solution, brine, dried (Na₂SO₄) and concentrated. The resulting yellowish oil was chromatographed on silica gel (eluted with CH₂Cl₂-acetone-Et₃N, 85:14:1, v/v/v); the product was obtained from the first 4 fractions upon evaporation as a colorless oil (142 mg, 90%) as a mixture of non-assigned *R*_{P} and *S*_{P} diastereoisomers; m.p. - 10-8 °C. ¹H NMR (CDCl₃, 500 MHz, mixture of diastereoisomers **X** and **Y** in a ratio of 2.6 : 1) : 7.64 (*S,* 0.72H, C(6)H, **X),** 7.59 (*S,* 0.28H, C(6)H, **Y),** 7.43-7.41 *(m,* 2H, CH ₐᵣ, **X, Y),** 7.33 - 7.28 *(m,* 6H, 6 CH ₐᵣ, **X, Y),** 7.27-7.23 *(m,* 1H, CH ₐᵣ, **X, Y),** 6.86-6.83 *(m,* 4H, 4 CH ₐᵣ, **X, Y),** 6.48-6.46 *(m,* 0.28H, C(1')H, **X**), 6.46-6.43 *(m,* 0.72H, C(1')H, **Y),** 4.79 - 4.71 *(m,* 2H, ≡CCH₂, **X, Y),** 4.69 - 4.63 *(m,* 1H, C(3')H, X, **Y),** 4.20 - 4.18 *(m,* 0.72H, C(4')H, **X**), 4.17 - 4.15 *(m,* 0.28H, C(4')H, **Y**), 3.81 *(s,* 4.3H, 2 OCH₃, **X**), 3.80 (s, 1.68H, 2 OCH₃, **Y**), 3.69 - 3.55 *(m,* 4H, POCH₂, 2 NCH, X, **Y**), 3.56 - 3.33 *(q_{AB},* 0.72H, H_{A} = 3.55, H_{B} = 3.34, *J*_{AB} = 10.6, *J*_{AX} = *J*_{BX} = 2.6, C(5')H₂, **X**), 3.51 - 3.31 *(q_{AB},* 0.28H, H_{A} = 3.49, H_{B} = 3.33, *J*_{AB} = 10.6, *J*_{AX} = *J*_{BX} = 2.6, C(5')H₂, **Y**), 3.36 (*br.s,* 2H, ≡CCH₂, **X**, **Y**), 2.65 - 2.61 (m, 2H, CH₂CN, **X, Y**), 2.60 - 2.55 *(m,* 0.28H, C(2')H₂, **Y**), 2.53 - 2.48 *(m,* 0.72H, C(2')H₂, **X**), 2.45 - 2.41 *(m,* 4H, CH₂NCH₂, **X**, **Y**), 2.35 - 2.29 *(m,* 1H, C(2')H₂, **X**, **Y**), 1.51 (*S*, 3H, C(7)H₃, **X**, **Y**), 1.45 - 1.39 *(m,* 4H, 2 NCH₂CH₂(CH₂)₁₅, **X**, **Y**), 1.28 (*br.s,* 60H), 1.20 - 1.18 *(m,* 12H, 2 CH(CH₃)₂, **X**, **Y**), 0.91 - 0.88 *(m,* 3H, CH₂CH₃, **X**, **Y**). ³¹P NMR (CDCl₃, 202.5 MHz): 149.17, 148.54.

*5'-O-(4,4'-Dimethoxytrityl)-3'-O-(t-butyldimethylsilyl)-2'-deoxy-3-[3-(dioctadecylamino)propyl]thymidine* **(67).** Powdered triphenylphosphine (48 mg, 0.182 mmol) was added in one portion to a stirred clear soln. of **64** (80 mg, 0.121 mmol) and alkohole **42** (70 mg, 0.121 mmol) in benzene (2 ml) at room temperature. The mixture was stirred for 5 min until all the precipitate had dissolved. Then, the mixture was cooled on an ice-bath, and diisopropyl azodicarboxylate (37 mg, 0.182 mmol) in benzene (0.5 ml) was added drop-wise within 1 min. After 5 min the cooling bath was removed, and the reaction mixture was stirred at ambient temperature overnight. The solvent was removed under vacuo, and the light-yellow solid residue was chromatographed over silica gel (eluted with a mixture of hexane-EtOAc-Et₃N, 12:6:1) yielding compd. **67** (71 mg, 48%) as a viscous yellowish mass. TLC (silica gel, hexane-AcOEt-Et₃N, 120:60:1, v/v/v): *R_{f}* 0.53. ¹H-NMR (CDCl₃): 7.61 *(s,* 1H, C(6)H), 7.41 *(d,* 2H, *J* = 7.65, CH ₐᵣ), 7.32 - 7.29 *(m,* 6H, 6 CH ₐᵣ), 7.23 *(t,* 1H, *J* = 7.3, CH ₐᵣ), 6.83 (*d*, 4H, *J* = 8.55, 4 CH ₐᵣ), 6.39 - 6.37 *(m,* 1H, C(1')H), 4.51 - 4.49 *(m,* 1H, C(3')H), 3.97 - 3.92 *(m,* 3H, C(4')H, CONCH₂), 3.79 (s, 6H, 2 OCH₃), 3.48 - 3.26 (*q_{AB},* 2H, H_{A} = 3.46, H_{B} = 3.27, *J*_{AB} = 10.6, *J*_{AX} = *J*_{BX} = 2.6, C(5')H₂), 2.54 - 2.51 *(m,* 2H, NCH₂(CH₂)₂N), 2.42 - 2.39 *(m,* 4H, 2 NCH₂(CH₂)₁₆), 2.36 - 2.31 *(m,* 1H, C(2')H₂), 2.21 - 2.17 *(m,* 1H, C(2')H₂), 1.80 - 1.76 *(m,* 2H, NCH₂CH₂CH₂N), 1.55 *(s,* 3H, C(7)H₃), 1.45 - 1.39 *(m,* 4H, 2 NCH₂CH₂(CH₂)₁₅), 1.26 (*br.s,* 60H), 0.88 (*t*, 3H, *J* = 6.5, CH₂CH₃), 0.84 (s, 9H, SiC(CH₃)₃), 0.03 (s, 3H, SiCH₃), - 0.03 *(s,* 3H, SiCH₃). ¹³C-NMR (CDCl₃): 163.40 (C4), 158.70 (CH₃OC ₐᵣ), 150.79 (C2), 144.36 (OCC ₐᵣ), 135.50 (C6), 133.34 (OCC ₐᵣ), 130.03 (OCC=CH ₐᵣ), 128.11 (CH ₐᵣ), 127.91 (CH ₐᵣ), 127.04 (CH ₐᵣ), 113.22 (CH₃OCCH ₐᵣ), 110.11 (C5), 86.76 (C4'), 86.62 (C1'), 85.41 (CH₂-OC), 72.03 (C3'), 62.89 (C5'), 55.18 (OCH₃), 53.83 (NCH₂(CH₂)₁₆), 51.55 (NCH₂(CH₂)₂N), 41.58 (C2'), 40.04 (CONCH₂), 31.88 (CH₃CH₂CH₂), 29.66 ((CH₂)₁₁), 29.31 (N(CH₂)₃CH₂), 27.58 (N(CH₂)₂CH₂CH₂), 26.95 (NCH₂ CH₂(CH₂)₁₅), 25.66 (SiCCH₃), 24.84 (NCH₂CH₂CH₂N), 22.64 (CH₃CH₂), 17.87 (SiC), 14.04 (CH₃CH₂), 12.67 (C7), - 4.72 (SiCH₃), - 4.94 (SiCH₃). ESI-MS (calculated mass: 1219): 522.7 [(C₁₈)₂NH₂]⁺, 1221.1 [M + H]⁺.

*5'-O-(4,4'-Dimethoxytrityl)-2'-deoxy-3-[3-(dioctadecylamino)propyl]thymidine* **(68).** A solution of tetrabutylammonium fluoride (0.05 ml, 1*M* in THF) was added to a solution of thymidine **67** (65 mg, 0.05 mmol) and H₂O (20 mg, 1 mmol) in THF (0.1 ml) at room temperature and the resulting mixture was stirred at 50°C overnight. The solvent was removed, the residue was dissolved in CH₂Cl₂ (1 ml) and filtered through SiO₂ layer (2 cm), washed consecutively with CH₂Cl₂ (40 ml), CH₂Cl₂-AcOEt (10:1, v/v, 40ml), EtOAc (40 ml) yielding deprotected thymidine **68** (54 mg, 90%) from the 3-d fraction as a colorless glassy mass. TLC (silica gel, EtOAc): *R_{f} 0.4.* ¹H-NMR (CDCl₃): 7.55 *(s,* 1H, C(6)H), 7.41 *(d,* 2H, *J* = 7.65, CH ₐᵣ), 7.32 - 7.29 *(m,* 6H, 6 CH ₐᵣ), 7.24 (t, 1H, *J* = 7.3, CH ₐᵣ), 6.84 *(d,* 4H, *J* = 8.55, 4 CH ₐᵣ), 6.45 - 6.43 *(m,* 1H, C(1')H), 4.57 - 4.54 *(m, 1H,* C(3')H), 4.06 - 4.03 *(m,* 1H, C(4')H), 3.98 - 3.90 *(m,* 2H, CONCH₂), 3.80 *(s,* 6H, 2 OCH₃), 3.50 - 3.37 *(q_{AB},* 2H, H_{A} = 3.49, H_{B} = 3.39, *J_{AB}* = 10.5, *J_{AX}* = *J_{BX}* = 2.9, C(5')H₂), 2.53 - 2.50 *(m,* 2H, NCH₂(CH₂)₂N), 2.44 - 2.39 *(m,* 5H, 2 NCH₂(CH₂)₁₆, C(2')H₂), 2.33 - 2.27 *(m,* 1H, C(2')H₂), 1.77 *(quint.,* 2H, *J* = 7.3, NCH₂CH₂CH₂N), 1.54 *(s,* 3H, C(7)H₃), 1.45 - 1.39 *(m,* 4H, 2 NCH₂CH₂(CH₂)₁₅), 1.27 *(br.s,* 60H), 0.90 *(t, 3H, J* = 6.9, CH₂CH₃). ¹³C-NMR (CDCl₃): 163.39 (C4), 158.75 (CH₃OC ₐᵣ), 150.84 (C2), 144.38 (OCC ₐᵣ), 135.49 (OCC ₐᵣ), 133.34 (C6), 130.07 (OCC=CH ₐᵣ), 128.14 (CH ₐᵣ), 127.96 (CH ₐᵣ), 127.10 (CH ₐᵣ), 113.29 (CH₃OCCH ₐᵣ), 110.27 (C5), 86.92 (CH₂OC), 85.91 (C4'), 85.25 (C1'), 72.21 (3'), 63.52 (C5'), 55.21 (OCH₃), 53.88 (NCH₂(CH₂)₁₆), 51.61 (NCH₂(CH₂)₂N), 41.06 (C2'), 40.10 (CONCH₂), 31.90 (CH₃CH₂CH₂), 29.69 (CH₂), 29.64 (CH₂), 29.33 (N(CH₂)₃CH₂), 27.62 (N(CH₂)₂CH₂CH₂), 26.92 (NCH₂ CH₂(CH₂)₁₅), 24.90 (NCH₂CH₂CH₂N), 22.66 (CH₃CH₂), 14.07 (CH₃CH₂), 12.65 (C7). ESI-MS (calculated mass: 1105): 1106.9 [M + H]⁺

## Claims

1. Compound represented by formula (I) 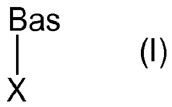 wherein X is selected from the group of formulae (II) to (IV) 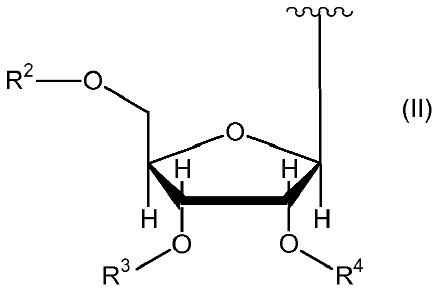 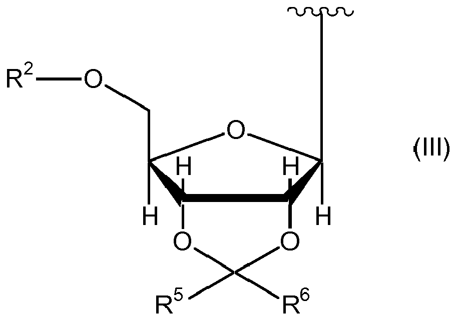 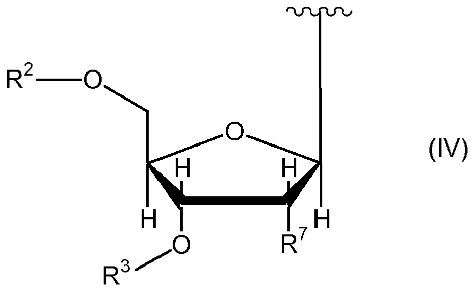 wherein
R² is H; or
R² is a Mono-phosphate, Di-phosphate, Tri-phosphate or phosphoramidite moiety; or
R² is -Y-X or -Y-L-Y¹- X;
Y and Y¹ are independently from each other a single bond or a functional connecting moiety,
X is a fluorescence marker (FA) and/or a polynucleotide moiety having up to 50 nucleotide residues, preferably 10 to 25 nucleotides, especially a polynucleotide having an antisense or antigen effect;
L is a linker by means of which Y and X are covalently linked together;
R³ and R⁴ represent independently from each other a C₁-C₂₈-alkyl moiety which may optionally be substituted or interrupted by one or more heteroatom(s) and/or functional group(s);or
R³ and R⁴ form a ring having at least 5 members, preferably a ring having 5 to 8 carbon atoms and wherein the ring may be substituted or interrupted by one or more hetero atom(s) and/or functional group(s); or
R³ and R⁴ represent independently from each other a C₁-C₂₈-alkyl moiety substituted with one or more moieties selected from the group -Y-X or -Y-L-Y¹- X; or
R³ and R⁴ represent independently from each other -Y-X or -Y-L-Y¹- X;
R⁵ and R⁶ represent independently from each other a C₁-C₂₈-alkyl moiety which may optionally be substituted or interrupted by one or more heteroatom(s) and/or functional group(s); or
R⁵ and R⁶ represent independently from each other a C₁-C₂₈-alkyl moiety substituted with one or more moieties selected from the group -Y-X or -Y-L-Y¹- X; or
R⁵ and R⁶ form a ring having at least 5 members, preferably a ring having 5 to 18 carbon atoms and wherein the ring may be substituted or interrupted by one or more hetero atom(s) and/or functional group(s); and/or one or more moieties selected from the group -Y-X or -Y-L-Y'- X;
R⁵ and R⁶ represent independently from each other -Y-X or -Y-L-Y¹- X;
R⁷ is a hydrogen atom or -O-R⁸;
R⁸ is H or C₁-C₂₈ chain which may be branched or linear and which may be saturated or unsaturated and which may optionally be interrupted and/or substituted by one or more hetero atom(s) (Het1) and/or functional group(s)(G1); or
R⁸ is -Y-X or -Y-L-Y¹- X; and
wherein
Bas is selected from the group of following formulae: 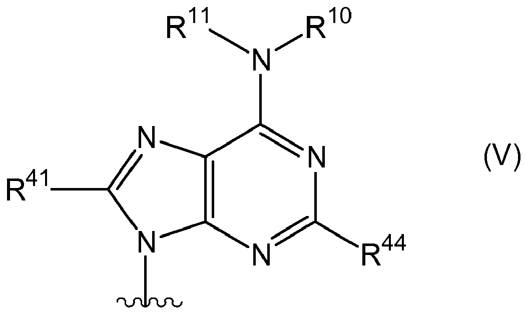 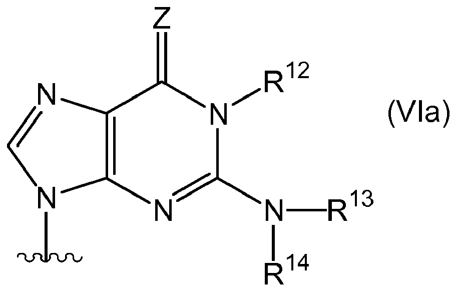 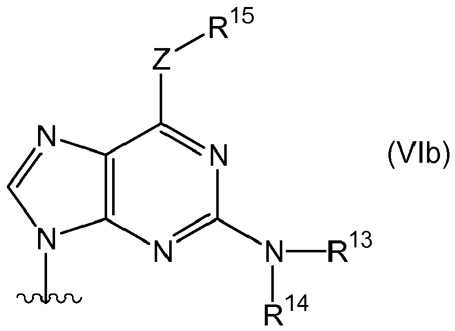 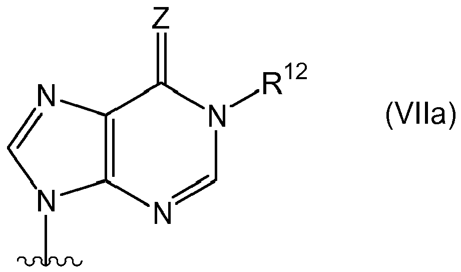 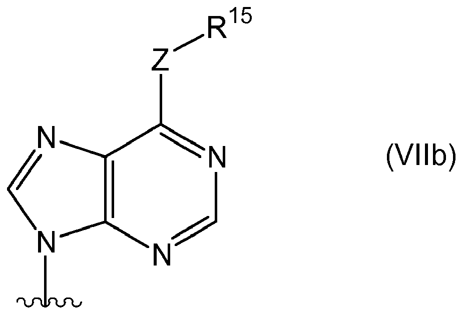 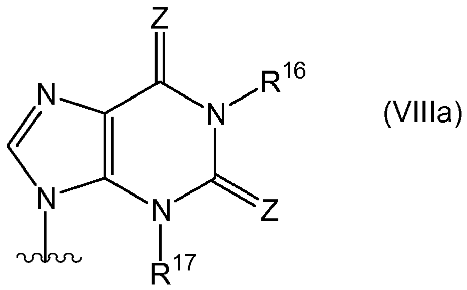 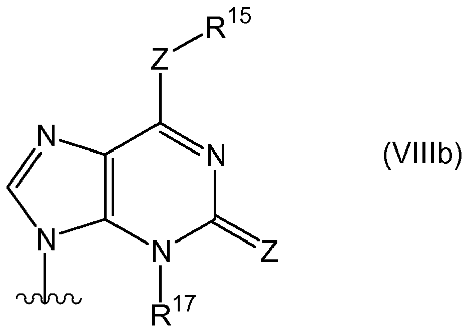 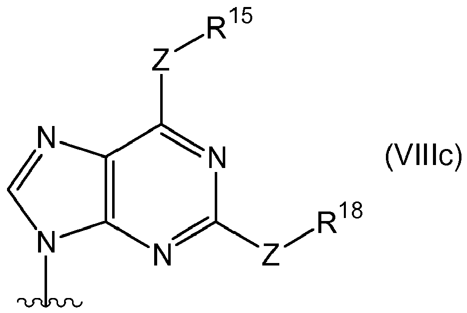 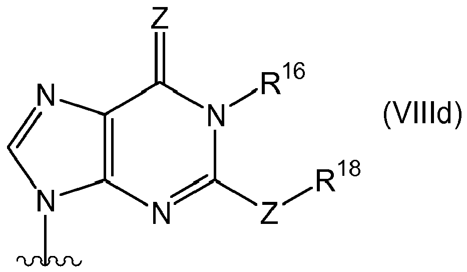 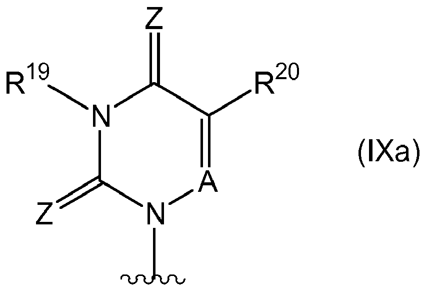 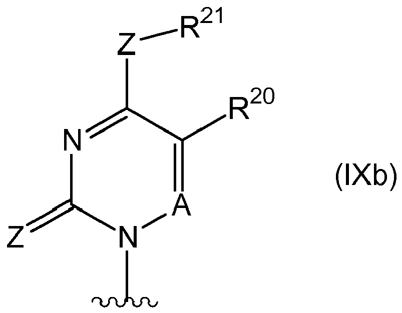 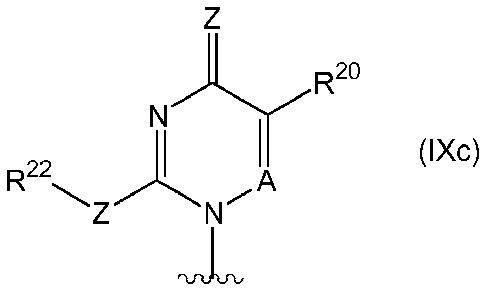 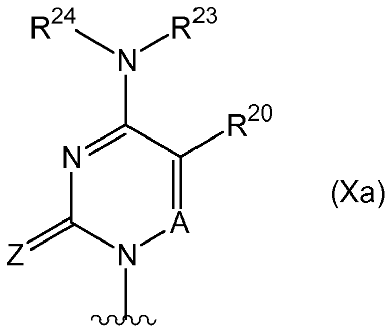 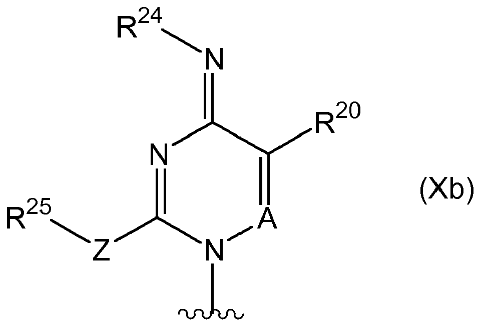 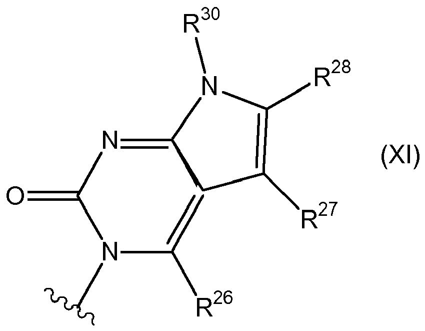 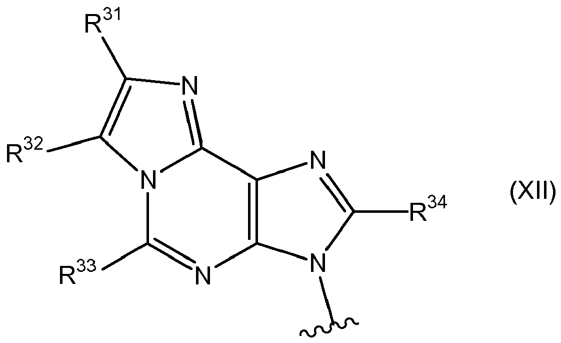 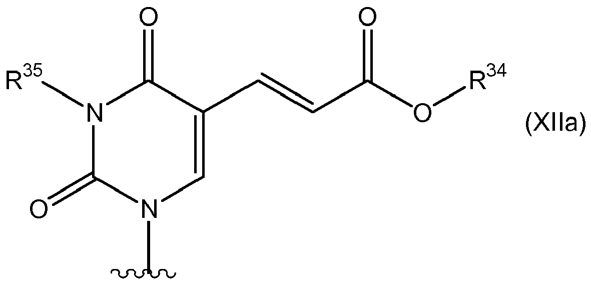 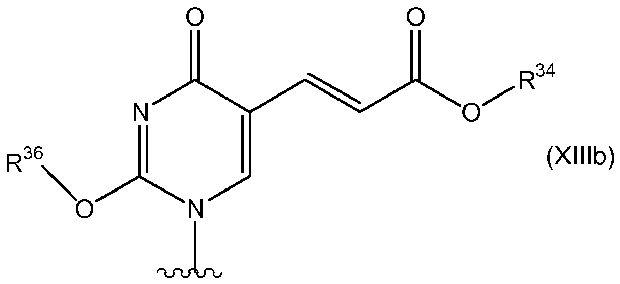 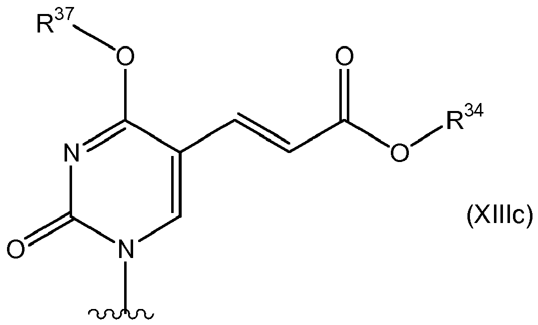 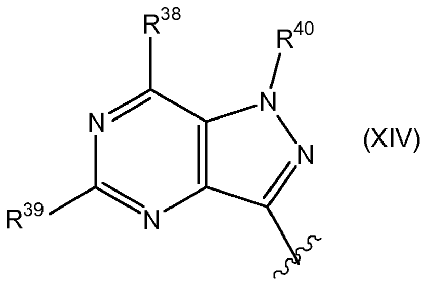 wherein
R¹⁰, R¹¹, R¹², R¹³, R¹⁴, R¹⁶, R¹⁷, R¹⁹, R²³, R²⁴, R²⁶, R²⁷, R²⁸, R³⁰, R³¹, R³², R³³, R³⁴, R³⁵, R³⁸, R³⁹ and R⁴⁰ are independently selected from H or
a C₁-C₅₀ chain which may be branched or linear and which may be saturated or unsaturated and which may optionally be interrupted and/or substituted by one or more hetero atom(s) (Het1) and/or functional group(s)(G1); or
a C₁-C₂₈ moiety which comprises at least one cyclic structure and which may be saturated or unsaturated and which may optionally be interrupted and/or substituted by one or more hetero atom(s) (Het1) and functional group(s)(G1);
R¹⁵, R¹⁸, R²¹, R²², R²⁵, R³⁶ and R³⁷ are independently selected from a C₁-C₅₀ chain which may be branched or linear and which may be saturated or unsaturated and which may optionally be interrupted and/or substituted by one or more hetero atom(s) (Het1) and/or functional group(s)(G1); or a C₁-C₂₈ moiety which comprises at least one cyclic structure and which may be saturated or unsaturated and which may optionally be interrupted and/or substituted by one or more hetero atom(s) (Het1) and functional group(s)(G1);
R²⁰ and R⁴¹ are selected from H, Cl, Br, I, CH₃, C₂₋₅₀ chain which may be branched or linear and which may be saturated or unsaturated and which may optionally be interrupted and/or substituted by one or more hetero atom(s) (Het1) and/or functional group(s)(G1); or
a C₁-C₂₈ moiety which comprises at least one cyclic structure and which may be saturated or unsaturated and which may optionally be interrupted and/or substituted by one or more hetero atom(s) (Het1) and functional group(s)(G1); or -O-C₁₋₂₈-alkyl, -S-C₁₋₂₈-alkyl, -NR⁴²R⁴³ with R⁴² and R⁴³ independently being H or a C₁₋₂₈-alkyl;
R³⁴ = H or CH₃;
R⁴⁴ is selected from H, F, Cl, Br and I;
Z is O or S; and
A is CH or N.

2. Compound according to claim 1 wherein
R¹², R¹⁶, R¹⁷, R¹⁹, R³⁰ and R³⁵ are selected from H, 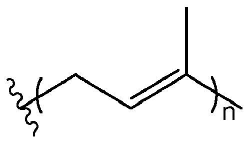 and 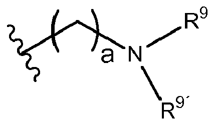 and
substituted or unsubstituted cyclic terpene moieties,
wherein
R⁹ and R^{9'} are independently selected from C₁ to C₃₀ alkyl,
n is an integer ranging 1 to 4, preferably n is 1 or 2; and
a is an integer ranging from 1 to 20, preferably 2 to 18

3. Compound according to one of claims 1 or 2 wherein the hetero atom(s) Het1 is selected from O, S and N.

4. Compound according to one or more of claims 1 to 3 wherein the linker L is a moiety comprising 1 to 30 carbon atoms which can be saturated or unsaturated, cyclic or alicyclic, branched or unbranched and which may be substituted or interrupted by heteroatoms.

5. Compound according to one or more of the preceding claims wherein
X is a polynucleotide moiety having up to 50 nucleotide residues, preferably 10 to 25 nucleotides, especially a polynucleotide having an antisense or antigen effect wherein the polynucleotide residue has preferably been coupled via a phosphoamidite precursor.

6. Process for preparing a compound represented by formula (I) 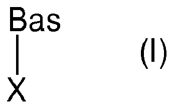 wherein
the introduction of a carbon containing substituent as defined in the preceding claims to the H-containing nitrogen ring atom, if present, comprises the following steps:
a) providing a compound of formula (I) wherein nitrogen ring atoms bonded to H are present and introducing protecting groups for hydroxyl groups, if present
b) converting an alcohol group containing carbon containing substituent in a Mitsunobu type reaction with the compound of step a) and
c) optionally, removing the protecting groups.

7. Method for detecting the presence or absence of nucleic acid having specific sequences in a sample comprising the steps of:
a) contacting a sample containing nucleic acids with nucleolipids comprising a lipophilic moiety and a poly-nucleotide moiety, whereby the polynucleotide moiety comprises a sequence which is at least partly, preferably substantially, complementary to a specific sequence of a nucleic acid present in the sample and which hybridizes under hybridizing conditions with the nucleic acid having a specific sequence;
b) detecting the formation of hybridization products of nucleolipids and a specific sequence of a nucleic acid present in the sample, wherein the nucleolipids are as defined in one or more of claims 1 to 5 and wherein the nucleoside or polynucleoside moiety is connected via a phosphoric diester.

8. The method according to claim 7, wherein the step of contacting the nucleic acids present in the sample with the nucleolipid is conducted in at least two individual compartments separated from each other, whereby in each of the compartments only nucleolipids with identical nucleoside-, oligo- or polynucleotide-moieties is present and wherein in the at least two individual compartments different nucleolipids are present.

9. A method for isolating nucleic acids from a sample containing nucleic acids, comprising the step of:
a) contacting the sample containing nucleic acids with nucleolipids as defined in one or more of claims 1 to 5, comprising a lipophilic moiety and an oligo- or polynucleotide moiety wherein the oligo or polynucleotide moiety is a able to hybridize at least partly with the nucleic acids present in the sample with the oligo- or polynucleotide moiety of the nucleolipids;
b) separating the hybridization products from the other ingredients of the sample and, optionally, washing the hybridization products.

10. The method according to claim 9, comprising the step of separating the hybridization products with a suitable device, like a dip-coater having a Wilhelmy-plate.

11. A kit for the detection of nucleic acids comprising one or more of the compounds as defined in one or more of claims 1 to 5.

12. A use of the compounds according to one or more of claims 1 to 5 for the preparation of nucleic acid arrays.

13. An array for the analysis of nucleic acids comprising at least two different nucleolipid compounds comprising a compound as defined in one or more of claims 1 to 5.

14. A system for the analysis of nucleic acids comprising a device having a lower part able to contain a liquid phase and an upper part which is not permanently attached to the lower part and which is insertable into the lower part, whereby the upper part has at least two compartments separated from each other wherein these compartments are formed from the upper to the lower side of the upper part and the device may optionally have a temperature device for at least the lower liquid phase and nucleolipids having a lipophilic moiety and an oligo- or polynucleotide moiety as defined in claims 1 to 9 wherein the nucleoside, oligo- or polynucleotide-moiety is linked via the functional moiety of a phosphoric acid diester.

15. The system according to claim 14, further comprising a spotting unit for introducing nucleolipids having specific nucleic acid sequences into the individual compartments.
